# EUROPEAN PATENT APPLICATION

(11) **EP 1 985 624 A2**
(43) Date of publication of application: **29.10.2008**
(21) Application number: 07090079.0
(22) Date of filing: 23.04.2007
(51) Int. Cl.: C07K 1/13, A61K 51/08, C07B 59/00, C07K 7/02, C07K 7/06

(54) **Single step method of radiofluorination of biologically active compounds or biomolecules**

(71) Applicant: Bayer Schering Pharma Aktiengesellschaft, 13342 Berlin (DE)
(72) Inventor: The designation of the inventor has not yet been filed

(57) **Abstract**

The invention relates to novel single step method of radiofluorination of biologically active compounds or biomolecules for obtaining radiopharmaceutical, especially for use in Positron Emitting Tomography (PET) imaging.

## Description

### FIELD OF INVENTION

The invention relates to a novel single step method of radiofluorination of biologically active compounds or biomolecules for obtaining radiopharmaceutical, especially for use in Positron Emitting Tomography (PET) imaging.

### BACKGROUND

Over the last few years, in-vivo scanning using Positron Emission Tomography (PET) has increased. PET is both a medical and research tool. It is used heavily in clinical oncology for medical imaging of tumors and the search for metastases, and for clinical diagnosis of certain diffuse brain diseases such as those causing various types of dementias. Radiotracer consisting of a radionuclide stably bound to a biomolecule is used for in vivo imaging of disorders.

In designing an effective radiopharmaceutical tracer for use as a diagnostic agent, it is imperative that the drug have appropriate in vivo targeting and pharmacokinetic properties. Fritzberg et al. (1992, J. Nucl. Med., 33:394) state further that radionuclide chemistry and associated linkages underscore the need to optimize the attachment and labelling chemical modifications of the biomolecule carrier. Hence the type of radionuclide, the type of biomolecule and the method used for linking them to one another may have a crucial effect onto the radiotracer properties.

The radionuclides used in PET scanning are typically isotopes with short half lives such as ¹¹C (∼20 min), ¹³N (~10 min), ¹⁵O (∼2 min), ⁶⁸Ga (~68 min) or ¹⁸F (∼110 min). Due to their short half lives, the radionuclides must be produced in a cyclotron which is not too far away in delivery-time from the PET scanner. These radionuclides are incorporated into biologically active compounds or biomolecules that have the function to vehicle the radionuclide into the body though the targeted site e.g. tumor.
¹⁸F-labeled compounds are gaining importance due to its availability as well as development of methods for labeling biomolecules. It has been shown that some compounds labeled with ¹⁸F, produce images of high quality. Additionally, the longer lifetime of ¹⁸F would permit longer imaging times and allows preparation of radiotracer batches for multiple patients and delivery of the tracer to other facilities, making the technique more widely available to clinical investigators. Additionally, it has been observed the development of PET cameras and availability of the instrumentation in many PET centers is increasing. Hence, it is increasingly important to develop new tracers labeled with ¹⁸F.

Peptides are biomolecules that play a crucial role in many physiological processes including actions as neurotransmitters, hormones, and antibiotics. Research has shown their importance in such fields as neuroscience, immunology, pharmacology, and cell biology. Some peptides can act as chemical messenger. They bind to receptor on the target cell surface and the biological effect of the ligand is transmitted to the target tissue. Hence the specific receptor binding property of the ligand can be exploited by labelling the ligand with a radionuclide. Theoretically, the high affinity of the ligand for the receptor facilitates retention of the radio labelled ligand in receptor expressing tissues. However, it's still under investigation which peptides can be efficiently labelled and under which conditions the labelling shall occur. It's well known that receptor specificity of ligand peptide may be altered during chemical reaction. therefore an optimal peptidic construct has to be determined.
Tumors overexpress various receptor types to which peptide bound specifically.
Boerman et al. (Seminar in Nuclear Medicine, 30,(3) July, 2000;pp195-208) provide a non exhaustive list of peptides binding to receptor involved in tumor i.e. somatostatin, Vasoactive intestinal peptide (VIP), Bombesin binding to Gastrin-releasing peptide (GRP) receptor, Gastrin, Cholecystokinin (CCK), and Calcitonin.

The linkage of the radionuclide to the biomolecule is done by various method resulting to the presence or not of a linker between the radionuclide and the biomolecule. Hence, various linkers are known. Smith CJ. et al. ("Radiochemical investigations of 177Lu-DOTA-8-Aoc-BBN[7-14]NH2: an in vitro/in vivo assessment of the targeting ability of this new radiopharmaceutical for PC-3 human prostate cancer cells." Nucl Med Bio 30(2):101-9;2003) disclose radiolabeled bombesin
wherein the linker is DOTA- X where X is a carbon tether. However, the radiolabel ¹⁷⁷Lu does not match the biological half-life of the native bombesin what makes the ¹⁷⁷Lu- DOTA- X-bombesin a non-appropriate radiotracer for imaging tumor.
Garcia Garayoa E. et al. ("Chemical and biological characterization of new Re(CO)3/[99mTc](CO)3 bombesin Analogues." Nucl Med Biol. 17-28; 2007) disclose a spacer between the radionuclide [^{99m}Tc] and the bombesin wherein the spacer is -β-Ala-β-Ala- and 3,6-dioxa-8-aminooctanoic acid. Garcia Garayoa E. et al conclude that the different spacer did not have a significant effect on stability or on receptor affinity.
Listed above linkers have been specifically designed for a specific type of radionuclide and determine the type and chemical conditions of the radiobinding method.
More recently, peptides have been conjugated to macrocyclic chelator for labelling of ⁶⁴Cu, ⁸⁶Y, and ⁶⁸Ga for PET application. However, such radionuclides interact with the in-vivo catabolism resulting into unwanted physiologic effects and chelate attachment.

Various methods of radiofluorination have been published using different precursor or starting material for obtaining ¹⁸F -labelled peptides. Due to the smaller size of peptides, both higher target-to-background ratios and rapid blood clearance can often be achieved with radiolabeled peptides. Hence, short-lived positron emission tomography (PET) isotopes are potential candidates for labelling peptides. Among a number of positron-emitting nuclides, fluorine-18 appears to be the best candidate for labelling bioactive peptides by virtue of its favourable physical and nuclear characteristics. The major disadvantage of labelling peptides with ¹⁸F is the laborious and time-consuming preparation of the ¹⁸F labelling agents. Due to the complex nature of peptides and several functional groups associated with the primary structure, ¹⁸F-labelled peptides are not prepared by direct fluorination. Hence, difficulties associated with the preparation of ¹⁸F-labeled peptide were alleviated with the employment of prosthetic groups as shown below. Several such prosthetic groups have been proposed in the literature, including N-succinimidyl-4- [¹⁸F] fluorobenzoate, m-maleimido-N-(p-[
¹⁸F]fluorobenzyl) -benzamide, N-(p- [¹⁸F]fluorophenyl) maleimide, and 4-[¹⁸ F] fluorophenacylbromide. Almost all of the methodologies currently used today for the labeling of peptides and proteins with ¹⁸F utilize active esters of the fluorine labeled synthon. 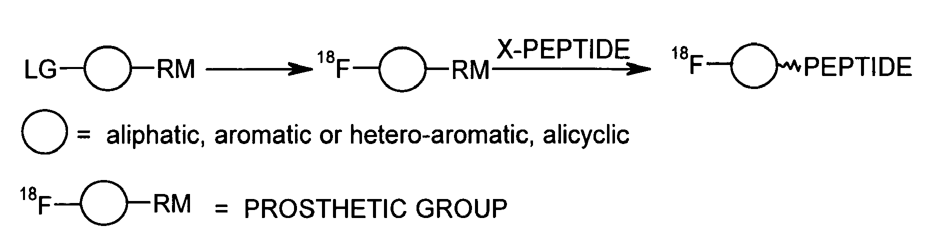 RM = reactive moiety
LG = Leaving group that can be replaced by ¹⁸F
X = functional group for reaction with RM

Okarvi et al. ("Recent progress in fluorine-18 labelled peptide radiopharmaceuticals." Eur. J. Nucl. Med. 2001 Jul;28(7):929-38)) present a review of the recent developments in ¹⁸F -labelled biologically active peptides used in PET.
Zhang Xianzhong et al. ("18F-labeled bombesin analogs for targeting GRP receptor-expressing prostate cancer." J. Nucl. Med. 47(3):492-501 (2006)) relates to the 2-step method detailed above. [Lys3]Bombesin ([Lys3]BBN) and aminocaproic acid-bombesin(7-14) (Aca-BBN(7-14)) were labeled with ¹⁸F by coupling the Lys3 amino group and Aca amino group, respectively, with N-succinimidyl-4-¹⁸F -fluorobenzoate (¹⁸F -SFB) under slightly basic condition (pH 8.5). Unfortunately, the obtained ¹⁸F -FB-[Lys3]BBN is relatively metabolically unstable having for result to reduce the extend of use of the ¹⁸F -FB-[Lys3]BBN for reliable imaging of tumor.
Poethko Thorsten et al. (,,Two-step methodology for high-yield routine radiohalogenation of peptides: 18F-labeled RGD and octreotide analogs." J. Nucl. Med. 2004 May;45(5):892-902) relates to a 2-step method for labelling RGD and octreotide analogs. The method discloses the steps of radiosynthesis of the ¹⁸F - labeled aldehyde or ketone and the chemoselective litigation of the ¹⁸F - labeled aldehyde or ketone to the aminooxy functionalized peptide.
Poethko Thorsten et al. ("First 18F-labeled tracer suitable for routine clinical imaging of somatostatin receptor-expressing tumors using positron emission tomography." Clin. Cancer Res. 2004 Jun 1;10(11):3593-606) apply the 2-step method for the synthesis of ¹⁸F-labeled carbohydrated Tyr(3)-octreotate (TOCA) analogs with optimized pharmacokinetics suitable for clinical routine somatostatin-receptor (sst) imaging.
WO 03/080544 A1 and WO 2004/080492 A1 relate to radiofluorination methods of bioactive peptides for diagnostics imaging using the 2-step method shown above.

The most crucial aspect in the successful treatment of any cancer is early detection. Likewise, it is crucial to properly diagnose the tumor and metastases.
Routine application of ¹⁸F-labeled peptides for quantitative in vivo receptor imaging of receptor-expressing tissues and quantification of receptor status using PET is limited by the lack of appropriate radiofluorination methods for routine large-scale synthesis of ¹⁸F-labeled peptides. There is a clear need for radiofluorination method that can be conducted rapidly without loss of receptor affinity by the peptide and leading to a positive imaging (with reduced background), wherein the radiotracer is stable and shows an enhanced clearance properties

It is therefore an object of the present invention to provide radiofluorination methods for obtaining radiotracer based on receptor specific peptides for the detection of tumors.

### INVENTION SUMMARY

In a first aspect, the invention relates to precursor for a single step radiolabeling. The precursor for single step radiofluorination is a compound of formula (I)

RG--L₁--B₁--Y--E (I)

In a second aspect, the invention relates radiopharmaceutical labelled with fluorine isotope of formula (II)

F--L₂--B₂--Y--E (II)

In a third aspect, the invention relates to compound of the formula II, when fluorine isotope is ¹⁸ F, for use as a positron emitting tomography (PET) diagnostic agent.

In a fourth aspect, the invention relates to compound of the formula II, when fluorine isotope is ¹⁹F, for use in biological assays and chromatographic identification.

In a fifth aspect, the invention relates to a method of radiofluorination of a compound of formula I for the manufacture of compound of formula II comprising the step of reacting a compound of formula I with a fluorination agent.

In a sixth aspect, the invention relates to a kit comprising a compound of the formula (I) as disclosed above along with an acceptable diluent, excipient or adjuvant supplied as a mixture with the compound of the formula (I) or independently for the manufacture of (II).

In a seventh aspect, the invention relates to bombesin analogs that binds specifically to human GRP receptors present in prostate tumor, breast tumor and metastasis. In a preferred embodiment, the bombesin analog is a peptide of sequence from Seq ID 1 to Seq ID 103.

### DETAILED DESCRIPTION

In a first aspect, the invention relates to precursor for a single step radiolabeling. The precursor for single step radiofluorination is a compound of formula (I)

RG--L₁--B₁--Y--E (I)

wherein
RG is a group or groups of atoms or a reactive moiety attached to L₁ that can be displaced or form an adduct with Fluorine isotope to provide chemically and biologically stable bond,
L₁ is a moiety group or bond to which the reactive group (RG) is attached,
B₁ is a functional group or a chain containing functional group connecting linker to spacer,
Y is a bond or a spacer,
E is a peptide or peptidomimetic
and pharmaceutically acceptable salts of inorganic or organic acids thereof.

In a preferred embodiment, RG is selected from the group a), b), c) or d)
wherein
a) is 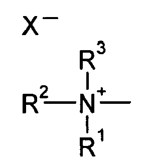 wherein R¹, R² and R³ are independently from each other C₁-C₆ alkyl or aralkyl, with the proviso that pharmaceutically acceptable salts thereof is a X- salt,
   wherein X⁻ is CF₃S(O)₂O⁻, C₄F₉S(O)₂O⁻, iodide anion, bromide anion, chloride anion, perchlorate anion (ClO₄⁻), phosphate anion, trifluoroaceate anion or other salts of inorganic or organic acids,
b) is 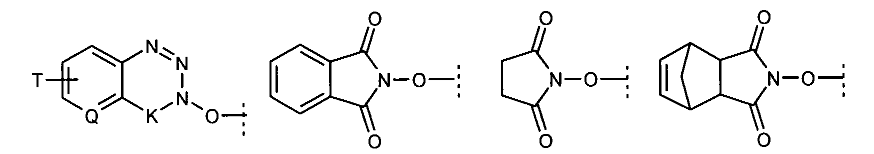 wherein,
   T is H, or CI,
   Q is CH, or N,
   K is absent, or C=O,
c) represents a leaving group suitable for fluorination, selected from hydrogen, or OR⁴, wherein R⁴ represents hydrogen, C₁-C₁₀ alkyl, C₂-C₁₀ alkenyl, C₂-C₁₀ alkynyl, aryl,
d) is N-substituted aziridine 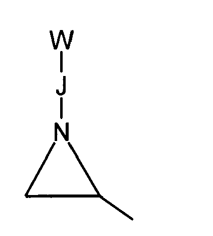
wherein J is SO₂, CO,
with the proviso that
when J is SO₂, then W is phenyl, substituted phenyl, NH₂, NHR³, NR³₂, linear or branched C₁-C₆ alkyl, polynuclear aryl, heteroaryl,
wherein substitution of the phenyl ring is independently or in combinations selected from linear or branched C₁-C₆ alkyl,
R³ is as defined above,
when J is CO, then W is phenyl, substituted phenyl, benzyloxy, fluorenylmethyl, methoxy, ethoxy, or allyloxy,
wherein substitution of the phenyl ring is independently or in combinations selected from linear or branched C₁-C₆ alkyl.

In a more preferred embodiment RG is a) as described above.
In a more preferred embodiment RG is b) as described above.
In a more preferred embodiment RG is c) as described above.
In a more preferred embodiment RG is d) as described above.

In a more preferred embodiment when RG is a) then R¹, R² and R³ are preferably and independently from each other aralkyl or linear or branched C₁-C₆ alkyl, whereas at least two moieties of R¹, R², R³ are alkyl.
More preferably, R¹ is aralkyl and R² and R³ are methyl.
More preferably, R¹, R² and R³ are methyl.

In a more preferred embodiment when RG is a) then X⁻ is CF₃S(O)₂O⁻ or C₄F₉S(O)₂O⁻. More preferably X⁻ is CF₃S(O)₂O⁻.

In a more preferred embodiment when RG is b), RG is selected from the group

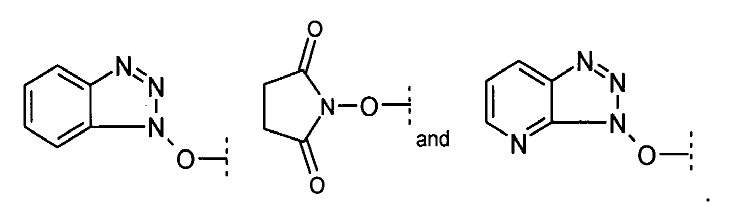

More preferably RG is

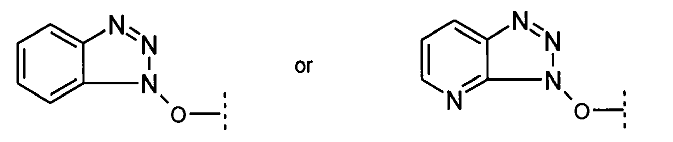

In a more preferred embodiment RG is c), RG represents a leaving group suitable for fluorination, selected from hydrogen or OR⁴, wherein R⁴ represents hydrogen, methyl, or ethyl.
More preferably RG is OR⁴, wherein R⁴ is methyl or ethyl.

In a more preferred embodiment RG is d) , RG is selected from N-benzenesulfonylaziridinyl, N-p-toluenesulfonylaziridinyl, N-2,4,6-triisopropylsulfonylaziridinyl, N-3,4-dimethoxy-phenylsulfonylaziridinyl. More preferably, RG is N-benzenesulfonylaziridinyl,p-toluenesulfonylaziridinyl or N-2,4,6-triisopropylsulfonylaziridinyl.

In a more preferred embodiment when RG is a) or b), L1 is 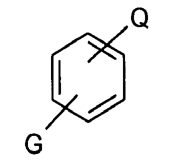 wherein
**G** is selected from -F, -Cl, -Br, -I, -NO, -NO₂, -NR⁴COCF₃, -NR⁴SO₂CF₃, - N(CF₃)₂, -NHCSNHR⁵, -N(SO₂R⁶)₂, -N(O)=NCONH₂, -NR⁵CN, -NHCSR⁶, -N≡C, -N=C(CF₃)₂, -N=NCF₃, -N=NCN, -NR⁵COR⁵, -NR⁵COOR⁶, -OSO₂CF₃ , - OS0₂C₆H₅, -OCOR⁶, -ONO₂, -OSO₂R⁶, -O-C=CH₂, -OCF₂CF₃, -OCOCF₃, -OCN, -OCF₃, -C=N, -C(NO₂)₃, -COOR⁵, -CONR⁵R⁶, -CSNH₂, -CH=NOR⁵, -CH₂SO₂R⁵, - COCF₃, -CF₃, -CF₂Cl-CBr₃, -CClF₂, -CCl₃, -CF₂CF₃, -C=CR⁴, -CH=NSO₂CF₃, - CH₂CF₃, -COR⁶, -CH=NOR⁶, -CH₂CONH₂, -CSNHR⁶, -CH=NNHCSNH₂, - CH=NNHCONHNH₂, -C≡CF₃, -CF=CFCF₃, -CF₂-CF₂-CF₃, -CR⁵(CN) ₂, - COCF₂CF₂CF₃, -C(CF₃)₃, -C(CN)₃, -CR⁵=C(CN)₂, -1-pyrryl, -C(CN)=C(CN)₂, -C-pyridyl, -COC₆H₅, -COOC₆H₅, -SOCF₃, -SO₂CF₃, -SCF₃, -SO₂CN, -SCOCF₃, - SOR⁶, -S(OR⁶), -SC=CR⁵, -SO₂R⁶, -SSO₂R⁶, -SR⁶, -SSR⁶, -SO₂CF₂CF₃, - SCF₂CF₃, -S(CF₃)=NSO₂CF₃, -SO₂C₆H₅, -SO₂N(R₆)₂, -SO₂C(CF₃)₃, -SC(CF₃)₃, - SO(CF₃)=NSO₂CF₃, -S(O)=NCF₃, -S(O)=NR⁶, -S-C=CH₂, -SCOR⁶, -SOC₆H₅, - P(O)C₃F₇, -PO(R⁶)₂, -PO(N(R⁶)₂)₂, -P(N(R⁶)₂)₂, -P(O)R⁶₂, -PO(OR⁶)₂, and electron-withdrawing groups, wherein the respective substituent can be in ortho, meta or para position
Q is hydrogen, -CN, -halo, -SO₂-R⁵ or nitro, wherein respective substituent can be in *ortho, meta* or *para* position,
R⁴ is hydrogen or linear or branched C₁-C₆ alkyl,
R⁵ is hydrogen or linear or branched C₁-C₆ alkyl,
R⁶ is C₁-C₆ linear or branched alkyl.

More preferably G is selected from -F, -Cl, -Br, -NO₂, -OSO₂R⁵, -OCF₃, -C≡N, - COOR⁴, -CONR⁴R⁵, -COCF₃, -CF₂CF₃, -COR⁵, -CF₃, -C≡CF₃, -CF₂-CF₂-CF₃, - COC₆H₅, -SO₂CF₃, -SCOCF₃, -SO₂R⁵, -SO₂CF₂CF₃, -SO₂C₆H₅, -SO₂N(R⁵) ₂, and - PO(OR⁵)₂, wherein the respective substituent can be in ortho, meta or para position and more preferably G is selected from -F, -Cl, -Br, -NO₂, , -C≡N, -CF₃, - SO₂CF₃, -SO₂R⁵, -SO₂C₆H₅, or -SO₂N(R⁵) ₂, wherein the respective substituent can be in ortho, meta or para position.

More preferably, Q is selected from hydrogen, -CN, -fluoro, -chloro, -bromo or nitro, wherein respective substituent can be in ortho, meta or para position, and more preferably Q is selected from hydrogen, -CN, -fluoro, or nitro, wherein respective substituent can be in ortho, meta or para position.

More preferably, R⁴ is hydrogen or linear or branched C₁-C₄ alkyl and even more preferably R⁴ is hydrogen or methyl.

More preferably, R⁵ is hydrogen or linear or branched C₁-C₄ alkyl and even more preferably R⁵ is hydrogen or methyl.

More preferably, R⁶ is linear or branched C₁-C₄ alkyl and even more preferably R⁶ is methyl.

In a more preferred embodiment when RG is c), L1 is

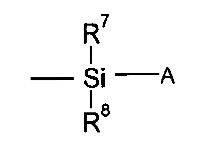

R⁷ and R⁸, independently, represent hydrogen, C₁-C₁₀ alkyl, branched C₁-C₈ alkyl, aryl, heteroaryl, or aralkyl and A represents a bond, C₁-C₁₀ alkyl or aryl, substituted aryl or heteroaryl.

In a more preferred embodiment when RG is d), L1 is bond, linear or branched C₁-C₆ alkyl. More preferably, when RG is d), L1 is bond.

In a more preferred embodiment, B1 is selected from
a bond,
-CO-,
-SO₂- with proviso that B1 is never SO₂ when RG is d),
-(CH₂)_{d}-CO-,
-SO-,
-C≡C-CO-, 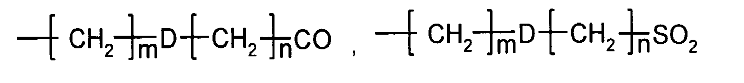 C(=O)-O,
-NR¹⁰,
-SO₂,
-NR¹⁰,
-NR¹¹,
-O,
-(S)p,
-C(=O)NR¹²,
-NR¹²,
-C(=S)NR¹²,
-C(=S)O,
C₁-C₆ cycloalkyl,
alkenyl,
heterocycloalkyl,
substituted aryl or heteroaryl,
aralkyl, heteroaralkyl,
alkoxy, aryloxy, aralkoxy, directly bound to an alkyl chain
aryl,
-SO₂NR¹³-,
-NR¹³SO₂-,
-NR¹³C(=O)O-,
-NR¹³C(=O)NR¹²-,
-NH-NH-, and
-NH-O-,
wherein;
d is an integer between 1 and 6,
m and n, independently, can be any integer between 0 to 5;
D represents a bond, a sulphur atom, an oxygen atom, or NR⁹,
R⁹ represents hydrogen, C₁-C₁₀ alkyl, aryl, heteroaryl, or aralkyl,
p can be any integer between 1 and 3;
R¹⁰, R¹¹ and R¹², independently, represent hydrogen, C₁-C₁₀ alkyl, aryl, heteroaryl, or aralkyl, and
R¹³ represents hydrogen, substituted or non-substituted, linear or branched C₁-C₆ alkyl, aryl, cycloalkyl, heterocycloalkyl, aryl, heteroaryl, aralkyl, or heteroaralkyl.

More preferably, B1 is preferably selected from
-CO-,
-SO₂ with proviso that B1 is never SO₂ when RG is d), and
-C≡C-CO-,
wherein the respective substituent can be in meta or para position.

More preferably B1 is selected from
-CO-, or
-SO₂ with proviso that B1 is never SO₂ when RG is d), wherein the respective substituent can be in meta or para position.

In a more preferred embodiment Y (spacer) is natural or unnatural amino acid sequence or non-amino acid group.
More preferably, Y is amino acid sequence with two (2) to twenty (20) amino acid residues.
More preferably, Y is Arg-Ser, Arg-Ava, Lys(Me)2-β-ala, Lys(Me)2-ser, Arg-β-ala, Ser-Ser, Ser-Thr, Arg-Thr, S-alkylcysteine, Cysteic acid, thioalkylcysteine (S-S-Alkyl) or 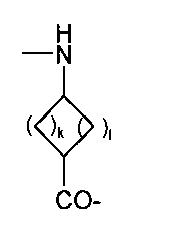 wherein k and I is 0-4.

More preferably, Y is non-amino acid moiety selected from
NH-(CH₂)ₚ-CO with p being an integer between 2 and 10, and
NH-(CH₂-CH₂-O)q-CH₂-CH₂-CO with q being an integer between 0 and 5.

In a more preferred embodiment E is a peptide comprising from 4 to 100 amino acids .
More preferably, E is selected from the group of bombesin, neuropeptide Y₁, Somatostatin receptor specific peptides, Cholecystokinin receptor peptides, Neurotensin analogs, LHRH agonists or antagonists, Gastrin releasing peptide, Integrins (α₃β₁, αᵥβ.₃, αᵥβ₅, αIIb₃), Vasoactive intestinal peptide (VIP), Pituitary adenylate cyclase activating peptide (PACAP), Epidermal growth factor, Insulin growth factor, Angiotensin, chemokines, Thyrotropin releasing hormone, substrates and inhibitors for cell surface matrix metalloproteinase, Interleukins (IL-1, IL-4 and IL-6), Prolactin, and analogs thereof.
More preferably, E is selected from the group of bombesin, somatostatin, neuropeptide Y₁, Vasoactive intestinal peptide (VIP), and analogs thereof.
More preferably, E is bombesin, somatostatin or neuropeptide Y₁ and analogs thereof.
More preferably, E is bombesin and analogs thereof.

Bombesin is a fourteen amino acid peptide that is an analog of human Gastrin releasing peptide (GRP) that binds with high specificity to human GRP receptors present in prostate tumor, breast tumor and metastasis. In a more preferred embodiment, bombesin analogs have the following sequence of formula (III)
AA₁-AA₂-AA₃-AA₄-AA₅-AA₆-AA₇-AA₈-NT₁T₂ (type A) III
T₁ = T₂ =H, T₁ = H,T₂ = OH, T₁ = CH₃, T₂ = OH
AA₁ = Gln, Asn, Phe(4-CO-NH₂)
AA₂ = Trp, D-Trp
AA₃ = Ala, Ser, Val
AA₄ = Val, Ser, Thr
AA₅ = Gly, (N-Me)Gly
AA₆ = His, His(3-Me), (N-Me)His, (N-Me)His(3-Me)
AA₇ = Sta, Statine analogs and isomers, 4-Am,5-MeHpA, 4-Am,5-MeHxA and γ-substituted aminoacids
AA₈ = Leu, Cpa, Cba, CpnA, Cha, t-buGly, tBuAla, Met, Nle, iso-Bu-Gly.

In a more preferred embodiment, bombesin analogs have the following sequence of formula (IV)
AA₁-AA₂-AA₃-AA₄-AA₅-AAₑ-AA₇-AA₈-NT₁T₂ (type B) IV
T₁ = T₂ =H, T₁ = H,T₂ = OH, T₁ = CH₃, T₂ = OH
AA₁ = Gln, Asn, Phe(4-CO-NH₂)
AA₂ = Trp, D-Trp
AA₃ = Ala, Ser, Val
AA₄ = Val, Ser. Thr
AA₅ = βAla, β²- and β³-amino acids as shown below

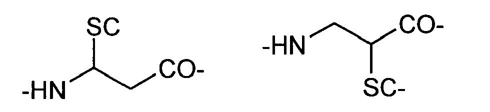

Wherein SC represents side chain found in proteinogenic amino acids and homologs of proteinogenic amino acids.
AA₆ = His, His(3-Me), (N-Me)His, (N-Me)His(3-Me)
AA₇ = Phe, Tha, Nal,
AA₈ = Leu, Cpa, Cba, CpnA, Cha, t-buGly, tBuAla, Met, Nle, iso-Bu-Gly.

In a more preferred embodiment, bombesin analogs have the following sequences:

| **Seq ID** | **E** | |
|---|---|---|
| Seq ID 1 | Gln-Trp-Ala-Val-NMeGly-His-Sta-Leu-NH2 | |
| Seq ID 2 | Gln-Trp-Ala-Val-Gly-His(Me)-Sta-Leu-NH2 | |
| Seq ID 3 | Gln-Trp-Ala-Val-NMeGly-His(3Me)-Sta-Leu-NH2 | |
| Seq ID 4 | Gln-Trp-Ala-Val-Gly-His(3Me)-Sta-Leu-NH2 | |
| Seq ID 5 | Gln-Trp-Ala-Val-Gly-His(3Me)-Sta-Leu-NH2 | |
| Seq ID 6 | Gln-Trp-Ala-Val-Gly-His(3Me)-Sta-Leu-NH2 | |
| Seq ID 7 | Gln-Trp-Ala-Val-NMeGly-His(3Me)-Sta-Cpa-NH2 | |
| Seq ID 8 | Gln-Trp-Ala-Val-Gly-His(3Me)-4-Am,5-MeHpA-Leu-NH2 | |
| Seq ID 9 | Gln-Trp-Ala-Val-Gly-His(3Me)-Sta-Leu-NH2 | |
| Seq ID 10 | | Gln-Trp-Ala-Val-Gly-His(3Me)-Sta-Leu-NH2 |
| Seq ID 11 | | Gln-Trp-Ala-Val-Gly-His(3Me)-Sta-Leu-NH2 |
| Seq ID 12 | | Gln-Trp-Ala-Val-Gly-His(3Me)-4-Am,5-MeHpA-Leu-NH2 |
| Seq ID 13 | | Gln-Trp-Ala-Val-Gly-His(3Me)-4-Am,5-MeHpA-Leu-NH2 |
| Seq ID 14 | | Gln-Trp-Ala-Val-Gly-His(3Me)-4-AM-5-MeHpA-Leu-NH2 |
| Seq ID 15 | | Gln-Trp-Ala-Val-Gly-His(3Me)-Sta-Leu-NH2 |
| Seq ID 16 | | Gln-Trp-Ala-Val-Gly-His(3Me)-4-Am,5-MeHpA-Leu-NH2 |
| Seq ID 17 | | Gln-Trp-Ala-Val-Gly-His-4-Am,5-MeHpA- -Leu-NH2 |
| Seq ID 18 | | Gln-Trp-Ala-Val-Gly-His(3Me)-Sta-Leu-NH2 |
| Seq ID 19 | | Gln-Trp-Ala-Val-NMeGly-His(3Me)-Sta-Leu-NH2 |
| Seq ID 20 | | Gln-Trp-Ala-Val-Gly-His(3Me)-Sta-Leu-NH2 |
| Seq ID 21 | | Gln-Trp-Ala-Val-Gly-His(3Me)-Sta-Leu-NH2 |
| Seq ID 22 | | Gln-Trp-Ala-Val-Gly-His(3Me)-4-Am,5-MeHpA-Leu-NH2 |
| Seq ID 23 | | Gln-Trp-Ala-Val-NMeGly-His(3Me)-4-Am,5-MeHpA-Cpa-NH2 |
| Seq ID 24 | | Gln-Trp-Ala-Val-Gly-His(3Me)-Sta-Leu-NH2 |
| Seq ID 25 | | Gln-Trp-Ala-Val-Gly-His(3Me)-Sta-Leu-NH2 |
| Seq ID 26 | | Gln-Trp-Ala-Val-NMeGly-His-Sta-Leu-NH2 |
| Seq ID 27 | | Gln-Trp-Ala-Val-NMeGly-His-FA02010-Cpa-NH2 |
| Seq ID 28 | | Gln-Trp-Ala-Val-NMeGly-His-4-Am,5-MeHpA-tbuGly-NH2 |
| Seq ID 29 | | Gln-Trp-Ala-Val-NMeGly-His(3Me)-Sta-Leu-NH2 |
| Seq ID 30 | | Gln-Trp-Ala-Val-NMeGly-His(3Me)-Sta-tBuGly-NH2 |
| Seq ID 31 | | Gln-Trp-Ala-Val-Gly-His(3Me)-Sta-Leu-NH2 |
| Seq ID 32 | | Gln-Trp-Ala-Val-NMeGly-His(3Me)-4-Am,5-MeHpA-Leu-NH2 |
| | | |
| Seq ID 33 | | Gln-DTrp-Ala-Val-Gly-His-4-Am,5-MeHpA-tbuGly-NH2 |
| | | |
| Seq ID 34 | | Gln-DTrp-Ala-Val-Gly-His-4-Am-5-MeHxA-Cpa-NH2 |
| Seq ID 35 | | Gln-Trp-Ala-Val-NMeGly-His(3Me)-Sta-Cpa-NH2 |
| Seq ID 36 | | Gln-DTrp-Ala-Val-Gly-His-Sta-tbuAla-NH2 |
| Seq ID 37 | | Gln-Trp-Ala-Val-NMeGly-His-Sta-Leu-NH2 |
| Seq ID 38 | | Gln-Trp-Ala-Val-Gly-His(3Me)-Sta-Leu-NH2 |
| Seq ID 39 | | Gln-Trp-Ala-Val-NMeGly-His(3Me)-Sta-Leu-NH2 |
| Seq ID 40 | | Gln-Trp-Ala-Val-Gly-His(3Me)-Sta-Leu-NH2 |
| Seq ID 41 | | Gln-Trp-Ala-Val-Gly-His(3Me)-Sta-Leu-NH2 |
| Seq ID 42 | | Gln-Trp-Ala-Val-Gly-His(3Me)-Sta-Cpa-NH2 |
| Seq ID 43 | | Gln-Trp-Ala-Val-Gly-His(3Me)-Sta-tBuGly-NH2 |
| Seq ID 44 | | Gln-Trp-Ala-Val-NMeGIy-His(3Me)-Sta-Leu-NH2 |
| Seq ID 45 | | Gln-Trp-Ala-Val-NMeGIy-His(3Me)-Sta-Leu-NH2 |
| Seq ID 46 | | Gln-Trp-Ala-Val-Gly-His(3Me)-4-Am,5-MeHpA-Leu-NH2 |
| Seq ID 47 | | Gln-Trp-Ala-Val-NMeGly-His(3Me)-4-Am,5-MeHpA-Cpa-NH2 |
| | | |
| Seq ID 48 | | GIn-Trp-Ala-Val-Gly-His(3Me)-4-Am,5-MeHpA-Leu-NH2 |
| Seq ID 49 | | Gln-Trp-Ala-Val-Gly-NMeHis-4-Am,5-MeHpA-Cpa-NH2 |
| Seq ID 49 | | Gln-Trp-Ala-Val-Gly-NMeHis(3Me)-4-Am,S-MeHpA-Leu-NH2 |
| | | |
| Seq ID 50 | | Gln-Trp-Ala-Val-Gly-NMeHis-4-Am,5-MeHpA-Leu-NH2 |
| Seq ID 51 | | Gln-Trp-Ala-Val-NMeGly-Hls-AHMHxA -Leu-NH2 |
| Seq ID 52 | | Gln-Trp-Ala-Val-βAla-NMeHis-Tha-Cpa-NH2 |
| Seq ID 53 | | Gln-Trp-Ala-Val-βAla-NMeHis-Phe-Cpa-NH2 |
| Seq ID 54 | | Gln-Trp-Ala-Val-βAla-NMeHis-Phe-Leu-NH2 |
| Seq ID 55 | | Gln-Trp-Ala-Val-βAla-DHis-Phe-Leu-NH2 |
| Seq ID 56 | | Gln-Trp-Ala-Val-βAla-His-ßhLeu-Leu-NH2 |
| Seq ID 57 | | Gln-Trp-Ala-Val-βAla-His-ßhlle-Leu-NH2 |
| Seq ID 58 | | Gln-Trp-Ala-Val-βAla-His-ßhLeu-tbuGly-NH2 |
| Seq ID 59 | | Gln-Trp-Ala-Val-βAla-His(3Me)-Phe-Tha-NH2 |
| Seq ID 60 | | Gln-Trp-Ala-Val-βAla-His(3Me)-Phe-Nle-NH2 |
| Seq ID 61 | | Gln-Trp-Ala-Val-βAla-NMeHis-Phe-tbuGly-NH2 |
| Seq ID 62 | | Gln-Trp-Ala-Val-βAla-NMeHis-Tha-tbuGly-NH2 |
| Seq ID 63 | | Gln-Trp-Ala-Val-βAla-His(3Me)-Tha-tbuGly-NH2 |
| Seq ID 64 | | Gln-Trp-Ala-Val-βAla-His(3Me)-Phe-Cpa-NH2 |
| Seq ID 65 | | Gln-Trp-Ala-NMeVal-βAla-His-Phe-Leu-NH2 |
| Seq ID 66 | | GIn-Trp-Ala-Val-βAla-His-NmePhe-Leu-NH2 |
| Seq ID 67 | | GIn-DTrp-Ala-Val-βAla-His-Phe-Leu-NH2 |
| Seq ID 68 | | Gln-Trp-DAIa-Val-βAla-His-Phe-Leu-NH2 |
| Seq ID 69 | | Gln-Trp-Ala-DVal-βAla-His-Phe-Leu-NH2 |
| Seq ID 70 | | Gln-Trp-Ala-Val-βAla-His-DPhe-Leu-NH2 |
| Seq ID 71 | | Gln-Trp-Ala-Val-βAla-His-βhlle-tbuGly-NH2 |
| Seq ID 72 | | Gln-Trp-Ala-Val-NMeGIy-His-4-Am,S-MeHpA-Cpa-NH2 |
| Seq ID 73 | | Gln-Trp-Ala-Val-NMeGIy-His-Sta-Cpa-NH2 |
| Seq ID 74 | | Gln-Trp-Ala-Val-NMeGIy-His-Sta-tbuAla-NH2 |
| Seq ID 75 | | Gln-Trp-Ala-Val-NMeGly-His-4-Am,5-MeHpA-tbuAla-NH2 |
| Seq ID 76 | | Gln-Trp-Ala-Val-NMeGIy-His-Sta-Leu-NH2 |
| Seq ID 77 | | Gln-Trp-Ala-Val-His(Me)-Sta-Leu-NH2 |
| Seq ID 78 | | Gln-Trp-Ala-Val-NMeGIy-His(3Me)-Sta-Leu-NH2 |
| Seq ID 79 | | Gln-Trp-Ala-Val-Gly-His(3Me)-Sta-Leu-NH2 |
| Seq ID 79 | | Gln-Trp-Ala-Val-Gly-His(3Me)-Sta-Leu-NH2 |
| Seq ID 80 | | Gln-Trp-Ala-Val-Gly-His(3Me)-Sta-Leu-NH2 |
| Seq ID 81 | | Gln-Trp-Ala-Val-NMeGly-His(3Me)-Sta-Cpa-NH2 |
| Seq ID 82 | | Gln-Trp-Ala-Val-Gly-His(3Me)-FA4-Am,5-MeHpA-Leu-NH2 |
| Seq ID 83 | | Gln-Trp-Ala-Val-Gly-His(3Me)-Sta-Leu-NH2 |
| Seq ID 84 | | Gln-Trp-Ala-Val-Gly-His(3Me)-Sta-Leu-NH2 |
| Seq ID 85 | | Gln-Trp-Ala-Val-Gly-His(3Me)-Sta-Leu-NH2 |
| Seq ID 86 | | Gln-Trp-Ala-Val-Gly-His(3Me)-4-Am,5-MeHpA-Leu-NH2 |
| Seq ID 87 | | Gln-Trp-Ala-Val-Gly-His(3Me)-4-Am,5-MeHpA-Leu-NH2 |
| Seq ID 88 | | Gln-Trp-Ala-Val-Gly-His(3Me)-4-Am,5-MeHpA-Leu-NH2 |
| Seq ID 89 | | Gln-Trp-Ala-Val-Gly-His(3Me)-Sta-Leu-NH2 |
| Seq ID 90 | | Gln-Trp-Ala-Val-Gly-His(3Me)-4-Am,S-MeHpA-Leu-NH2 |
| Seq ID 91 | | Gln-Trp-Ala-Val-Gly-His-4-Am,5-MeHpA-Leu-NH2 |
| | | |
| Seq ID 92 | | Gln-Trp-Ala-Val-Gly-His(3Me)-Sta-Leu-NH2 |
| Seq ID 93 | | Gln-Trp-Ala-Val-NMeGly-His(3Me)-Sta-Leu-NH2 |
| Seq ID 94 | | Gln-Trp-Ala-Val-Gly-His(3Me)-Sta-Leu-NH2 |
| Seq ID 95 | | Gln-Trp-Ala-Val-Gly-His(3Me)-Sta-Leu-NH2 |
| Seq ID 96 | | Gln-Trp-Ala-Val-Gly-His(3Me)-4-Am,5-MeHpA-Leu-NH2 |
| Seq ID 97 | | Gln-Trp-Ala-Val-Gly-His(3Me)-4-Am,5-MeHpA-Leu-NH2 |
| | | |
| Seq ID 98 | | Gln-Trp-Ala-Val-NMeGly-His(3Me)-4-Am,5-MeHpA-Cpa-NH2 |
| | | |
| Seq ID 99 | | Gln-Trp-Ala-Val-NMeGly-His-Sta-Leu-NH2 |
| | | |
| Seq ID 100 | | Gln-Trp-Ala-Val-Gly-His(3Me)-Sta-Leu-NH2 |
| | | |
| Seq ID 101 | | Gln-Trp-Ala-Val-Gly-His(3Me)- 4-Am-5-MeHpA - 4-amino-5-methylheptanoic acid -Leu-NH2 |
| | | |
| Seq ID 102 | | Gln-Trp-Ala-Val-NMeGly-His(3Me)- 4-Am-5-MeHpA - 4-amino-5-methylheptanoic acid -Cpa-NH2 |
| | | |
| Seq ID 103 | | Gln-Trp-Ala-Val-NMeGly-His(3Me)- 4-Am-5-MeHpA - 4-amino-5-methylheptanoic acid -Cpa-NH2 |

In a more preferred embodiment, somatostatin analogs have the following sequences:

| | |
|---|---|
| Seq ID 104 | ----c[Lys-(NMe)Phe-1Nal-D-Trp-Lys-Thr] |
| Seq ID 105 | ----c[Dpr-Met-(NMe)Phe-Tyr-D-Trp-Lys] |

In a more preferred embodiment, neuropeptide Y₁ analogs have the following sequences:

| | |
|---|---|
| Seq ID 106 | -DCys-Leu-Ile-Thr-Arg-Cys-Arg-Tyr-NH₂ |
| Seq ID 107 | -DCys-Leu-Ile-Val-Arg-Cys-Arg-Tyr-NH₂ |
| (_ indicates disulfide bridge) | |

In a preferred embodiment, the precursor for a single step radiolabeling is selected from the following list wherein E is a bombesin analog:
la-1 4-(Trimethylammonium)-3-cyano-benzoyl-Arg-Ava-Gln-Trp-Ala-Val-NMeGly-His-Sta-Leu-NH2,
la-2 4-(Trimethylammonium)-3-cyano-benzoyl-Arg-Ava-Gln-Trp-Ala-Val-Gly-His(Me)-Sta-Leu-NH2,
la-3 4-(Trimethylammonium)-3-cyano-benzoyl-Arg-Ava-Gln-Trp-Ala-Val-NMeGly-His(3Me)-Sta-Leu-NH2,
la-4 4-(Trimethylammonium)-3-cyano-benzoyl-1,4-cis-Achc-Gln-Trp-Ala-Val-Gly-His(3Me)-Sta-Leu-NH2,
la-5 4-(Trimethylammonium)-3-cyano-benzoyl-Gln-Trp-Ala-Val-Gly-His(3Me)-Sta-Leu-NH2,
la-6 4-(Trimethylammonium)-3-cyano-benzoyl-AOC-Gln-Trp-Ala-Val-Gly-His(3Me)-Sta-Leu-NH2,
la-7 4-(Trimethylammonium)-3-cyano-benzoyl-Ava-Gln-Trp-Ala-Val-NMeGly-His(3Me)-Sta-Cpa-NH2,
la-8 4-(Trimethylammonium)-3-cyano-benzoyl-Ava-Gln-Trp-Ala-Val-Gly-His(3Me)-4-Am,5-MeHpA-Leu-NH2,
la-9 4-(Trimethylammonium)-3-cyano-benzoyl-Ava-Gln-Trp-Ala-Val-Gly-His(3Me)-Sta-Leu-NH2,
la-10 4-(Trimethylammonium)-3-cyano-benzoyl-Lys(Me)2-βAla-Gln-Trp-Ala-Val-Gly-His(3Me)-Sta-Leu-NH2,
la-11 4-(Trimethylammonium)-3-cyano-benzoyl-Lys(Me)2-βAla-Gln-Trp-Ala-Val-Gly-His(3Me)-Sta-Leu-NH2,
la-12 4-(Trimethylammonium)-3-cyano-benzoyl-Arg-Ser-Gln-Trp-Ala-Val-Gly-His(3Me)-4-Am,5-MeHpA-Leu-NH2,
la-13 4-(Trimethylammonium)-3-cyano-benzoyl-Ser-Ser-Gln-Trp-Ala-Val-Gly-His(3Me)-4-Am,5-MeHpA-Leu-NH2,
la-14 4-(Trimethylammonium)-3-cyano-benzoyl-Lys(Me)2-Ser-Gln-Trp-Ala-Val-Gly-His(3Me)-4-AM-5-MeHpA-Leu-NH2,
la-15 4-(Trimethylammonium)-3-cyano-benzoyl-Arg-Ser-Gln-Trp-Ala-Val-Gly-His(3Me)-Sta-Leu-NH2,
la-16 4-(Trimethylammonium)-3-cyano-benzoyl-Lys(Me)2-βAla-Gln-Trp-Ala-Val-Gly-His(3Me)-4-Am,5-MeHpA-Leu-NH2,
la-17 4-(Trimethylammonium)-3-cyano-benzoyl-Ava-Gln-Trp-Ala-Val-Gly-His-4-Am,5-MeHpA- -Leu-NH2,
la-18 4-(Trimethylammonium)-3-trifluoromethyl-benzoyl-Arg-Ava-Gln-Trp-Ala-Val-Gly-His(3Me)-Sta-Leu-NH2,
la-19 4-(Trimethylammonium)-3-trifluoromethyl-benzoyl-Arg-Ava-Gln-Trp-Ala-Val-NMeGly-His(3Me)-Sta-Leu-NH2,
la-20 4-(Trimethylammonium)-3-trifluoromethyl-benzoyl-1,4-cis-Achc-Gln-Trp-Ala-Val-Gly-His(3Me)-Sta-Leu-NH2,
la-21 4-(Trimethylammonium)-3-trifluoromethyl-benzoyl-Gln-Trp-Ala-Val-Gly-His(3Me)-Sta-Leu-N H2,
la-22 4-(Trimethylammonium)-3-trifluoromethyl-benzoyl-Arg-βAla-Gln-Trp-Ala-Val-Gly-His(3Me)-4-Am,5-MeHpA-Leu-NH2,
la-23 4-(Trimethylammonium)-3-cyano-benzoyl-Ava-Gln-Trp-Ala-Val-NMeGly-His(3Me)-4-Am,5-MeHpA-Cpa-NH2,
la-24 4-(Trimethylammonium)-3-cyano-benzoyl-Ser-Ser-Gln-Trp-Ala-Val-Gly-His(3Me)-Sta-Leu-NH2,
la-25 4-(Trimethylammonium)-3-cyano-benzoyl-DOA-Gln-Trp-Ala-Val-Gly-His(3Me) Sta-Leu-NH2,

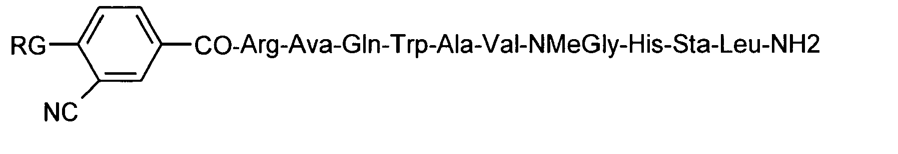

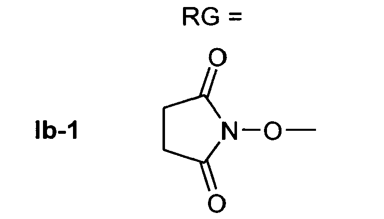

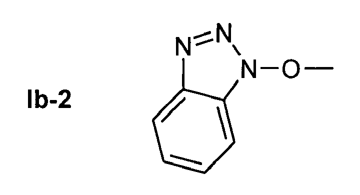

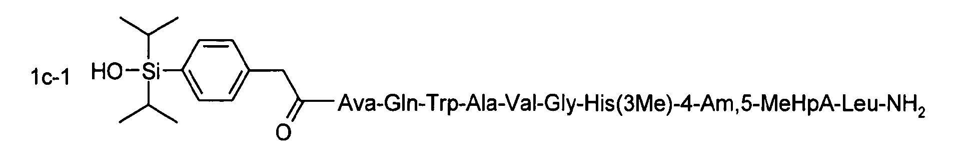

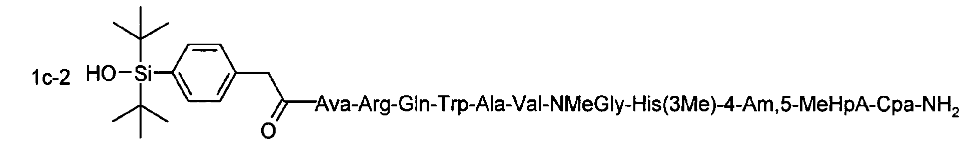

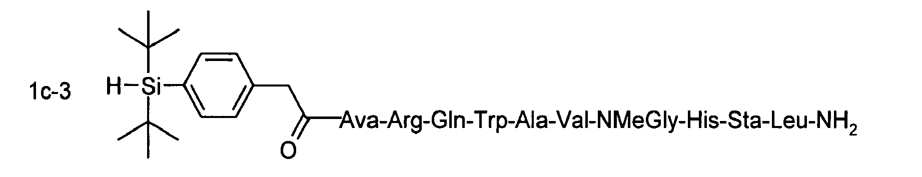

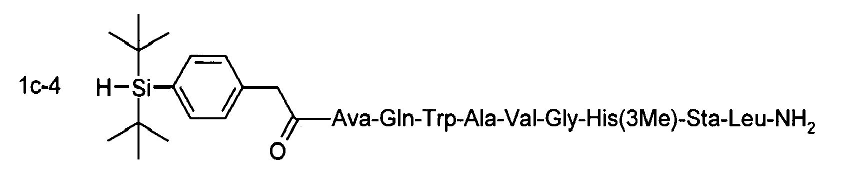

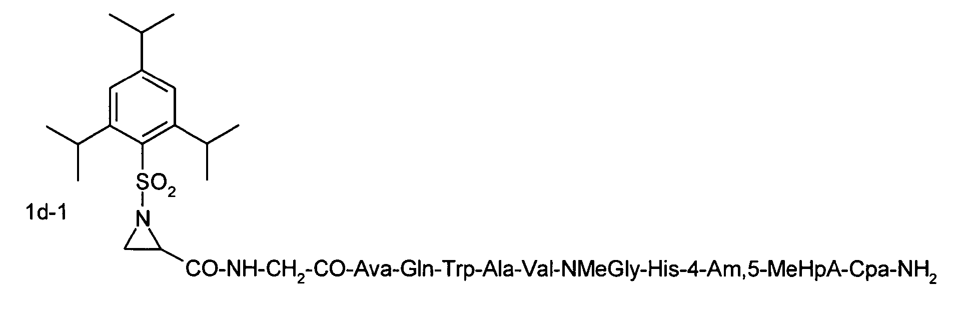

In a preferred embodiment, the precursor for a single step radiolabeling is selected from the following list wherein E is somatostatin analogs:
1a-66: 4-(Trimethylammonium)-3-cyano-benzoyl- Ava-ε-c[Lys-(NMe)Phe-1Nal-D-Trp-Lys-Thr]
1a-67: 4-(Trimethylammonium)-3-cyano-benzoyl- Ava-β-c[Dpr-Met-(NMe)Phe-Tyr-D-Trp-Lys]

In a preferred embodiment, the precursor for a single step radiolabeling is selected from the following list wherein E is neuropeptide Y₁ analogs:
1 a-68: 4-(Trimethylammonium)-3-cyano-benzoyl- Ava-DCys-Leu-Ile-Thr-Arg-Cys-Arg-Tyr-NH₂]
1 a-69: 4-(Trimethylammonium)-3-cyano-benzoyl- Ava-DCys-Leu-Ile-Val-Arg-Cys-Arg-Tyr-NH₂]

In a second aspect, the invention relates radiopharmaceutical labelled with fluorine of formula (II)

F--L₂--B₂--Y--E (II)

wherein
F is fluorine isotope
L₂ is a moiety group or bond to which F is attached
B₂ is a functional group or chain containing functional group connecting L₂ with the spacer Y
Y is a bond or spacer
E is a peptide or peptidomimetic
and pharmaceutically acceptable salts of inorganic or organic acids thereof.

In a preferred embodiment F is ¹⁸F or ¹⁹F.
More preferably, when F is ¹⁸F then radiopharmaceutical labelled with fluorine is of formula IIA.

[18]F--L₂--B₂--Y--E (IIA)

More preferably, when F is ¹⁹F then radiopharmaceutical labelled with fluorine is of formula IIB.

[19]F--L₂--B₂--Y--E (IIB)

L₂ is the linkage moiety between the fluorine isotope and the peptide.
L₂ is a moiety group or bond to which F is attached as described for L₁ when RG is a). In such embodiment for L₂ is identical to for L₁.
L₂ is a moiety group or bond to which F is attached as described for L₁ when RG is b). In such embodiment for L₂ is identical to for L₁.
L₂ is a moiety group or bond to which F is attached as described for L₁ when RG is c). In such embodiment for L₂ is identical to for L₁.

L₂ is α-(substituted)amino-ethyl to which F is attached at β-position when RG is d), J and W are defined above

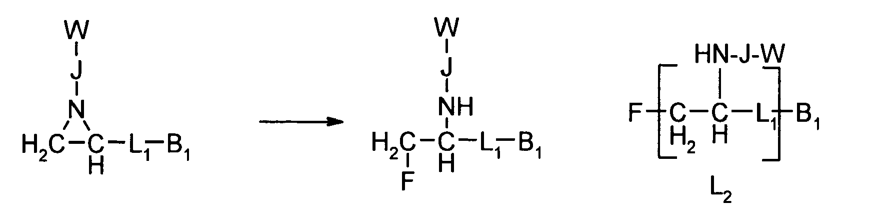

In a more preferred embodiment L₂ is identical to L1 when RG is a) b), or c) or L₂ is 1-[(substituted)amino]ethyl when RG is d).
More preferably, L₂ is α-(substituted)amino-ethyl to which F is attached at β-position when RG is d), J and W are defined above

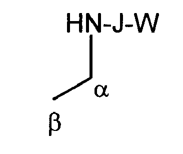

In a more preferred embodiment, B2 of formula II is identical to B1 of formula I and preferred embodiment.

In a more preferred embodiment, Y of formula II is identical to Y of formula I and preferred embodiment.

In a more preferred embodiment, E of formula II is identical to E of formula I and preferred embodiment. More preferably, E is bombesin or bombesin analogs having the sequences listed above. More preferably, E is somatostatin or somatostatin analogs having the sequences listed above. More preferably, E is neuropeptide Y₁ or neuropeptide Y₁ analogs having the sequences listed above.

In a preferred embodiment, the radiopharmaceutical labelled with fluorine is selected from the following list wherein E is bombesin analog:
IIA-a-1 4-[18]Fluoro-3-cyano-benzoyl-Arg-Ava-Gln-Trp-Ala-Val-NMeGly-His-Sta-Leu-NH2,
IIA-a-2 4-[18]Fluoro-3-cyano-benzoyl-Arg-Ava-Gln-Trp-Ala-Val-His(Me)-Sta-Leu-NH2,
IIA-a-3 4-[18]Fluoro-3-cyano-benzoyl-Arg-Ava-Gln-Trp-Ala-Val-NMeGly-His(3Me)-Sta-Leu-NH2,
IIA-a-4 4-[18]Fluoro-3-cyano-benzoyl-1,4-cis-Achc-Gln-Trp-Ala-Val-Gly-His(3Me)-Sta-Leu-NH2,
IIA-a-5 4-[18]Fluoro-3-cyano-benzoyl-Gln-Trp-Ala-Val-Gly-His(3Me)-Sta-Leu-NH2,
IIA-a-6 4-[18]Fluoro-3-cyano-benzoyl-AOC-Gln-Trp-Ala-Val-Gly-His(3Me)-Sta-Leu-NH2,
IIA-a-7 4-[18]Fluoro-3-cyano-benzoyl-Ava-Gln-Trp-Ala-Val-NMeGly-His(3Me)-Sta-Cpa-NH2,
IIA-a-8 4-[18]Fluoro-3-cyano-benzoyl-Ava-Gln-Trp-Ala-Val-Gly-His(3Me)-FA4-Am,5-MeHpA-Leu-NH2,
IIA-a-9 4-[18]Fluoro-3-cyano-benzoyl-Ava-Gln-Trp-Ala-Val-Gly-His(3Me)-Sta-Leu-NH2,
IIA-a-10 4-[18]Fluoro-3-cyano-benzoyl-Lys(Me)2-βAla-Gln-Trp-Ala-Val-Gly-His(3Me)-Sta-Leu-NH2,
IIA-a-11 4-[18]Fluoro-3-cyano-benzoyl-Lys(Me)2-βAla-Gln-Trp-Ala-Val-Gly-His(3Me)-Sta-Leu-NH2,
IIA-a-12 4-[18]Fluoro-3-cyano-benzoyl-Arg-Ser-Gln-Trp-Ala-Val-Gly-His(3Me)-4-Am,5-MeHpA-Leu-NH2,
IIA-a-13 4-[18]Fluoro-3-cyano-benzoyl-Ser-Ser-Gln-Trp-Ala-Val-Gly-His(3Me)-4-Am,5-MeHpA-Leu-NH2,
IIA-a-14 4-[18]Fluoro-3-cyano-benzoyl-Lys(Me)2-Ser-Gln-Trp-Ala-Val-Gly-His(3Me)-4-Am,5-MeHpA-Leu-NH2,
IIA-a--15 4-[18]Fluoro-3-cyano-benzoyl-Arg-Ser-Gln-Trp-Ala-Val-Gly-His(3Me)-Sta-Leu-NH2,
IIA-a-16 4-[18]Fluoro-3-cyano-benzoyl-Lys(Me)2-βAla-Gln-Trp-Ala-Val-Gly-His(3Me)-4-Am,5-MeHpA-Leu-NH2,
IIA-a-17 4-[18]Fluoro-3-cyano-benzoyl-Ava-GIn-Trp-Ala-Val-Gly-His-4-Am,5-MeHpA-Leu-NH2,
IIA-a-18 4-[18]Fluoro-3-trifluoromethyl-benzoyl-Arg-Ava-Gln-Trp-Ala-Val-Gly-His(3Me)-Sta-Leu-NH2,
IIA-a-19 4-[18]Fluoro-3-trifluoromethyl-benzoyl-Arg-Ava-Gln-Trp-Ala-Val-NMeGly-His(3Me)-Sta-Leu-NH2,
IIA-a-20 4-[18]Fluoro-3-trifluoromethyl-benzoyl-1,4-cis-Achc-Gln-Trp-Ala-Val-Gly-His(3Me)-Sta-Leu-NH2,
IIA-a-21 4-[18]Fluoro-3-trifluoromethyl-benzoyl-Gln-Trp-Ala-Val-Gly-His(3Me)-Sta-Leu-NH2 ,
IIA-a-22 4-[18]Fluoro-3-trifluoromethyl-benzoyl-Arg-βAla-Gln-Trp-Ala-Val-Gly-His(3Me)-4-Am,5-MeHpA-Leu-NH2,
IIA-c-2: ¹⁸F-Si(tBu)₂-C₆H₄-CH₂-CO-Ava--Gln-Trp-Ala-Val-NMeGly-His(3Me)-4-Am,5-MeHpA-Cpa-NH2,
IIB-c-1: ¹⁹F-Si(iPr)₂-C₆H₄-CH₂-CO-Ava--Gln-Trp-Ala-Val-Gly-His(3Me)-4-Am,5-MeHpA-Leu-NH2,
IIB-c-2: ¹⁹F-Si(tBu)₂-C₆H₄-CH₂-CO-Ava--Gln-Trp-Ala-Val-NMeGly-His(3Me)-4-Am,5-MeHpA-Cpa-NH2,
IIB-a-1 4-[19]-Fluoro-3-cyano-benzoyl-Arg-Ava-Gln-Trp-Ala-Val-N MeGly-His-Sta-Leu-NH2,
IIB-a--2 4-[19]-Fluoro-3-cyano-benzoyl-Arg-Ava-Gln-Trp-Ala-Val-His(Me)-Sta-Leu-NH2,
IIB-a-3 4-[19]-Fluoro-3-cyano-benzoyl-Arg-Ava-Gin-Trp-Ala-Val-NMeGly-His(3Me)-Sta-Leu-NH2,
IIB-a-4 4-[19]-Fluoro-3-cyano-benzoyl-1,4-cis-Achc-Gln-Trp-Ala-Val-Gly-His(3Me)-Sta-Leu-NH2,
IIB-a-5 4-[19]-Fluoro-3-cyano-benzoyl-Gln-Trp-Ala-Val-Gly-His(3Me)-Sta-Leu-NH2,
IIB-a-6 4-[19]-Fluoro-3-cyano-benzoyl-AOC-Gln-Trp-Ala-Val-Gly-His(3Me)-Sta-Leu-NH2,,
IIB-a-7 4-[19]-Fluoro-3-cyano-benzoyl-Ava-Gln-Trp-Ala-Val-NMeGly-His(3Me)-Sta-Cpa-NH2,
IIB-a-8 4-[19]-Fluoro-3-cyano-benzoyl-Ava-Gln-Trp-Ala-Val-Gly-His(3Me)-4-Am,5-MeHpA-Leu-NH2,
IIB-a-9 4-[19]-Fluoro-3-cyano-benzoyl-Ava-Gln-Trp-Ala-Val-Gly-His(3Me)-Sta-Leu-NH2,
IIB-a-10 4-[19]-Fluoro-3-cyano-benzoyl-Lys(Me)2-βAla-Gln-Trp-Ala-Val-Gly-His(3Me)-Sta-Leu-NH2,
IIB-a-11 4-[19]-Fluoro-3-cyano-benzoyl-Lys(Me)2-βAla-Gln-Trp-Ala-Val-Gly-His(3Me)-Sta-Leu-NH2,
IIB-a-12 4-[19]-Fluoro-3-cyano-benzoyl-Arg-Ser-Gln-Trp-Ala-Val-Gly-His(3Me)-4-Am,5-MeHpA-Leu-NH2,
IIB-a-13 4-[19]-Fluoro-3-cyano-benzoyl-Ser-Ser-Gln-Trp-Ala-Val-Gly-His(3Me)-4-Am,5-MeHpA-Leu-NH2,
IIB-a-14 4-[19]-Fluoro-3-cyano-benzoyl-Lys(Me)2-Ser-Gln-Trp-Ala-Val-Gly-His(3Me)-4-Am,5-MeHpA-Leu-NH2,
IIB-a-15 4-[19]-Fluoro-3-cyano-benzoyl-Arg-Ser-Gln-Trp-Ala-Val-Gly-His(3Me)-Sta-Leu-NH2,
IIB-a-16 4-[19]-Fluoro-3-cyano-benzoyl-Lys(Me)2-βAla-Gln-Trp-Ala-Val-Gly-His(3Me)-4-Am,5-MeHpA-Leu-NH2,
IIB-a-17 4-[19]-Fluoro-3-cyano-benzoyl-Ava-Gln-Trp-Ala-Val-Gly-His-4-Am,5-MeHpA-Leu-NH2,
IIB-a-18 4-[19]-Fluoro-3-trifluoromethyl-benzoyl-Arg-Ava-Gln-Trp-Ala-Val-Gly-His(3Me)-Sta-Leu-NH2,
IIB-a-19 4-[19]-Fluoro-3-trifluoromethyl-benzoyl-Arg-Ava-Gln-Trp-Ala-Val-NMeGly-His(3Me)-Sta-Leu-NH2,
IIB-a-20 4-[19]-Fluoro-3-trifluoromethyl-benzoyl-1,4-cis-Achc-Gln-Trp-Ala-Val-Gly-His(3Me)-Sta-Leu-NH2,
IIB-a-21 4-[19]-Fluoro-3-trifluoromethyl-benzoyl-Gln-Trp-Ala-Val-Gly-His(3Me)-Sta-Leu-NH2,
IIB-a-22 4-[19]-Fluoro-3-trifluoromethyl-benzoyl-Arg-βAla-Gln-Trp-Ala-Val-Gly-His(3Me)-4-Am,5-MeHpA-Leu-NH2,
IIB-a-23 4-[19]-Fluoro-3-cyano-benzoyl-Ava-Gln-Trp-Ala-Val-NMeGIy-His(3Me)-4-Am,5-MeHpA-Cpa-NH2,
IIB-a-24 4-[19]-Fluoro-3-cyano-benzoyl -Ser-Ser-Gln-Trp-Ala-Val-Gly-His(3Me)-Sta-Leu-NH2 ,
IIB-a-25 4-[19]-Fluoro-3-cyano-benzoyl-DOA-Gln-Trp-Ala-Val-Gly-His(3Me)-Sta-Leu-NH2,
IIB-a-26 3,4-[19]-Difluorobenzoyl-Ava-Gln-Trp-Ala-Val-NMeGIy-His-Sta-Leu-NH2,
IIB-a-27 3,4-[19]-Difluorobenzoyl-Ava-Gln-Trp-Ala-Val-NMeGly-His-FA02010-Cpa-NH2,
IIB-a-28 3,4-[19]-Difluorobenzoyl-Ava-Gln-Trp-Ala-Val-NMeGly-His-4-Am,5-MeHpA-tbuGly-NH2,
IIB-a-29 3,4-[19]-Difluorobenzoyl-Ava-Gln-Trp-Ala-Val-NMeGly-His(3Me)-Sta-Leu-NH2,
IIB-a-30 3,4-[19]-Difluorobenzoyl-Ava-Gln-Trp-Ala-Val-NMeGly-His(3Me)-StatBuGly-NH2,
IIB-a-31 3,4-[19]-Difluorobenzoyl-Ava-Gln-Trp-Ala-Val-Gly-His(3Me)-Sta-Leu-NH2,
IIB-a-32 3,4-[19]-Difluorobenzoyl-Ava-Gln-Trp-Ala-Val-NMeGly-His(3Me)-4-Am,5-MeHpA-Leu-NH2,
IIB-a-33 3,4-[19]-Difluorobenzoyl-Ava-Gln-DTrp-Ala-Val-Gly-His-4-Am,5-MeHpAtbuGly-NH2,
IIB-a-34 3,4-[19]-Difluorobenzoyl-Ava-Gln-DTrp-Ala-Val-Gly-His-4-Am-5-MeHxA-Cpa-NH2,
IIB-a-35 3,4-[19]-Difluorobenzoyl-Ava-Gln-Trp-Ala-Val-NMeGIy-His(3Me)-Sta-Cpa-NH2,
IIB-a-36 3,4-[19]-Difluorobenzoyl-Ava-Gln-DTrp-Ala-Val-Gly-His-Sta-tbuAla-NH2,
IIB-a-37 3,4-[19]-Difluorobenzoyl-Arg-Ava-GIn-Trp-Ala-Val-NmeGly-His-Sta-Leu-NH2,
IIB-a-38 3,4-[19]-Difluorobenzoyl-Gln-Trp-Ala-Val-Gly-His(3Me)-Sta-Leu-NH2,
IIB-a-39 3,4-[19]-Difluorobenzoyl-Arg-Ava-Gln-Trp-Ala-Val-NMeGIy-His(3Me)-Sta-Leu-NH2,
IIB-a-40 3,4-[19]-Difluorobenzoyl-Arg-Ava-Gln-Trp-Ala-Val-Gly-His(3Me)-Sta-Leu-NH2,
IIB-a-41 3,4-[19]-Difluorobenzoyl-Arg-βAla-Arg-Gln-Trp-Ala-Val-Gly-His(3Me)-Sta-Leu-NH2,
IIB-a-42 3,4-[19]-Difluorobenzoyl-Ava-Gln-Trp-Ala-Val-Gly-His(3Me)-Sta-Cpa-NH2,
IIB-a-43 3,4-[19]-Difluorobenzoyl-Ava-Gln-Trp-Ala-Val-Gly-His(3Me)-Sta-tBuGly-NH2,
IIB-a-44 3,4-[19]-Difluorobenzoyl-Arg-Arg-Gln-Trp-Ala-Val-NMeGly-His(3Me)-Sta-Leu-NH2,
IIB-a-45 3,4-[19]-Difluorobenzoyl-Arg-βAla-Gln-Trp-Ala-Val-NMeGly-His(3Me)-Sta-Leu-NH2 ,
IIB-a-46 3,4-[19]-Difluorobenzoyl-Gln-Trp-Ala-Val-Gly-His(3Me)-4-Am,5-MeHpA-Leu-NH2,
IIB-a-47 3,4-[19]-Difluorobenzoyl-Ava-Gln-Trp-Ala-Val-NMeGIy-His(3Me)-4-Am,5-MeHpA-Cpa-NH2,
IIB-a-48 3,4-[19]-Difluorobenzoyl-Ava-Gln-Trp-Ala-Val-Gly-His(3Me)-4-Am,5-MeHpA-Leu-NH2,
IIB-a-49 3,4-[19]-Difluorobenzoyl-Ava-Gln-Trp-Ala-Val-Gly-NMeHis-4-Am,5-MeHpA-Cpa-NH2,
IIB-a-49 3,4-[19]-Difluorobenzoyl-Ava-Gln-Trp-Ala-Val-Gly-NMeHis(3Me)-4-Am,5-MeHpA-Leu-NH2,
IIB-a-50 3,4-[19]-Difluorobenzoyl-Ava-Gln-Trp-Ala-Val-Gly-NMeHis-4-Am,5-MeHpA-Leu-NH2,
IIB-a-51 3,4-[19]-Difluorobenzoyl-Ava-Gln-Trp-Ala-Val-NMeGly-Hls-AHMHxA - Leu-NH2,
IIB-a-52 3,4-[19]-Difluorobenzoyl-Ava-Gln-Trp-Ala-Val-βAla-NMeHis-Tha-Cpa-NH2,
IIB-a-53 3,4-[19]-Difluorobenzoyl-Ava-Gln-Trp-Ala-Val-βAla-NMeHis-Phe-Cpa-NH2,
IIB-a-54 3,4-[19]-Difluorobenzoyl-Ava-Gln-Trp-Ala-Val-βAla-NMeHis-Phe-Leu-NH2,
IIB-a-55 3,4-[19]-Difluorobenzoyi-Ava-Gln-Trp-Ala-Val-βAla-DHis-Phe-Leu-NH2,
IIB-a-56 3,4-[19]-Difluorobenzoyl-Ava-Gln-Trp-Ala-Val-βAla-His-ßhLeu-Leu-NH2,
IIB-a-57 3,4-[19]-Difluorobenzoyl-Ava-Gln-Trp-Ala-Val-βAla-His-ßhlle-Leu-NH2,
IIB-a-58 3,4-[19]-Difluorobenzoyl-Ava-Gln-Trp-Ata-Val-βAta-His-ßhLeu-tbuGly-NH2,
IIB-a-59 3,4-[19]-Difluorobenzoyl-Ava-Gln-Trp-Ala-Val-βAla-His(3Me)-Phe-Tha-NH2,
IIB-a-60 3,4-[19]-Difluorobenzoyl-Ava-Gln-Trp-Ala-Val-βAla-His(3Me)-Phe-Nle-NH2,
IIB-a-51 3,4-[19]-Difluorobenzoyl-Ava-Gin-Trp-Ala-Val-βAla-NMeHis-Phe-tbuGly-NH2,
IIB-a-52 3,4-[19]-Difluorobenzoyl-Ava-Gln-Trp-Ala-Val-βAla-NMeHis-Tha-tbuGly-NH2,
IIB-a-53 3,4-[19]-Difluorobenzoyl-Ava-Gln-Trp-Ala-Val-βAla-His(3Me)-Tha-tbuGly-NH2,
IIB-a-54 3,4-[19]-Difluorobenzoyl-Ava-Gln-Trp-Ala-Val-βAla-His(3Me)-Phe-Cpa-NH2,
IIB-a-55 3,4-[19]-Diftuorobenzoyl-Ava-Gln-Trp-Ala-NMeVal-βAla-His-Phe-Leu-NH2,
IIB-a-56 3,4-[19]-Difluorobenzoyl-Ava-GIn-Trp-Ala-Val-βAla-His-NmePhe-Leu-NH2,
IIB-a-57 3,4-[19]-Difluorobenzoyl-Ava-Gln-DTrp-Ala-Val-βAla-His-Phe-Leu-NH2,
IIB-a-58 3,4-[19]-Difluorobenzoyl-Ava-Gln-Trp-DAla-Val-βAla-His-Phe-Leu-NH2,
IIB-a-59 3,4-[19]-Difluorobenzoyl-Ava-Gln-Trp-Ala-DVal-βAla-His-Phe-Leu-NH2,
IIB-a-60 3,4-[19]-Difluorobenzoyl-Ava-Gln-Trp-Ala-Val-βAla-His-DPhe-Leu-NH2,
IIB-a-61 3,4-[19]-Difluorobenzoyl-Ava-Gln-Trp-Ala-Val-βAla-His-Bhlle-tbuGly-NH2,
IIB-a-62 4-[19]-Fluoro-3-cyano-phenylsulfonyl -Ava-Gln-Trp-Ala-Val-NMeGly-His-4-Am,5-MeHpA-Cpa-NH2,
IIB-a-63 4-[19]-Fluoro-3-cyano-phenylsulfonyl -Ava-Gln-Trp-Ala-Val-NMeGly-His-Sta-Cpa-NH2,
IIB-a-64 4-[19]-Fluoro-3-cyano-phenylsulfonyl -Ava-Gln-Trp-Ala-Val-NMeGly-His-Sta-tbuAla-NH2 ,
IIB-a-65 4-[19]-Fluoro-3-cyano-phenylsulfonyl -Ava-Gln- Trp-Ala-Val-NMeGly-His-4-Am,5-MeHpA-tbuAla-NH2.

In a preferred embodiment, the radiopharmaceutical labelled with fluorine is selected from the following list wherein E is somatostatin analog:
IIA-a-66: 4-[18]Fluoro-3-cyano-benzoyl- Ava-ε-c[Lys-(NMe)Phe-1Nal-D-Trp-Lys-Thr]
IIA-a-67: 4-[18]Fluoro-3-cyano-benzoyl- Ava-β-c[Dpr-Met-(NMe)Phe-Tyr-D-Trp-Lys]
IIB-a-66: 4-[19]Fluoro-3-cyano-benzoyl- Ava-ε-c[Lys-(NMe)Phe-1Nal-D-Trp-Lys-Thr]
IIB-a-67: 4-[19]Fluoro-3-cyano-benzoyl- Ava-β-c[Dpr-Met-(NMe)Phe-Tyr-D-Trp-Lys]

In a preferred embodiment, the radiopharmaceutical labelled with fluorine is selected from the following list wherein E is neuropeptide Y₁ analog:
IIA-a-68: 4-[18]Fluoro-3-cyano-benzoyl- Ava-DCys-Leu-Ile-Thr-Arg-Cys-Arg-Tyr-NH₂
IIA-a-69: 4-[18]Fluoro-3-cyano-benzoyl- Ava- DCys-Leu-Ile-Val-Arg-Cys-Arg-Tyr-NH₂
IIA-a-68: 4-[19]Fluoro-3-cyano-benzoyl- Ava-DCys-Leu-Ile-Thr-Arg-Cys-Arg-Tyr-NH₂
IIA-a-69: 4-[19]Fluoro-3-cyano-benzoyl- Ava- DCys-Leu-Ile-Val-Arg-Cys-Arg-Tyr-NH₂

In a third aspect, the invention relates to compound of the formula II for use as a positron emitting tomography (PET) diagnostic agent. More preferably, the invention relates to compound of the formula IIA for use as a positron emitting tomography (PET) diagnostic agent, wherein the fluorine isotope is ¹⁸F. More preferably, the invention relates to the use of compound according to the formula (I) for the manufacture of formula (IIA) as a diagnostic agent. Most preferably, the use is for imaging of tumors, imaging of inflammatory and/or neurodegenerative diseases, such as multiple sclerosis or Alzheimer's disease, or imaging of angiogenesis-associated diseases, such as growth of solid tumors, and rheumatoid arthritis.

In a fourth aspect, the invention relates to compound of the formula II for use in biological assays and chromatographic identification. More preferably, the invention relates to compound of the formula IIB for use in biological assays and chromatographic identification, wherein the fluorine isotope is ¹⁹F. More preferably, the invention relates to the use of compound according to the formula (I) for the manufacture of formula (IIB) as a measurement agent.

In a fifth aspect, the invention relates to a method of radiofluorination of a compound of formula I for the manufacture of compound of formula II comprising the step of reacting a compound of formula I with a fluorination agent..
In a preferred embodiment, the method of radiofluorination occurs at a reaction temperature between room temperature to 80°C.

In a preferred embodiment, the fluorination agent is 4,7,13,16,21,24-Hexaoxa-1,10-diazabicyclo[8.8.8]-hexacosane K18F (crownether salt Kryptofix K18F), K¹⁸F, H¹⁸F, KH¹⁸F₂ or tetraalkylammonium salt of ¹⁸F. More preferably, fluorination agent is K¹⁸F, H¹⁸F, or KH¹⁸F₂.
a new method is warranted in which the final product is prepared in a single step from the precursor. Only one purification step is necessary thereby the preparation can be accomplished in a short time (considering the half-life of ¹⁸F). In a typical prosthetic group preparation, very often temperatures of 100°C and above are employed. The invention provides methods to accomplish the preparation at temperatures (80°C or below) that preserve the biological properties of the final product. Additionally, single purification step is optionally carried out thereby the preparation can be accomplished in a short time (considering the half-life of ¹⁸F).
¹⁸F-fluoride (up to 40GBq) was azeotropically dried in the presence of Kryptofix 222 (5mg in 1.5ml MeCN) and cesium carbonate (2,3mg in 0.5m) water) by heating under a stream of nitrogen at 110-120°C for 20-30 minutes. During this time 3 x 1ml MeCN were added and evaporated. After drying, a solution of the precursor (2 mg) in 150µl DMSO was added. The reaction vessel was sealed and heated at 50-70°C for 5-15mins to effect labeling. The reaction was cooled to room temperature and dilute with water (2.7ml). The crude reaction mixture was analyzed using an analytical HPLC. The product was obtained by preparative radio HPLC to give to desired ¹⁸F labeled peptide.

In a sixth aspect, the invention relates to a kit comprising a compound of the formula (I) as disclosed above along with an acceptable diluent, excipient or adjuvant supplied as a mixture with the compound of the formula (I) or independently for the manufacture of (II). More preferably, the present invention relates to a kit comprising a compound or composition, as defined herein above, in powder form, and a container containing an appropriate solvent for preparing a solution of the compound or composition for administration to an animal, including a human.

In a seventh aspect, the invention relates to bombesin analogs that binds specifically to human GRP receptors present in prostate tumor, breast tumor and metastasis. In a preferred embodiment, the bombesin analog is a peptide of sequence from Seq ID 1 to Seq ID 103 and preferably one of them. More preferably the bombesin analog is additionally radiolabeled with a fluorine isotope (F) wherein F is ¹⁸F or ¹⁹F. More preferably the bombesin analog is radiolabeled using the radiofluorination method of the present invention.

As used herein, the term "alkyl", by itself or as part of another group, refers to a straight chain or branched chain alkyl group with 1 to 20 carbon atoms such as, for example methyl, ethyl, propyl, isopropyl, butyl, isobutyl, tert-butyl, pentyl, isopentyl, neopentyl, heptyl, hexyl, decyl. Alkyl groups can also be substituted, such as by halogen atoms, hydroxyl groups, C1-C4-alkoxy groups or C6-C12-aryl groups (which, intern, can also be substituted, such as by 1 to 3 halogen atoms). "Alkenyl" and "alkinyl" are similarly defined, but contain at least one carbon-carbon double or triple bond, respectively.

The term "inorganic acid" and "organic acid" as employed herein refers to mineral acids, including but not limited to: acids such as carbonic, nitric, phosphoric, hydrochloric, perchloric or sulphuric acid or acidic salts such as potassium hydrogen sulphate or to appropriate organic acids which includes but not limited to: acids such as aliphatic, cycloaliphatic, aromatic, araliphatic, heterocyclic, carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic, trifluormethansulfonic and sulfanilic acid.

As used herein, the term "aryl", by itself or as part of another group, refers to monocyclic or bicyclic aromatic groups containing from 6 to 12 carbons in the ring portion, preferably 6-10 carbons in the ring portion, such as phenyl, naphthyl or tetrahydronaphthyl.

As used herein, the term "heteroaryl", by itself or as part of another group, refers to groups having 5 to 14 ring atoms; 6, 10 or 14 Π electrons shared in a cyclic array; and containing carbon atoms and 1, 2, 3 or 4 oxygen, nitrogen or sulfur heteroatoms Examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxythiinyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, 4aH-carbazolyl, carbazolyl, carbolinyl, phenanthridinyl, acridinyl, perimidinyl, phenanthrolinyl, phenazinyl, isothiazolyl, phenothiazinyl, isoxazolyl, furazanyl und phenoxazinyl.

As used herein, the term "peptide" refers to a sequence of 4 to 100 amino acids.

As used herein, the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" within the above definition.

For the purpose of the specification, an amino acid sequence may comprise naturally occurring and/or synthetic amino acid residues, proteinogenic and/or non-proteinogenic amino acid residues. The non-proteinogenic amino acid residues may be further classified as (a) homoanalogues of proteinogenic amino acids, (b) α-homoanalogues of proteinogenic amino acid residues and (c) further non-proteinogenic amino acid residues.

Accordingly, the amino acid residues are derived from the corresponding amino acids, e.g. from
proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or non-proteinogenic amino acids, such as homoanalogues of proteinogenic amino acids wherein the side chain has been extended by a methylene group, e.g. homoalanine (Hal), homoarginine (Har), homocysteine (Hcy), homoglutamine (Hgl), homohistidine (Hhi), homo isoleucine (Hil), homoleucine (Hie), homolysine (Hly), homomethionine (Hme), homophenylnine (Hph), homoproline (Hpr), homoserine (Hse), homothreonine (Hth), homotryptophane (Htr), homotyrosine (Hty) and homovaline (Hva); β-homoanalogues of proteinogenic amino acids wherein a methylene group has been inserted between the α-carbon and the carboxyl group yielding β-amino acids, e.g. β-homoalanine (βHal), β-homoarginine (βHar), β-homoasparagine (βHas), β-homocysteine (βHcy), β-homoglutamine (βHgl), β-homohistidine (βHhi), β-homoisoleucine (βHil), β-homoleucine (βHle), β-homolysine (βHly), β-homomethionine (βHme), β-homophenylalanine (βHph), β-homoproline (βHpr), β-homoserine (βHse), β-homothreonine (βHth), β-homotryptophane (βHtr), β-homotyrosine (βHty) and β-homovaline (βHva);
further non-proteinogenic amino acids, e.g. α-aminoadipic acid (Aad), β-aminoadipic acid (βAad), α-aminobutyric acid (Abu), α-aminoisobutyric acid (Aib), β-alanine (βAla), 4-aminobutyric acid (4-Abu), 5-aminovaleric acid (5-Ava), 6-aminohexanoic acid (6-Ahx), 8-aminooctanoic acid (8-Aoc), 9-aminononanoic acid (9-Anc), 10-aminodecanoic acid (10-Adc), 12-aminododecanoic acid (12-Ado), α-aminosuberic acid (Asu), azetidine-2-carboxylic acid (Aze), β-cyclohexylalanine (Cha), citrulline (Cit), dehydroalanine (Dha), γ-carboxyglutamic acid (Gla), α-cyclohexylglycine (Chg), propargylglycine (Pra), pyroglutamic acid (Glp), α-tert-butylglycine (Tle), 4-benzoylphenylalanine (Bpa), δ-hydroxylysine (Hyl), 4-hydroxyproline (Hyp), allo-isoleucine (alle), lanthionine (Lan), (1-naphthyl)alanine (1-Nal), (2-naphthyl)alanine (2-Nal), norleucine (Nle), norvaline (Nva), ornithine (Orn), phenylglycin (Phg), pipecolic acid (Pip), sarcosine (Sar), selenocysteine (Sec), statine (Sta), β-thienylalanine (Thi), 1,2,3,4-tetrahydroisochinoline-3-carboxylic acid (Tic), allo-threonine (allo-Thr), thiazolidine-4-carboxylic acid (Thz), γ-aminobutyric acid (GABA), iso-cysteine (iso-Cys), diaminopropionic acid (Dpr), 2,4-diaminobutyric acid (Dab), 3,4-diaminobutyric acid (γ,βDab), biphenylalanine (Bip), phenylalanine substituted in para-position with -C₁-C₆-alkyl, -halide, -NH₂ or -CO₂H (Phe(4-R) wherein R = -C₁-C₆-alkyl, -halide, -NH₂, or -CO₂H); peptide nucleic acids (PNA, cf. P.E. Nielsen, Acc.Chem.Res. 32, 624-30) or their N-alkylated analogues, such as their N-methylated analogues.
Cyclic amino acids may be proteinogenic or non-proteinogenic, such as Pro, Aze, Glp, Hyp, Pip, Tic and Thz.
For further examples and details reference can be made to e.g. J.H. Jones, J. Peptide Sci. 2003, 9, 1-8.

The terms "non-proteinogenic amino acid" and "non-proteinogenic amino acid residue" also encompasses derivatives of proteinogenic amino acids. For example, the side chain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.
For the purpose of the specification, a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

Examples for peptides which can be used as targeting agents in the compounds of the present invention are, but are not limited to, somatostatin and derivatives thereof and related peptides, neuropeptide Y and derivatives thereof and related peptides, bombesin and derivatives thereof and related peptides, gastrin, gastrin releasing peptide derivatives thereof and related peptides, epidermal growth factor (EGF of various origin), insulin growth factor (IGF) and IGF-1, LHRH agonists and antagonists, transforming growth factors, particularly TGF-α, angiotensin, cholecystokinin (CCK) and analogs, neurotensin and analogs, thyrotropin releasing hormone, prolactin, tumor necrosis factor, IL-1, IL-2, IL-4 or IL-6, interferons, VIP and related peptides, PACAP and related peptides. Such peptides comprise from 4 to 100 amino acids, wherein the amino acids are selected from natural and non-natural amino acids and also comprise modified natural and non-natural amino acids.

Peptidomimetic are molecules related to peptides, but with different properties. A peptidomimetic is a small protein-like chain designed to mimic a peptide. They typically arise from modification of an existing peptide in order to alter the molecule's properties. For example, they may arise from modifications to change the molecule's stability or biological activity. This can have a role in the development of drug-like compounds from existing peptides. These modifications involve changes to the peptide that will not occur natural

As used herein, the term "peptide analogs", by itself refers to synthetic or natural compounds which resemble naturally occurring peptides in structure and/or function.

As used herein the term "pharmaceutically acceptable salt" comprises salts of inorganic and organic acids such an mineral acids, including, but not limited to, acids such as carbonic, nitric or sulfuric acid, or organic acids, including, but not limited to acids such as aliphatic, cycloaliphatic, aromatic, araliphatic, heterocyclic, carboxylic and sulphonic acids, examples of which are formic, acetic, trifluoroacetic, propionic, succinic, glycolic, gluconic, lactic, malic, fumaric, pyruvic, benzoic, anthranilic, mesylic, salicylic, phenylacetic, mandelic, embonic, methansulfonic, ethanesulfonic, benzenesulfonic, phantothenic, toluenesulfonic and sulfanilic acid.
If a chiral center or another form of an isomeric center is present in a compound according to Formula I, II or III of the present invention, all forms of such isomers, including enantiomers and diastereoisomers, are intended to be covered herein. Compounds containing a chiral center may be used as a racemic mixture or as an enantiomerically enriched mixture, or the racemic mixture may be separated using well-known techniques and an individual enantiomer maybe used alone. In cases in which compounds have unsaturated carbon-carbon double bonds, both the cis-isomer and trans-isomers are within the scope of this invention. In cases in which compounds may exist in tautomeric forms, such as keto-enol tautomers, each tautomeric form is contemplated as being included within this invention whether existing in equilibrium or predominantly in one form.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

The entire disclosure[s] of all applications, patents and publications, cited herein are incorporated by reference herein.

The following examples can be repeated with similar success by substituting the generically or specifically described reactants and/or operating conditions of this invention for those used in the preceding examples.

From the foregoing description, one skilled in the art can easily ascertain the essential charac- teristics of this invention and, without departing from the spirit and scope thereof, can make various changes and modifications of the invention to adapt it to various usages and conditions.

### General Method for the Preparation of Compounds

The compounds of the present invention are shown below according to formula I.

RG--L₁--B₁--Y--E (I)

These compounds can be synthesized depending on the nature of RG--L₁. The peptide portion of the molecule B₁-Y-E can be conveniently prepared according generally established techniques known in the art of peptide synthesis, such as solid-phase peptide synthesis. They are amenable Fmoc-solid phase peptide synthesis, employing alternate protection and deprotection. These methods are well documented in peptide literature. (Reference: "Fmoc Solid Phase Peptide Synthesis" A practical approach, Edited by W. C. Chan and P. D. White, Oxford University Press 2000) (For Abbreviations see Descriptions)

### Example 1:

### Synthesis of RG--L₁--Y--E (Ia-Id)

Synthesis of H-Y--E: Solid-phase peptide synthesis (SPPS) involves the stepwise addition of amino acid residues to a growing peptide chain that is linked to an insoluble support or matrix, such as polystyrene. The C-terminal residue of the peptide is first anchored to a commercially available support (e.g., Rink amide resin) with its amino group protected with an N-protecting agent, fluorenylmethoxycarbonyl (FMOC) group. The amino protecting group is removed with suitable deprotecting agent such as piperidine for FMOC and the next amino acid residue (in N-protected form) is added with a coupling agents such as dicyclohexylcarbodiimide (DCC), di-isopropyl-cyclohexylcarbodiimide (DCCI), hydroxybenzotriazole (HOBt). Upon formation of a peptide bond, the reagents are washed from the support. After addition of the final residue of (Y), the peptide is attached to the solid support is ready for the coupling of RG--L₁--B₁-OH.

### Synthesis of RG--L₁--B₁--Y--E (la)

la: RG = ⁺N(CH₃)₃, B₁ =CO, X⁻ = CF₃SO₃⁻ 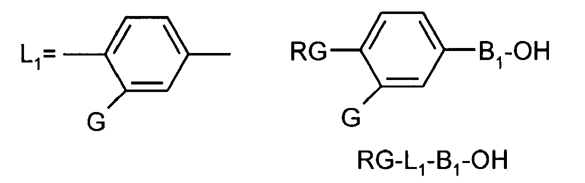 G = CN, CF₃, F and others specified

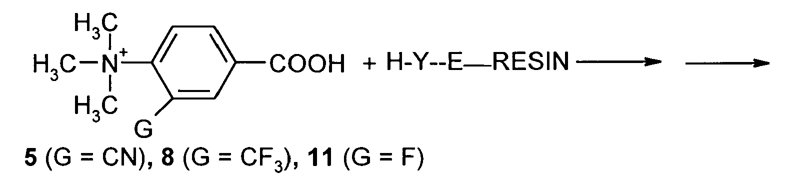

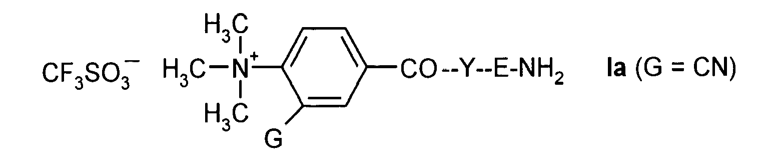

To a suspension of the resin -Y--E--RESIN in DMF (0.1 to 0.25 mmol), RG-L₁-B₁-OH (**5, 8,** or **11,** 2 -4 equivalents) was added along with 4-(4,6-dimethoxy-1,3,5-triazin-2-yl)-4-methylmorpholinium tetrafluoroborate.

Example 2 describes the preparation of **5**, **8** and **11**. After 3-8 hours, the resin washed with DMF and dichloromethane. The peptide la) was isolated from the resin using TFA:diisopropylsilane:phenol:water cocktail with concomitant removal of the protecting groups of amino acids. The product was purified by HPLC using appropriate TFA:H₂O:0.1TFA gradient using C₁₈-reverse phase column. The products were identified mass spectra.
la-1: 4-(Trimethylammonium)-3-cyano-benzoyl-Arg-Ava-Gln-Trp-Ala-Val-NMeGly-His-Sta-Leu-NH2: Molecular wt: Calculated: 1422.73, Found: 711.86 ((M⁺+1)/2)
Table 1 lists all the trimethylammonium compounds precursors la-1 - la-25 used for F-18 labeling

**Table 1**

| | | | |
|---|---|---|---|
| RG ((CH₃)₃N⁺)--L₁--B₁--Y--E (la) (G = 3-cyano, 3-trifluormethyl as indicated; B₁ = O) (RG is a) and X⁻ is triflate) | | | |
| 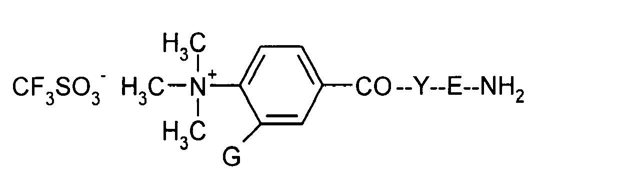 | | | |

| Trimethylammonium--L₁(G)--B₁ | | Y | E |
|---|---|---|---|
| Ia-1 | 4-(Trimethylammonium)-3-cyano-benzoyl-Arg-Ava-Gln-Trp-Ala-Val-NMeGly-His-Sta-Leu-NH2 | | |
| la-2 | 4-(Trimethylammonium)-3-cyano-benzoyl-Arg-Ava-Gln-Trp-Ala-Val-Gly-His(Me)-Sta-Leu-NH2 | | |
| la-3 | 4-(Trimethylammonium)-3-cyano-benzoyl-Arg-Ava-Gln-Trp-Ala-Val-NMeGly-His(3Me)-Sta-Leu-NH2 | | |
| la-4 | 4-(Trimethylammonium)-3-cyano-benzoyl-1,4-cis-Achc-Gln-Trp-Ala-Val-Gly-His(3Me)-Sta-Leu-NH2 | | |
| Ia-5 | 4-(Trimethylammonium)-3-cyano-benzoyl-Gln-Trp-Ala-Val-Gly-His(3Me)-Sta-Leu-NH2 (Y = bond) | | |
| la-6 | 4-(Trimethylammonium)-3-cyano-benzoyl-AOC-Gln-Trp-Ala-Val-Gly-His(3Me)-Sta-Leu-NH2 | | |
| la-7 | 4-(Trimethylammonium)-3-cyano-benzoyl-Ava-Gln-Trp-Ala-Val-NMeGly-His(3Me)-Sta-Cpa-NH2 | | |
| la-8 | 4-(Trimethylammonium)-3-cyano-benzoyl-Ava-Gln-Trp-Ala-Val-Gly-His(3Me)-4-Am,5-MeHpA-Leu-NH2 | | |
| la-9 | 4-(Trimethylammonium)-3-cyano-benzoyl-Ava-Gln-Trp-Ala-Val-Gly-His(3Me)-Sta-Leu-NH2 | | |
| la-10 | 4-(Trimethylammonium)-3-cyano-benzoyl-Lys(Me)2-βAla-Gln-Trp-Ala-Val-Gly-His(3Me)-Sta-Leu-NH2 | | |
| Ia-11 | 4-(Trimethylammonium)-3-cyano-benzoyl-Lys(Me)2-βAla-Gln-Trp-Ala-Val-Gly-His(3Me)-Sta-Leu-NH2 | | |
| la-12 | 4-(Trimethylammonium)-3-cyano-benzoyl-Arg-Ser-Gln-Trp-Ala-Val-Gly-His(3Me)-4-Am,5-MeHpA-Leu-NH2 | | |
| la-13 | 4-(Trimethylammonium)-3-cyano-benzoyl-Ser-Ser-Gln-Trp-Ala-Val-Gly-His(3Me)-4-Am,5-MeHpA-Leu-NH2 | | |
| la-14 | 4-(Trimethylammonium)-3-cyano-benzoyl-Lys(Me)2-Ser-Gln-Trp-Ala-Val-Gly-His(3Me)-4-AM-5-MeHpA-Leu-NH2 | | |
| la-15 | 4-(Trimethylammonium)-3-cyano-benzoyl-Arg-Ser-Gln-Trp-Ala-Val-Gly-His(3Me)-Sta-Leu-NH2 | | |
| la-16 | 4-(Trimethylammonium)-3-cyano-benzoyl-Lys(Me)2-βAla-Gln-Trp-Ala-Val-Gly-His(3Me)-4-Am,5-MeHpA-Leu-NH2 | | |
| la-17 | 4-(Trimethylammonium)-3-cyano-benzoyl-Ava-Gln-Trp-Ala-Val-Gly-His-4-Am,5-MeHpA- -Leu-NH2 | | |
| | | | |
| la-18 | 4-(Trimethylammonium)-3-trifluoromethyl-benzoyl-Arg-Ava-Gln-Trp-Ala-Val-Gly-His(3Me)-Sta-Leu-NH2 | | |
| la-19 | 4-(Trimethylammonium)-3-trifluoromethyl-benzoyl-Arg-Ava-Gln-Trp-Ala-Val-NmeGly-His(3Me)-Sta-Leu-NH2 | | |
| la-20 | 4-(Trimethylammonium)-3-trifluoromethyl-benzoyl-1,4-cis-Achc-Gln-Trp-Ala-Val-Gly-His(3Me)-Sta-Leu-NH2 | | |
| Ia-21 | 4-(Trimethylammonium)-3-trifluoromethyl-benzoyl-Gln-Trp-Ala-Val-Gly-His(3Me)-Sta-Leu-NH2 (Y = bond) | | |
| la-22 | 4-(Thmethylammonium)-3-trifluoromethyl-benzoyl-Arg-βAla-Gln-Trp-Ala-Val-Gly-His(3Me)-4-Am,5-MeHpA-Leu-NH2 | | |
| | | | |
| la-23 | 4-(Trimethylammonium)-3-cyano-benzoyl-Ava-Gln-Trp-Ala-Val-NMeGly-His(3Me)-4-Am,5-MeHpA-Cpa-NH2 | | |
| la-24 | 4-(Trimethylammonium)-3-cyano-benzoyl -Ser-Ser-Gln-Trp-Ala-Val-Gly-His(3Me)-Sta-Leu-NH2 | | |
| la-25 | 4-(Trimethylammonium)-3-cyano-benzoyl-DOA-Gln-Trp-Ala-Val-Gly-His(3Me)-Sta-Leu-NH2 | | |

### Example 2

### Preparation of RG-L₁-B₁-OH 5 (G = CN)

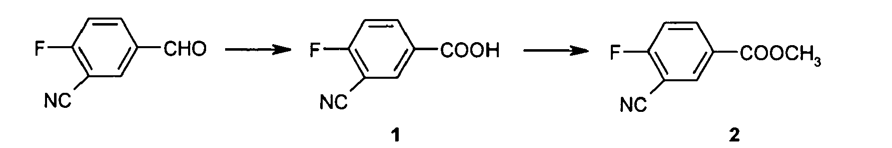

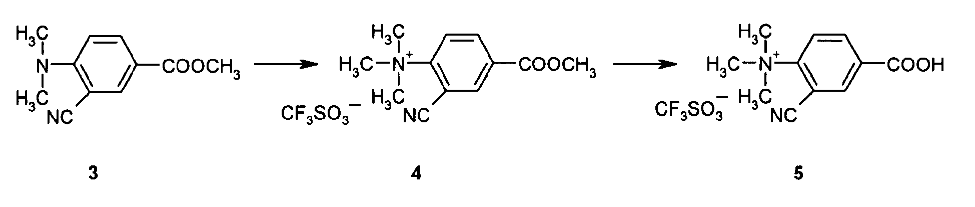

a) Synthesis of 3-Cyano-4-fluoro-benzoic acid: To a stirred solution of 15.0 g (97,6 mmol) 2-fluoro-5-formyl-benzonitrile (Aldrich), 150 ml dest. water and 630 ml t-butanol are added 40.8 g (361 mmol) sodium chlorite and 35.9 g (230 mmol) sodium hydrogen phosphate dihydrate. The reaction mixture is stirred over night and poured into a diluted aqueous hydrogen chloride solution (pH=3.5). The pH value is readjusted to pH=3.5 by aqueous hydrogen chloride. The aqueous solution is extracted trice with dichloromethane/isopropanol (10:1). The combined organic phases are dried (sodium sulfate) and concentrated. The residue is purified by extraction with sodium hydrogen carbonate solution and dichloromethane, acidification with aqueous solution and subsequent filtering. The solid crude product **1** is obtained in 90% yield (14.5 g, 87.8 mmol) and is used for the next step without purification.
MS-ESI: 166 (M⁺ +1, 77),

| Elementary analysis: | | | | |
|---|---|---|---|---|
| Calculated: | C 58.19% | H 2.44% | F 11.51% | N 8.48% |
| Found: | C 58.81% | H 2.42% | F 11.41% | N 8,47% |

b) Synthesis of 3-Cyano-4-fluoro-benzoic acid methyl ester **2**: To a stirred suspension of 16.0 g (96,9 mmol) **1** and 161 ml methanol are added 30.4 g (387,6 mmol) acetyl chloride drop wisely at 0°C. The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **2** is obtained in 78,1 % yield (13.5 g; 75.7 mmol)
MS-ESI: 180 (M⁺ +1, 57),

| Elementary analysis: | | | | |
|---|---|---|---|---|
| Calculated: | C 60.34% | H 3.38% | F 10.60% | N 7.82% |
| Determined: | C 60.51% | H 3.39% | F 10.57% | N 7,80% |

c) Synthesis of 3-Cyano-4-dimethylamino-benzoic acid methyl ester **3**: To a stirred solution of 24.0 g (134 mmol) **2** and 240 ml dimethylsulphoxid are added 13.2 g (161 mmol) dimethylamine hydrochloride and 38,9 g (281 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **3** is obtained in 94% yield (25,7 g, 126 mmol) and is used for the next step without purification.
MS-ESI: 205 (M⁺ +1, 59),

| Elementary analysis: | | | |
|---|---|---|---|
| Calculated: | C 64.69% | H 5.92% | N 13.72% |
| Found: | C 64.79% | H 5,95% | N 13.69% |

d) Synthesis of (2-Cyano-4-methoxycarbonyl-phenyl)-trimethyl-ammonium trifluoro-methanesulfonate **4**: To a stirred solution of 6.16 g (30.2 mmol) **3** and 110 ml dichloromethane are added 50.0 g (302 mmol) methyltriflate (Aldrich) drop wisely. The reaction mixture is stirred over night and diethylether is added. After evaporation of one third of the solvent volume the desired compound precipitates and the rest of the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **4** is obtained in 69 % yield (20.8 mmol, 7.68 g).
MS-ESI: 219 (M⁺, 100),

| Elementary analysis: | | | | |
|---|---|---|---|---|
| Calculated: | C 42.39% | H 4.10% | F 15.47% | N 7.61% |
| Found: | C 42.42% | H 4.12% | F 15.41% | N 7.59% |

e) Synthesis of Trifluoro-methanesulfonate(4-carboxy-2-cyano-phenyl)-trimethylammonium **5**: A solution of 4,01 g (10.9 mmol) **4**, 95 ml dest. water and 95 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **5** is obtained in 93% yield (3.59 g, 10.1 mmol) and crude compound **5** is used for the next step without purification.
MS-ESI: 205 (M⁺, 100),

| Elementary analysis: | | | | |
|---|---|---|---|---|
| Calculated: | C 40.68% | H 3.70% | F 16.09% | N 7.91% |
| Found: | C 40.72% | H 3.71% | F 16.06% | N 7,91% |

### Preparation of RG-L₁-B₁-OH 8 (G = CF₃)

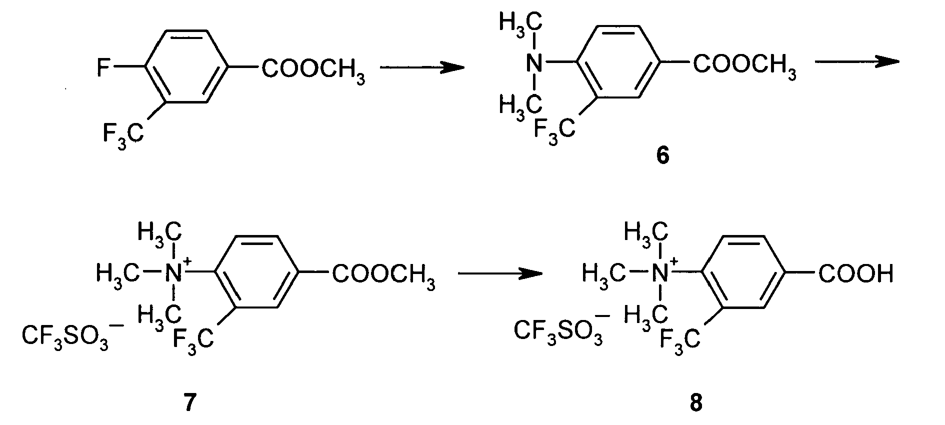

a) 4-Dimethylamino-2-trifluoromethyl-benzoic acid methyl ester **6**:
a) To a stirred solution of 4,48 g (22.5 mmol) 4-Fluoro-2-trifluoromethyl-benzoic acid methyl ester (Rarechem) and 60.0 ml dimethylsulfoxide are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product 6 is obtained in 72 % yield (4,00 g, 16,2 mmol).
MS-ESI: 248 (M⁺ +1, 78).

| | | | | |
|---|---|---|---|---|
| Elementary analysis: | C 53.44% | H 4.89% | F 23.05% | N 5.67% |
| Found: | C 53.46% | H 4.91% | F 23,04% | N 5.64% |

b) Trifluoro-methanesulfonate(4-methoxycarbonyl-3-trifluoromethyl-phenyl)-trimethyl-ammonium **7**: To a stirred solution of 3,09 g (12,5 mmol) **6** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitrile/water - gradient 1:99 to 80:20). The desired compound **7** is obtained in 69% yield (3,55 g, 8,63 mmol).
MS-ESI: 262 (M⁺, 87),

| Elementary analysis: | | | | |
|---|---|---|---|---|
| Calculated: | C 37.96% | H 3.68% | F 27.71% | N 3.41% |
| Determined: | C 38.01% | H 3.63% | F 27.69% | N 3.41% |

c) Trifluoro-methanesulfonate(4-carboxy-3-trifluoromethyl-phenyl)-trimethylammonium **8:** A solution of 2,84 g (6,92 mmol) **7**, 60 ml dist. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 89% yield (2,45 g; 6.16 mmol) and crude compound **8** is used for the next step without purification.
MS-ESI: 248 (M⁺, 59),

| Elementary analysis: | | | | |
|---|---|---|---|---|
| Calculated: | C 36.28% | H 3.30% | F 28.69% | N 3.53% |
| Determined: | C 36.30% | H 3,32% | F 28.67% | N 3,52% |

### Preparation of RG-L₁-B₁-OH 10 (G = F)

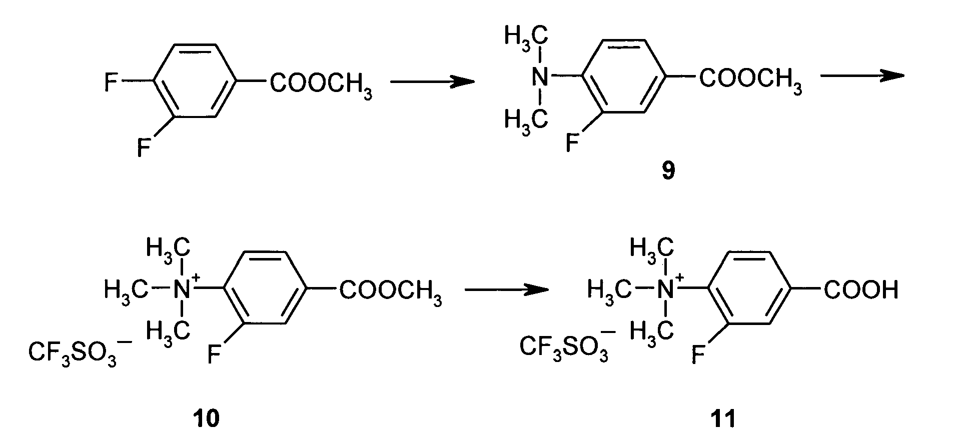

a) 3-Fluoro-4-dimethylamino-benzoic acid methyl ester 9: To a stirred solution of 38,7g (225 mmol) 3.4-difluoro-benzoic acid methyl ester (Apollo) and 600 ml dimethylsulphoxide are added 22.3 g (270 mmol) dimethylamine hydrochloride and 65.4 g (473 mmol) potassium carbonate. The reaction mixture is stirred for 5h at 55°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **9** is obtained in 71% yield (31.5 g, 160.0 mmol) and is used for the next step without purification.
MS-ESI: 198 (M⁺ +1, 72).

| Elementary analysis: | | | | |
|---|---|---|---|---|
| Calculated: | C 60.91% | H 6.13% | F 9.63% | N 7.10% |
| Found: | C 60.99% | H 6.15% | F 9.60% | N 7.07% |

b) Synthesis of (2-fluoro-4-methoxycarbonyl-phenyl)-trimethyl-ammonium trifluoro-methanesulfonate **10**: To a stirred solution of 3,90 g (19,8 mmol) **9** and 70 ml dichloromethane are added 32.5 g (198 mmol) methyltriflate (Aldrich) drop wisely. The reaction mixture is stirred for 2.5 days at room temperature and diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **10** is obtained in 80% yield (5,72 g, 15.84 mmol).
MS-ESI: 212 (M⁺, 76),

| Elementary analysis: | | | | |
|---|---|---|---|---|
| Calculated: | C 39.89% | H 4.18% | F 21.03% | N 3.88% |
| Found: | C 39.93% | H 4.20% | F 21.01% | N 3.84% |

c) Synthesis of (4-carboxy-2-fluoro-phenyl)-trimethyl-ammonium trifluoro-methanesulfonate **11**: A solution of 4,00 g (11.1 mmol) **10**, 96 ml dest. water and 96 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **11** is obtained in 92% yield (3.54 g, 10.2 mmol) and crude compound **11** is used for the next step without purification.
MS-ESI: 198 (M⁺, 76),

| Elementary analysis: | | | | |
|---|---|---|---|---|
| Calculated: | C 38.04% | H 3.77% | F 21.88% | N 4.03% |
| Found: | C 38.10% | H 3.79% | F 21.81% | N 4,00% |

### Example 3

### Synthesis of F--L₂--B₂--Y--E (IIB-a)

For the identification ¹⁸F--L₂--B₂--Y--E, reference standards ¹⁹F--L₂--B₂--Y--E were prepared according to the scheme shown below.

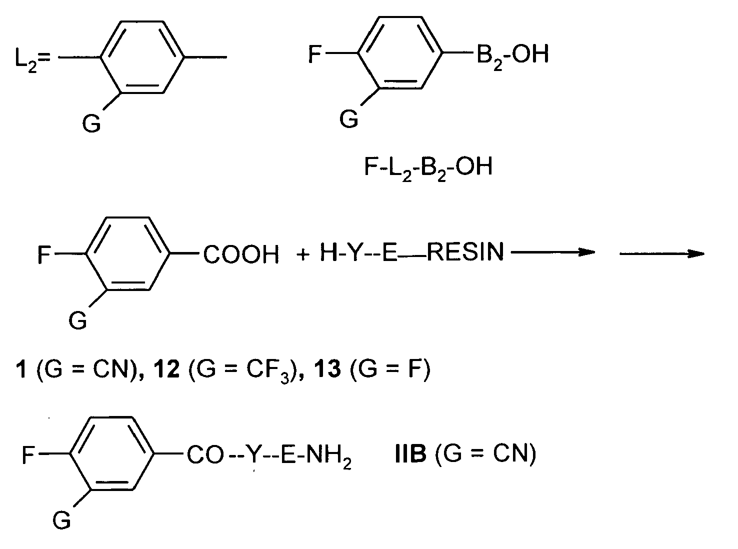

### Compounds 1, 12 and 13 were purchased commercially.

Synthesis of H-Y--E--RESIN and coupling of ¹⁹F--L₂--B₂-OH were accomplished according to the methods described in Example 1.
IIB-a-1: 4-[19]-Fluoro-3-cyano-benzoyl-Arg-Ava-Gln-Trp-Ala-Val-NMeGly-His-Sta-Leu-NH2, Molecular Weight, Calculated: 1382.62, Found: 691.9 ((M⁺+1)/2)
Table 2 lists all the F-19 compounds (IIB-a-1 to IIB-a-25) prepared for the chromatographic identification for products resulting from Ia-1 - la-25 used for F-18 labeling as well as binding affinities. The table lists all other F-19 compounds used in the SA analysis for the selection of high affinity analogs. Measurement of binding constants is described in Example 4.

**Table 2**

| | | | | |
|---|---|---|---|---|
| [19]F--L₂--B₂--Y₂--E(IIB-a) (G = 3-cyano, 3-trifluoromethyl or 3-fluoro; B₁ = CO as indicated) | | | | |
| 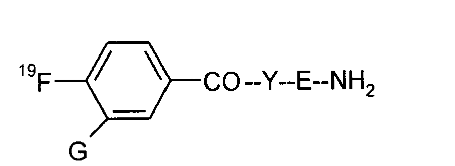 | | | | |

| [19]F--L₂(G)--B₂ | Y | | E | Kᵢ(nM) |
|---|---|---|---|---|
| IIB-a-1 | 4-[19]-Fluoro-3-cyano-benzoyl-Arg-Ava-Gin-Trp-Ala-Val-NMeGly-His-Sta-Leu-NH2 | | | 0.7 |
| IIB-a--2 | 4-[19]-Fluoro-3-cyano-benzoyl-Arg-Ava-Gln-Trp-Ala-Val-His(Me)-Sta-Leu-NH2 | | | 0.25 |
| IIB-a-3 | 4-[19]-Fluoro-3-cyano-benzoyl-Arg-Ava-Gln-Trp-Ala-Val-NMeGIy-His(3Me)-Sta-Leu-NH2 | | | 0.5 |
| IIB-a-4 | 4-[19]-Fluoro-3-cyano-benzoyl-1,4-cis-Achc-Gln-Trp-Ala-Val-Gly-His(3Me)-Sta-Leu-NH2 | | | 0.8 |
| IIB-a-5 | 4-[19]-Fluoro-3-cyano-benzoyl-Gln-Trp-Ala-Val-Gly-His(3Me)-Sta-Leu-NH2 (Y = bond) | | | 0.75 |
| IIB-a-6 | 4-[19]-Fluoro-3-cyano-benzoyl-AOC-Gln-Trp-Ala-Val-Gly-His(3Me)-Sta-Leu-NH2 | | | 0.40 |
| IIB-a-7 | 4-[19]-Fluoro-3-cyano-benzoyl-Ava-Gln-Trp-Ala-Val-NMeGIy-His(3Me)-Sta-Cpa-NH2 | | | 200 |
| IIB-a-8 | 4-[19]-Fluoro-3-cyano-benzoyl-Ava-Gln-Trp-Ala-Val-Gly-His(3Me)-4-Am,S-MeHpA-Leu-NH2 | | | 9.6 |
| IIB-a-9 | 4-[19]-Fluoro-3-cyano-benzoyl-Ava-Gln-Trp-Ala-Val-Gly-His(3Me)-Sta-Leu-NH2 | | | 7.0 |
| IIB-a-10 | 4-[19]-Fluoro-3-cyano-benzoyl-Lys(Me)2-βAla-Gln-Trp-Ala-Val-Gly-His(3Me)-Sta-Leu-NH2 | | | 3.0 |
| IIB-a-11 | 4-[19]-Fluoro-3-cyano-benzoyl-Lys(Me)2-βAla-Gln-Trp-Ala-Val-Gly-His(3Me)-Sta-Leu-NH2 | | | 1.6 |
| IIB-a-12 | 4-[19]-Fluoro-3-cyano-benzoyl-Arg-Ser-Gln-Trp-Ala-Val-Gly-His(3Me)-4-Am,5-MeHpA-Leu-NH2 | | | 22.0 |
| IIB-a-13 | 4-[19]-Fluoro-3-cyano-benzoyl-Ser-Ser-Gln-Trp-Ala-Val-Gly-His(3Me)-4-Am,5-MeHpA-Leu-NH2 | | | >25 |
| IIB-a-14 | 4-[19]-Fluoro-3-cyano-benzoyl-Lys(Me)2-Ser-Gln-Trp-Ala-Val-Gly-His(3Me)-4-Am,5-MeHpA-Leu-NH2 NA | | | |
| IIB-a-15 | 4-[19]-Fluoro-3-cyano-benzoyl-Arg-Ser-Gln-Trp-Ala-Val-Gly-His(3Me)-Sta-Leu-NH2 | | | 6.4 |
| IIB-a-16 | 4-[19]-Fluoro-3-cyano-benzoyl-Lys(Me)2-βAla-Gln-Trp-Ala-Val-Gly-His(3Me)-4-Am,5-MeHpA-Leu-NH2 | | | 12.2 |
| IIB-a-17 | 4-[19]-Fluoro-3-cyano-benzoyl-Ava-Gln-Trp-Ala-Val-Gly-His-4-Am,S-MeHpA-Leu-NH2 | | | 1.9 |
| | | | | |
| IIB-a-18 | 4-[19]-Fluoro-3-trifluoromethyl-benzoyl-Arg-Ava-Gln-Trp-Ala-Val-Gly-His(3Me)-Sta-Leu-NH2 | | | 0.9 |
| IIB-a-19 | 4-[19]-Fluoro-3-trifluoromethyl-benzoyl-Arg-Ava-Gln-Trp-Ala-Val-N MeGly-H is(3Me)-Sta-Leu-N H2 | | | 0.65 |
| IIB-a-20 | 4-[19]-Fluoro-3-trifluoromethyl-benzoyl-1,4-cis-Achc-Gln-Trp-Ala-Val-Gly-His(3Me)-Sta-Leu-NH2 | | | 0.77 |
| IIB-a-21 | 4-[19]-Fluoro-3-trifluoromethyl-benzoyl-Gln-Trp-Ala-Val-Gly-His(3Me)-Sta-Leu-NH2 (Y = bond) | | | 0.6 |
| IIB-a-22 | 4-[19]-Fluoro-3-trifluoromethyl-benzoyl-Arg-βAla-Gln-Trp-Ala-Val-Gly-His(3Me)-4-Am,5-MeHpA-Leu-NH2 | | | 9.8 |
| | | | | |
| IIB-a-23 | 4-[19]-Fluoro-3-cyano-benzoyl-Ava-Gln-Trp-Ala-Val-NMeGly-His(3Me)-4-Am,5-MeHpA-Cpa-NH2 | | | |
| IIB-a-24 | 4-[19]-Fluoro-3-cyano-benzoyl -Ser-Ser-Gln-Trp-Ala-Val-Gly-His(3Me)-Sta-Leu-NH2 | | | |
| IIB-a-25 | 4-[19]-Fluoro-3-cyano-benzoyl-DOA-Gln-Trp-Ala-Val-Gly-His(3Me)-Sta-Leu-NH2 | | | 13.5 |
| | | | | |
| IIB-a-26 | 3,4-[19]Difluorobenzoyl-Ava-Gln-Trp-Ala-Val-NMeGly-His-Sta-Leu-NH2 | | | 0.1 |
| IIB-a-27 | 3,4-[19]Difluorobenzoyl-Ava-Gln-Trp-Ala-Val-NMeGly-His-FA02010-Cpa-NH2 | | | 2.4 |
| IIB-a-28 | 3,4-[19]Difluorobenzoyl-Ava-Gin-Trp-Ala-Val-NMeGly-His-4-Am,5-MeHpA-tbuGly-NH2 | | | 2.4 |
| IIB-a-29 | 3,4-[19]Difluorobenzoyl-Ava-Gln-Trp-Ala-Val-NMeGly-His(3Me)-Sta-Leu-NH2 | | | 2.8 |
| IIB-a-30 | 3,4-[19]Difluorobenzoyl-Ava-Gln-Trp-Ala-Val-NMeGly-His(3Me)-Sta-tBuGly-NH2 | | | 2.8 |
| IIB-a-31 | 3,4-[19]Difluorobenzoyl-Ava-Gln-Trp-Ala-Val-Gly-His(3Me)-Sta-Leu-NH2 | | | 4.9 |
| IIB-a-32 | 3,4-[19]Difluorobenzoyl-Ava-Gln-Trp-Ala-Val-NMeGly-His(3Me)-4-Am,5-MeHpA-Leu-NH2 6008995 | | | 9.6 |
| IIB-a-33 | 3,4-[19]Difluorobenzoyl-Ava-Gln-DTrp-Ala-Val-Gly-His-4-Am,5-MeHpA-tbuGly-NH2 374432 | | | 30 |
| IIB-a-34 | 3,4-[19]Difluorobenzoyl-Ava-Gin-DTrp-Ala-Val-Gly-His-4-Am-5-MeHxA-Cpa-NH2 374431 | | | 30 |
| IIB-a-35 | 3,4-[19]Difluorobenzoyl-Ava-Gln-Trp-Ala-Val-NMeGly-His(3Me)-Sta-Cpa-NH2 | | | 31 |
| IIB-a-36 | 3,4-[19]Difluorobenzoyl-Ava-Gln-DTrp-Ala-Val-Gly-His-Sta-tbuAla-NH2 | | | >500 |
| IIB-a-37 | 3,4-[19]Difluorobenzoyl-Arg-Ava-Gln-Trp-Ala-Val-NMeGly-His-Sta-Leu-NH2 6049354 | | | 0.4 |
| IIB-a-38 | 3,4-[19]Difluorobenzoyl-GIn-Trp-Ala-Val-Gly-His(3Me)-Sta-Leu-NH2 6048906 | | | 0.55 |
| IIB-a-39 | 3,4-[19]Difluorobenzoyl-Arg-Ava-Gln-Trp-Ala-Val-NMeGly-His(3Me)-Sta-Leu-NH2 6049338 | | | 0.58 |
| IIB-a-40 | 3,4-[19]Difluorobenzoyl-Arg-Ava-Gln-Trp-Ala-Val-Gly-His(3Me)-Sta-Leu-NH2 6049341 | | | 1.02 |
| IIB-a-41 | 3,4-[19]Difluorobenzoyl-Arg-βAla-Arg-Gln-Trp-Ala-Val-Gly-His(3Me)-Sta-Leu-NH2 6049346 | | | 7.5 |
| IIB-a-42 | 3,4-[19]Difluorobenzoyl-Ava-Gln-Trp-Ala-Val-Gly-His(3Me)-Sta-Cpa-NH2 | | | 14.2 |
| IIB-a-43 | 3,4-[19]Difluorobenzoyl-Ava-Gln-Trp-Ala-Val-Gly-His(3Me)-Sta-tBuGly-NH2 6049232 | | | >25 |
| IIB-a-44 | 3,4-119]Difluorobenzoyl-Arg-Arg-Gln-Trp-Ala-Val-NMeGly-His(3Me)-Sta-Leu-NH2 | | | >25 |
| IIB-a-45 | 3,4-[19]Difluorobenzoyl-Arg-βAla-Gln-Trp-Ala-Val-NMeGly-His(3Me)-Sta-Leu-NH2 | | | >25 |
| IIB-a-46 | 3,4-[19]Difluorobenzoyl-Gln-Trp-Ala-Val-Gly-His(3Me)-4-Am,5-MeHpA-Leu-NH2 | | | >25 |
| IIB-a-47 | 3,4-[19]Difluorobenzoyl-Ava-Gln-Trp-Ala-Val-NmeGly-His(3Me)-4-Am,5-MeHpA-Cpa-NH2 | | | >25 |
| IIB-a-48 | 3,4-[19]Difluorobenzoyl-Ava-Gln-Trp-Ala-Val-Gly-His(3Me)-4-Am,S-MeHpA-Leu-NH2 | | | >25 |
| IIB-a-49 | 3,4-[19]Difluorobenzoyl-Ava-Gln-Trp-Ala-Val-Gly-NMeHis-4-Am,5-MeHpA-Cpa-NH2 | | | >25 |
| IIB-a-49 | 3,4-[19]Difluorobenzoyl-Ava-Gln-Trp-Ala-Val-Gly-NMeHis(3Me)-4-Am,5-MeHpA-Leu-NH2 | | | >25 |
| IIB-a-50 | 3,4-[19]Difluorobenzoyl-Ava-Gln-Trp-Ala-Val-Gly-NMeHis-4-Am,5-MeHpA-Leu-NH2 | | | >25 |
| IIB-a-51 | 3,4-[19]Difluorobenzoyl-Ava-Gln-Trp-Ala-Val-NMeGIy-Hls-AHMHxA -Leu-NH2 | | | >25 |
| IIB-a-52 | 3,4-[19]Difluorobenzoyl-Ava-Gln-Trp-Ala-Val-βAla-NMeHis-Tha-Cpa-NH2 | | | 0.4 |
| IIB-a-53 | 3,4-[19]Difluorobenzoyl-Ava-Gln-Trp-Ala-Val-βAla-NMeHis-Phe-Cpa-NH2 | | | 0.6 |
| IIB-a-54 | 3,4-[19]Difluorobenzoyl-Ava-Gln-Trp-Ala-Val-βAla-NMeHis-Phe-Leu-NH2 | | | 0.4 |
| IIB-a-55 | 3,4-[19]Difluorobenzoyl-Ava-Gln-Trp-Ala-Val-βAla-DHis-Phe-Leu-NH2 | | | 19.0 |
| IIB-a-56 | 3,4-[19]Difluorobenzoyl-Ava-Gln-Trp-Ala-Val-βAla-His-ßhLeu-Leu-NH2 | | | 45.0 |
| IIB-a-57 | 3,4-[19]Difluorobenzoyl-Ava-Gln-Trp-Ala-Val-βAla-His-ßhlle-Leu-NH2 | | | >50 |
| IIB-a-58 | 3,4-[19]Difluorobenzoyl-Ava-Gln-Trp-Ala-Val-βAla-His-ßhLeu-tbuGly-NH2 | | | >50 |
| IIB-a-59 | 3,4-[19]Difluorobenzoyl-Ava-Gln-Trp-Ala-Val-βAla-His(3Me)-Phe-Tha-NH2 | | | >50 |
| IIB-a-60 | 3,4-[19]Difluorobenzoyl-Ava-Gln-Trp-Ala-Val-βAla-His(3Me)-Phe-Nle-NH2 | | | >50 |
| IIB-a-51 | 3,4-[19]Difluorobenzoyl-Ava-Gln-Trp-Ala-Val-βAla-NMeHis-Phe-tbuGly-NH2 | | | NA |
| IIB-a-52 | 3,4-[19]Difluorobenzoyl-Ava-Gln-Trp-Ala-Val-βAla-NMeHis-Tha-tbuGly-NH2 | | | NA |
| IIB-a-53 | 3,4-[19]Difluorobenzoyl-Ava-Gln-Trp-Ala-Val-βAla-His(3Me)-Tha-tbuGly-NH2 | | | NA |
| IIB-a-54 | 3,4-[19]Difluorobenzoyl-Ava-Gln-Trp-Ala-Val-βAla-His(3Me)-Phe-Cpa-NH2 | | | NA |
| IIB-a-55 | 3,4-[19]Difluorobenzoyl-Ava-Gin-Trp-Ala-NMeVal-βAla-His-Phe-Leu-NH2 | | | NA |
| IIB-a-56 | 3,4-[19]Difluorobenzoyl-Ava-Gln-Trp-Ala-Val-βAla-His-NMePhe-Leu-NH2 | | | NA |
| IIB-a-57 | 3,4-[19]Difluorobenzoyl-Ava-Gln-DTrp-Ala-Val-βAla-His-Phe-Leu-NH2 6000816 | | | NA |
| IIB-a-58 | 3,4-[19]Difluorobenzoyl-Ava-Gln-Trp-DAla-Val-βAla-His-Phe-Leu-NH2 6000815 | | | NA |
| IIB-a-59 | 3,4-[19]Difluorobenzoyl-Ava-Gln-Trp-Ala-DVal-βAla-His-Phe-Leu-NH2 6000814 | | | NA |
| IIB-a-60 | 3,4-[19]Difluorobenzoyl-Ava-Gln-Trp-Ala-Val-βAla-His-DPhe-Leu-NH2 6000812 | | | NA |
| IIB-a-61 | 3,4-[19]Difluorobenzoyl-Ava-Gln-Trp-Ala-Val-βAla-His-ßhlle-tbuGly-NH2 | | | NA |

| | | | |
|---|---|---|---|
| [19]F--L₁--B₁--Y--E(II) (G = 3-cyano; B₁ = SO₂ as indicated) | | | |
| 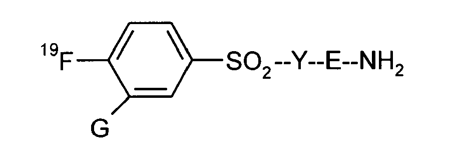 | | | |

| [19]F--L₁(G)--B₁ | Y | E | Kᵢ(nM) |
|---|---|---|---|
| IIB-a-62 | 4-[19]Fluoro-3-cyano-phenylsulfonyl -Ava-Gln-Trp-Ala-Val-NMeGly-His-4-Am,5-MeHpA-Cpa-NH2 | | 0.4 |
| IIB-a-63 | 4-[19]Fluoro-3-cyano-phenylsulfonyl -Ava-Gln-Trp-Ala-Val-NMeGIy-His-Sta-Cpa-NH2 | | 1.1 |
| IIB-a-64 | 4-[19]Fluoro-3-cyano-phenylsulfonyl -Ava-Gln-Trp-Ala-Val-NMeGIy-His-Sta-tbuAla-NH2 | | 2.5 |
| IIB-a-65 | 4-[19]Fluoro-3-cyano-phenylsulfonyl -Ava-Gln-Trp-Ala-Val-NMeGly-His-4-Am,5-MeHpA-tbuAla-NH2 | | 2.5 |

### Example 4

*In vitro* binding affinity and specificity of Bombesin analogs for the human bombesin 2 receptor (GRPR) were assessed via a competitive receptor-binding assay using ¹²⁵I-[Tyr⁴]-Bombesin (Perkin Elmer; specific activity 81.4 TBq/mmol) as GRPR-specific radioligand. The assay was performed based on the scintillation proximity assay (SPA) technology (Carpenter JW et al., Meth Mol Biol 2002; 190:31-49) using GRPR-containing cell membranes (Perkin Elmer) and wheat germ agglutinin (WGA)-coated PVT beads (Amersham Bioscience).

Briefly, GRPR-containing membranes and WGA-PVT beads were mixed in assay buffer (50 mM Tris/HCl pH 7.2, 5 mM MgCl₂, 1 mM EGTA, Complete protease inhibitor (Roche Diagnostics GmbH) and 0.3% PEI) to give final concentrations of approximately 100 µg/ml protein and 40 mg/ml PVT-SPA beads. The ligand ¹²⁵I-[Tyr⁴]-Bombesin was diluted to 0.5 nM in assay buffer. The test compounds were dissolved in DMSO to give 1 mM stock solutions Later on, they were diluted in assay buffer to 8 pM - 1.5 µM.

The assay was then performed as follows: First, 10 µl of compound solution to be tested for binding were placed in white 384 well plates (Optiplate-384, Perkin-Elmer). At next, 20 µl GRPR/WGA-PVT bead mixture and 20 µl of the ligand solution were added. After 90 minutes incubation at room temperature, another 50 µl of assay buffer were added, the plate sealed and centrifuged for 10 min at 520xg at room temperature. Signals were measured in a TopCount (Perkin Elmer) for 1 min integration time per well. The IC₅₀ was calculated by nonlinear regression using the GraFit data analysis software (Erithacus Software Ltd.). Furthermore, the K_{I} was calculated based on the IC₅₀ for test compound as well as the K_{D} and the concentration of the ligand ¹²⁵I-[Tyr⁴]-Bombesin. Experiments were done with quadruple samples.

The Binding affinities measured for all the cold F-19 compounds are listed Table 2.

### Example 5:

### General Radiolabeling Method

In a 5 mL Wheaton vial, ¹⁸F-fluoride (up to 40GBq) was azeotropically dried in the presence of Kryptofix 222 (5mg in 1.5ml CH₃CN) and potassium carbonate (1mg in 0.5ml water) or cesium carbonate (2,3mg in 0.5ml water) by heating under a stream of nitrogen at 110-120°C for 20-30 minutes. During this time 3 x 1ml CH₃CN were added and evaporated. After drying, a solution of **la-1** (2 mg) in 150µl DMSO was added. The reaction vessel was sealed and heated at 50-70°C for 5-15mins to effect labeling. The reaction was cooled to room temperature and dilute with water (2.7ml). The crude reaction mixture is analyzed using an analytical HPLC (Column Zorbax SB C18, 50x4.6mm, 1.8µ, 2ml/min, solvent A: H2O, solvent B: MeCN, gradient: 5%-95% B in 7 min or Column Econosphere C18 , 53x7mm, 3µ, 3ml/min (Alltech), solvent A: H2O + 0.1%TFA, solvent B: MeCN/Water 9/1 + 0.1% TFA, gradient: 5-95% B in 7 min. The F-18 labeled product **IIA-a-1** is confirmed by co-injection with the cold F-19 fluoro standard (**IIB-a-1**) on the Econsphere analytical HPLC
The product **IIA-a-1** was obtained by preparative radio HPLC to give to desired F-18 labeled peptide. 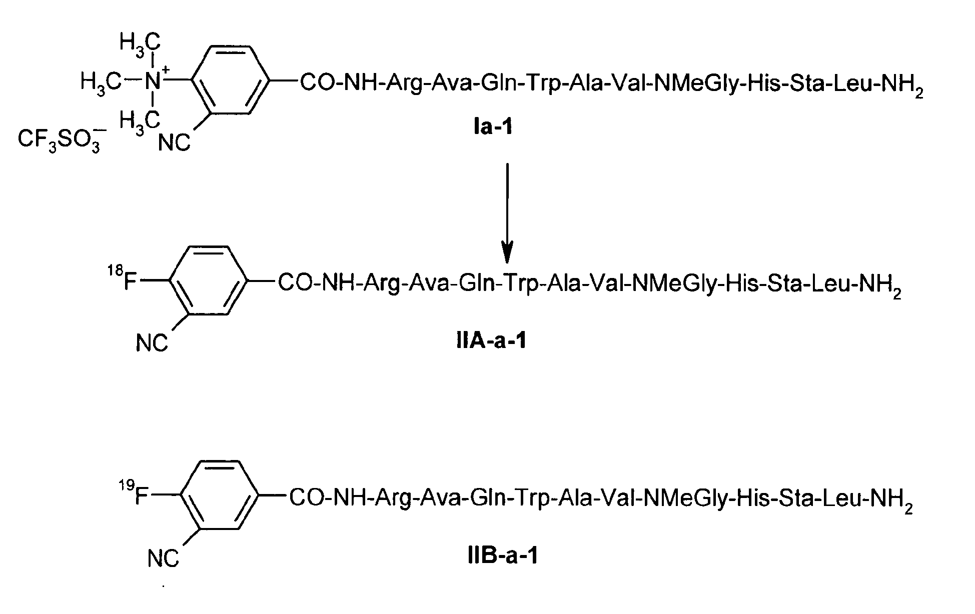 HPLC of **Ia-1, IIA-a-1** and **IIB-a-1** are shown below.

### HPLC of Ia-1

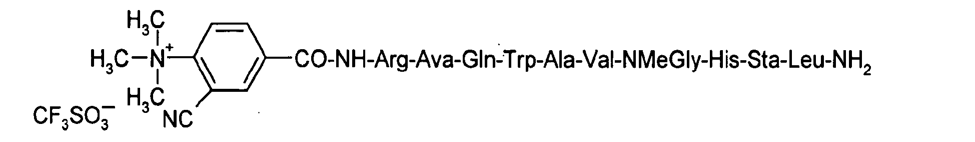

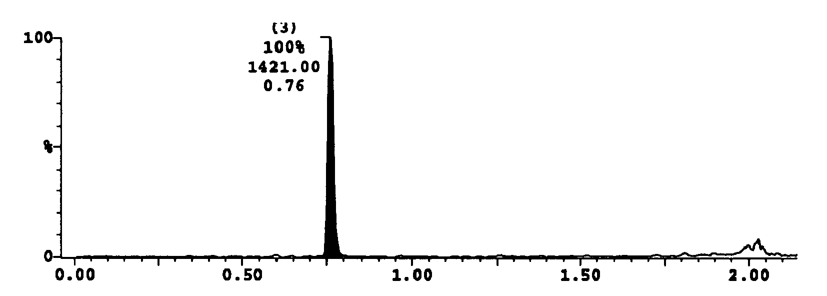

Column: C-18, Gradient: A: 10% CH₃CN: 90% H₂O, B:90% CH₃CN: 10% H₂O containing 0.1 % TFA (UPLC); Flow rate: 2 mL, 1% A to 99% B in 2 min.

### HPLC of IIA-a-1 (radiometric trace) and IIB-a-1 (UV detector)

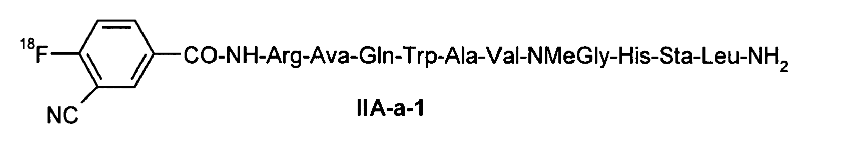

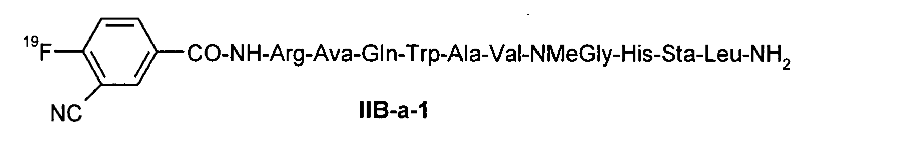

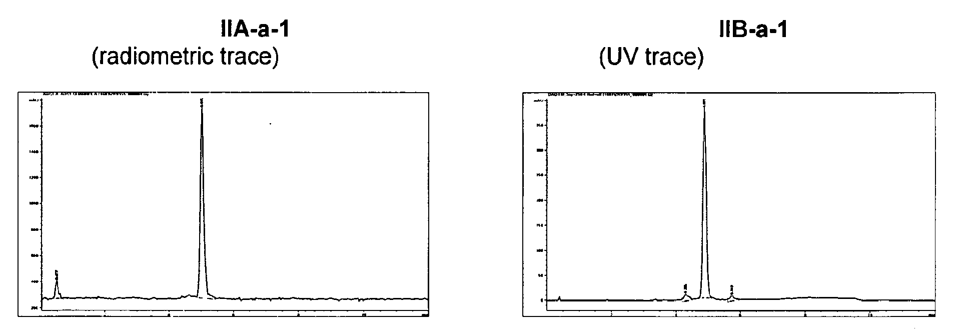

Column: C-18, Gradient: A: 10mM K₂HPO₄ pH:9,04 ; B: 10mM K₂HPO₄ pH:9,04/ACN 3/ 7; Flow rate: 2 mL, 5% A to 95% B in 7 min. Retention time **llA-a-**1 - 5.01 min. **IIB-a-1**- 4.89 min.

In a similar manner, Compounds shown in Table 1 (**la-2** to **la-22**) were labeled with F-18 to yield F-18 labeled peptides, **IIA-a-2** to **IIA-a-22** (Table 3) respectively. The chromatographic behavior of **IIA-a-2** to **IIA-a-22** were compared with **IIB-a-2** to **IIB-a-22** respectively for complete characterization in rodent studies and imaging.

### Human serum Stability of IIA-a-1

To qualify for pre-clinical and clinical use, it is necessary to establish the stability of the compound in human serum. The compound a 70 □L of human serum containing the F-18 labeled peptide (5.89 MBq/mL) was incubated at 37°C for 90 min. An aliquot was withdrawn at various intervals and the purity assessed by HPLC. The purity was evaluated in under two different conditions. Potassium hydrogen phosphate buffer system was used as a mobile phase to measure the stability of attached F-18 label.
Column: C-18, Gradient: A: 10mM K₂HPO₄ pH:9,04 ; B: 10mM K₂HPO₄ pH:9,04/ACN 3/ 7; Flow rate: 2 mL, 5% A to 95% B in 7 min.

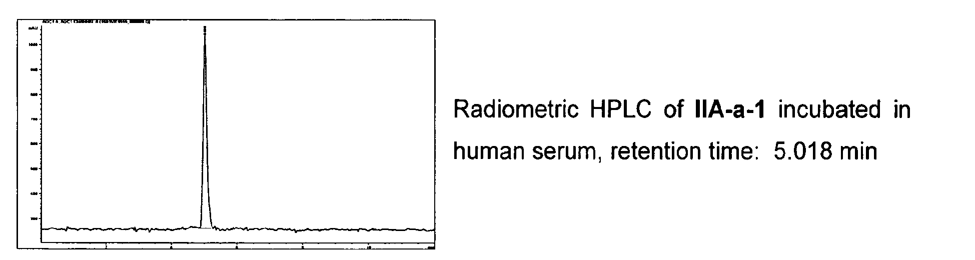

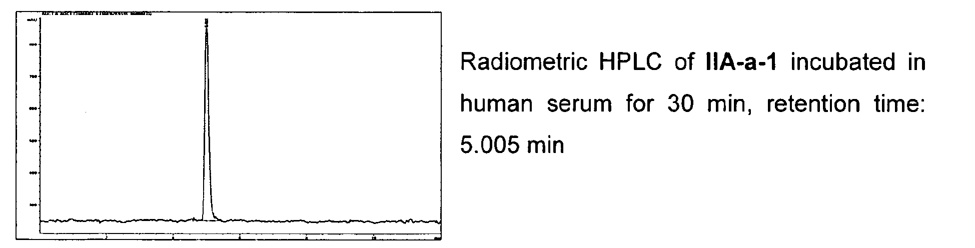

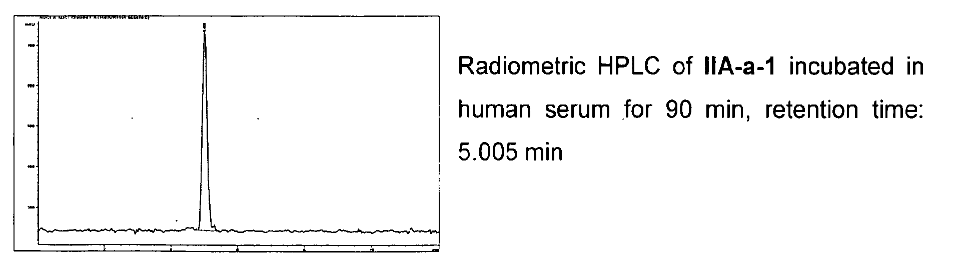

Trifluoroacetic acid system was used as a mobile phase to measure the stability of the entire molecule.
Column: C-18, Gradient: A: 10% CH₃CN: 90% H₂O, B:90% CH₃CN: 10% H₂O containing 0.1 % TFA.

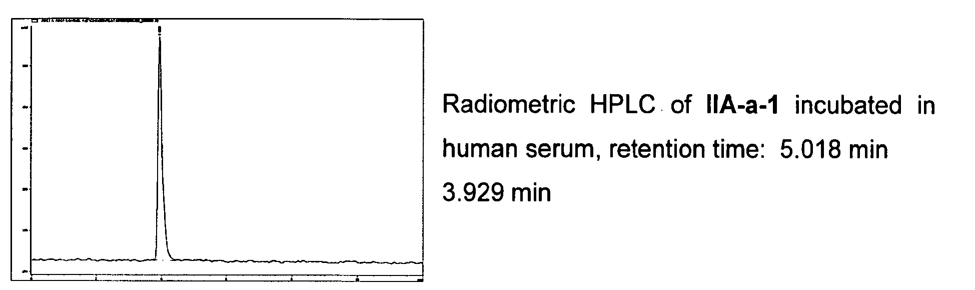

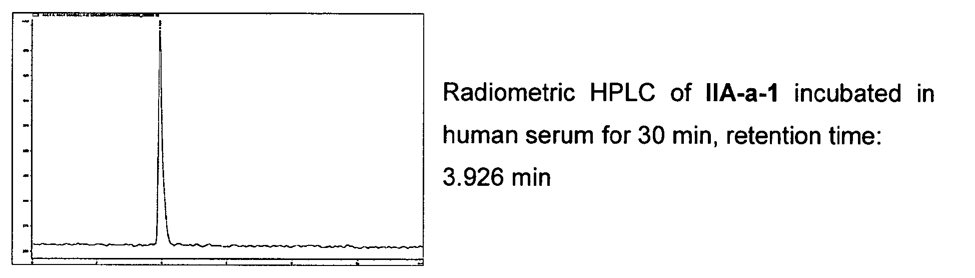

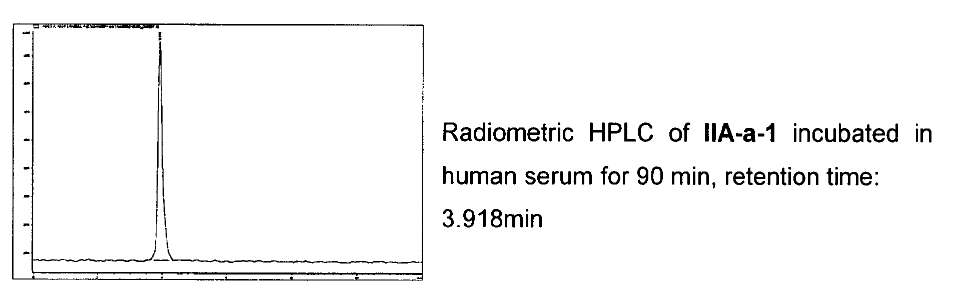

The above experiment clearly show that in the compounds of invention that F-18 isotope and the [18]F--L₂--B₂-Y--(IIA), targeting molecule of invention are stable in human serum.

In a similar manner, Compounds shown in Table 1 **(Ia-1** to **la-22)** were labelled with F-18 to yield F-18 labelled peptides, **IIA-a-1** to **IIA-a-22** (Table 3) respectively. The chromatographic behaviour of **IIA-a-1** to **IIA-a-22** were compared with **IIB-a-1** to **IIB-a-22** respectively for complete characterization in rodent studies and imaging.

**TABLE 3**

| | | |
|---|---|---|
| [18]F-L₂-B₂--Y--E (IIB-a-) (G = 3-cyano or 3-trifluoromethyl B₂ = CO as indicated) | | |
| 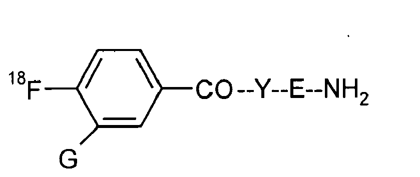 | | |

| [18]F--L₂(G)--B₂ | Y | E |
|---|---|---|
| IIA-a-1 | 4-[18]Fluoro-3-cyano-benzoyl-Arg-Ava-Gln-Trp-Ala-Val-NMeGly-His-Sta-Leu-NH2 | |
| IIA-a-2 | 4-[18]Fluoro-3-cyano-benzoyl-Arg-Ava-Gln-Trp-Ala-Val-His(Me)-Sta-Leu-NH2 | |
| IIA-a-3 | 4-[18]Fluoro-3-cyano-benzoyl-Arg-Ava-Gln-Trp-Ala-Val-NMeGly-His(3Me)-Sta-Leu-NH2 | |
| IIA-a-4 | 4-[18]Fluoro-3-cyano-benzoyl-1,4-cis-Achc-Gln-Trp-Ala-Val-Gly-His(3Me)-Sta-Leu-NH2 | |
| IIA-a-5 | 4-[18]Fluoro-3-cyano-benzoyl-Gln-Trp-Ala-Val-Gly-His(3Me)-Sta-Leu-NH2 (Y = bond) | |
| IIA-a-6 | 4-[18]Fluoro-3-cyano-benzoyl-AOC-Gln-Trp-Ala-Val-Gly-His(3Me)-Sta-Leu-NH2 | |
| IIA-a-7 | 4-[18]Fluoro-3-cyano-benzoyl-Ava-Gln-Trp-Ala-Val-NMeGly-His(3Me)-Sta-Cpa-NH2 | |
| IIA-a-8 | 4-[18]Fluoro-3-cyano-benzoyl-Ava-Gln-Trp-Ala-Val-Gly-His(3Me)-FA4-Am,5-MeHpA-Leu-NH2 | |
| IIA-a-9 | 4-[18]Fluoro-3-cyano-benzoyl-Ava-Gln-Trp-Ala-Val-Gly-His(3Me)-Sta-Leu-NH2 | |
| IIA-a-10 | 4-[18]Fluoro-3-cyano-benzoyl-Lys(Me)2-βAla-Gln-Trp-Ala-Val-Gly-His(3Me)-Sta-Leu-NH2 | |
| IIA-a-11 | 4-[18]Fluoro-3-cyano-benzoyl-Lys(Me)2-βAla-Gln-Trp-Ala-Val-Gly-His(3Me)-Sta-Leu-NH2 | |
| IIA-a-12 | 4-[18]Fluoro-3-cyano-benzoyl-Arg-Ser-Gln-Trp-Ala-Val-Gly-His(3Me)-4-Am,5-MeHpA-Leu-NH2 | |
| IIA-a-13 | 4-[18]Fluoro-3-cyano-benzoyl-Ser-Ser-Gln-Trp-Ala-Val-Gly-His(3Me)-4-Am,S-MeHpA-Leu-NH2 | |
| IIA-a-14 | 4-[18]Fluoro-3-cyano-benzoyl-Lys(Me)2-Ser-Gln-Trp-Ala-Val-Gly-His(3Me)-4-Am,5-MeHpA-Leu-NH2 | |
| IIA-a--15 | 4-[18]Fluoro-3-cyano-benzoyl-Arg-Ser-Gln-Trp-Ala-Val-Gly-His(3Me)-Sta-Leu-NH2 | |
| IIA-a-16 | 4-[18]Fluoro-3-cyano-benzoyl-Lys(Me)2-βAla-Gin-Trp-Ala-Val-Gly-His(3Me)-4-Am,5-MeHpA-Leu-NH2 | |
| IIA-a-17 | 4-[18]Fluoro-3-cyano-benzoyl-Ava-Gln-Trp-Ala-Val-Gly-His-4-Am,5-MeHpA-Leu-NH2 | |
| IIA-a-18 | 4-[18]Fluoro-3-trifluoromethyl-benzoyl-Arg-Ava-Gln-Trp-Ala-Val-Gly-His(3Me)-Sta-Leu-NH2 | |
| IIA-a-19 | 4-[18]Fluoro-3-trifluoromethyl-benzoyl-Arg-Ava-Gln-Trp-Ala-Val-NMeGly-His(3Me)-Sta-Leu-NH2 | |
| IIA-a-20 | 4-[18]Fluoro-3-trifluoromethyl-benzoyl-1,4-cis-Achc-Gln-Trp-Ala-Val-Gly-His(3Me)-Sta-Leu-NH2 | |
| IIA-a-21 | 4-[18]Fluoro-3-trifluoromethyl-benzoyl-Gln-Trp-Ala-Val-Gly-His(3Me)-Sta-Leu-NH2 (Y = bond) | |
| IIA-a-22 | 4-[18]Fluoro-3-trifluoromethyl-benzoyl-Arg-βAla-Gln-Trp-Ala-Val-Gly-His(3Me)-4-Am,5-MeHpA-Leu-NH2 | |

### Example 6

### Synthesis and F-18 Radiolabeling of lb)

### Synthesis of RG--L₁--B₁--Y--E (Ib) (G = CN)

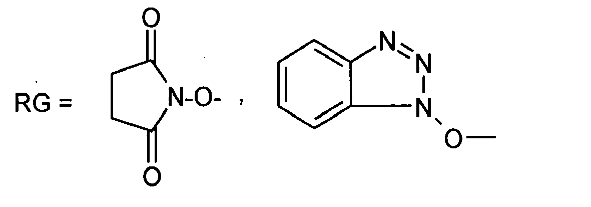

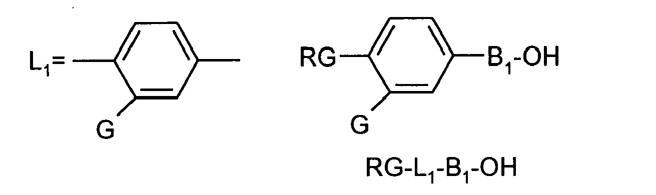

a) Synthesis of Trifluoro-methanesulfonate salt of 4-trimethyl-ammonium-3-cyano-benzoic acid succinimidate ester **14**: 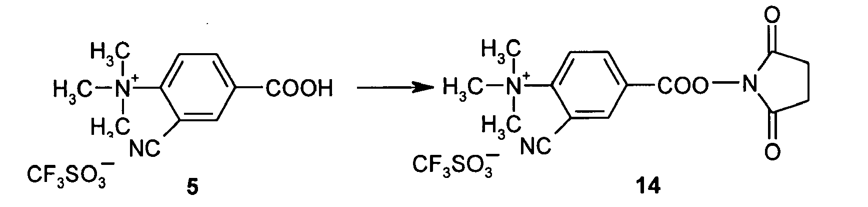 To a solution of 100 □mol trifluoro-methanesulfonate salt of 4-trimethylammonium-3-cyano-benzoic acid (Example 1) in 5 mL methylene chloride, an equimolar amounts of N-hydroxysuccinimide and DIC was added and the solution was stirred for 6-8 hrs. The urea was filtered and the solution was washed with water and evaporated. The product was used without further purification.
b) Reaction of **14** with -H-Y-RESIN to give Ib-1 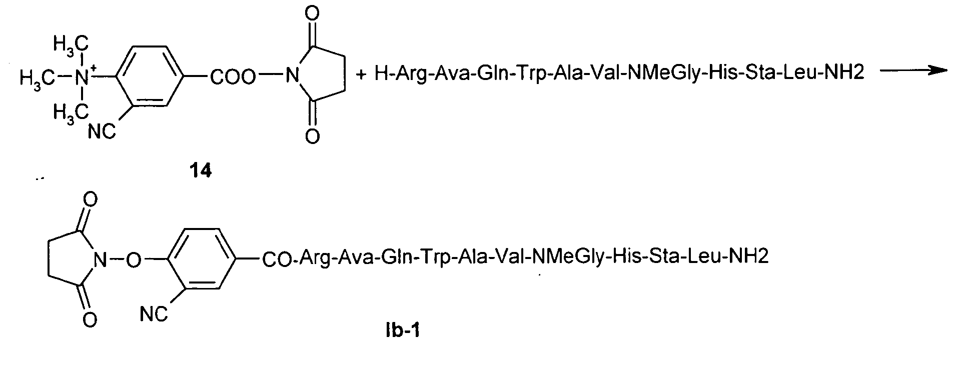 To a suspension of the resin -Y--E--RESIN in DMF (50 µmol in 0.5 mL) **14** was added. After 3-8 hours, the resin washed with DMF and dichloromethane. The peptide la) was isolated from the resin using TFA:diisopropylsilane:phenol:water cocktail with concomitant removal of the protecting groups of amino acids. The product was purified by HPLC using appropriate TFA:H₂O:0.1TFA gradient using C₁₈-reverse phase column. The products were identified mass spectra.
   MS Data: Mol. Wt.: Calculated: 1477.98, Found: 739.6 (M⁺+1)/2
   In a similar reaction, corresponding O-benzotriazole compound **lb-2** can be prepared. 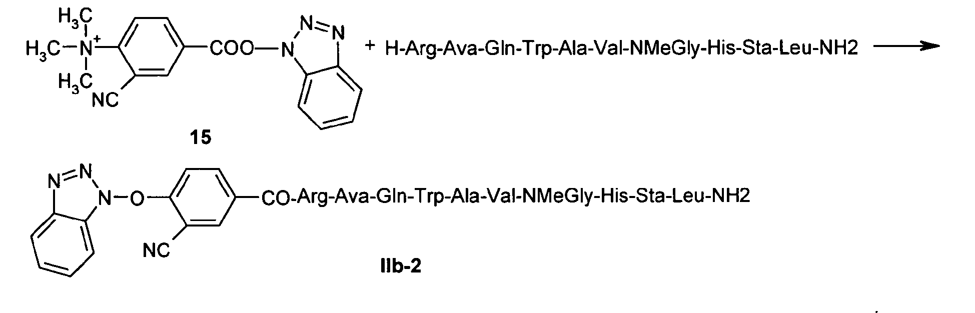 Compounds Ib-1 and Ib-2 can be radiolabeled with F-18 to yield the corresponding F-18 compound. The resulting products are identical to **IIA-a-1,** (Table 3) obtained upon radiolabeling of **Ia-1** (RG = (CH₃)₃N⁺) (Table 1). They can be identified by comparing the chromatographic behaviour with **IIB-a-1** (Table 2).

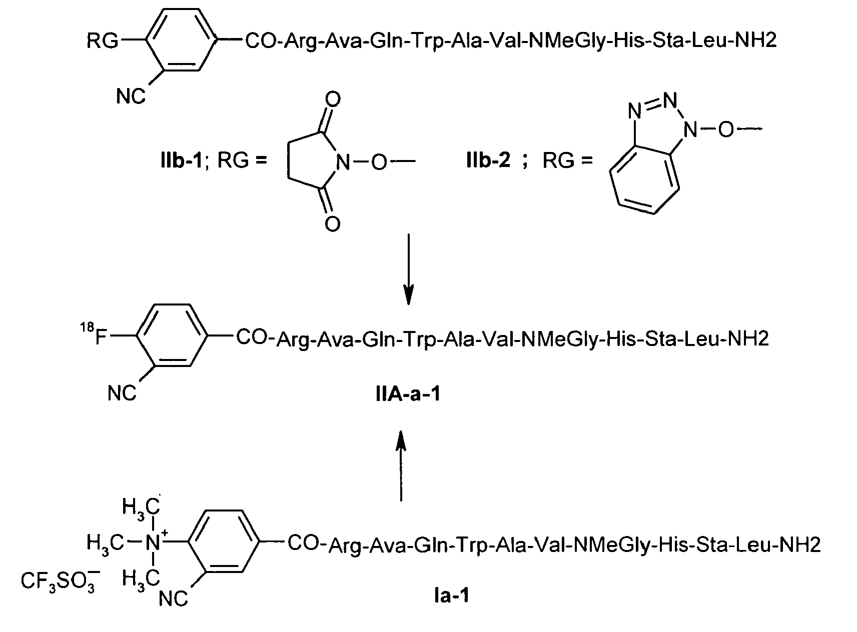

### Example 7

### Synthesis of RG--L₁--B₁--Y--E (I-c)

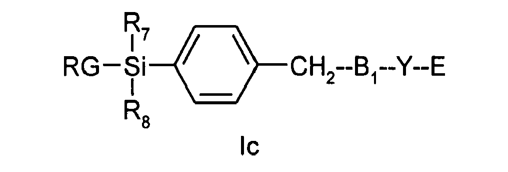

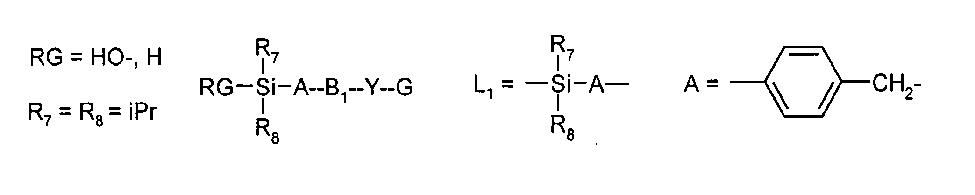

a) Preparation of RG-L₁-B₁-OSucc. (RG = HO) **20**: 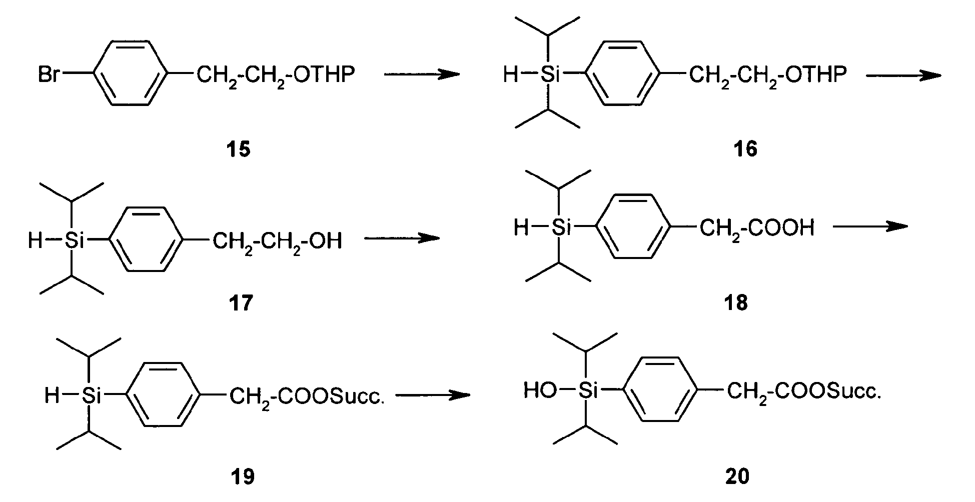 14.2 mL of a 2M isopropylmagnesium bromide solution in tetrahydrofuran (THF) was diluted with 140 mL THF and cooled to 5°C. 22.6 mL of a 2.5M solution of butyl-lithium in n-hexane was added followed by the solution of 14.2 mmol p-bromophenyethanol **15** in 12 mL THF. After 2 hours at 5-10°C 17.6 g chloro-diisopropyl-silane was added, the cooling bath removed and stirring continued for 2 hours. The reaction mixture was added to a solution of sodium bicarbonate and extracted with ethyl acetate. The combined organic extracts were washed with brine and dried over sodium sulfate. After filtration and removal of the solvent the crude product was purified by chromatography on silica gel to give 93-99% of **16**.
   To a solution 9.36 mmol **16** in 120 mL ethanol were added 1.61 g p-toluenesulfonic acid monohydrate and the mixture was stirred for 2 hours at 23°C. The reaction mixture was added to a solution of sodium bicarbonate extracted with dichloromethane. The combined organic extracts were washed with brine and dried over sodium sulfate. After filtration and removal of the solvent the crude product was purified by chromatography on silica gel to give 70-93% of **17**.
   To a solution of 2 mmol **17** in 14 mL acetone was added at 0°C 2.25 mL of Jones reagent. After 15 minutes water was added and the mixture extracted with ethyl acetate. The combined organic extracts were washed with brine and dried over sodium sulfate. After filtration and removal of the solvent the crude product was purified by chromatography on silica gel to give 52-70% of **18**.
   To a solution of 600 µmol **18** in 6 mL dichloro-methane were added 76 mg N-hydroxy-succinimide, 126 mg (3-Dimethylamino-propyl)-ethyl-carbodiimide hydrochloride and the mixture was stirred for 16 hours at 23°C. After addition of water and extraction with dichloro-methane the combined organic extracts were dried over sodium sulfate. After filtration and solvent evaporation the crude product was purified by chromatography on silica gel to give 73-99% of **19**.
   ¹H-NMR (CDCl₃): δ = 7.51 (2H), 7.32 (2H), 3.93 (3H), 2.83 (4H), 1.22 (2H), 1.06 (6H), 0.98 (6H) ppm.
   To a solution of 399 µmol **19** in 1.68 mL tetrachloromethane was added 7.27 mg palladium (10% on charcoal) and the mixture stirred for 16 hours at 23°C. 72 µL water were added and stirring was continued for additional 75 hours at 23°C. Sodium sulfate was added and after filtration the solvent was evaporated. The crude product was purified by chromatography on silica gel to give 64-84% of 20.
   ¹H-NMR (CDCl₃): δ = 7.55 (2H), 7.35 (2H), 3.94 (2H), 2.83 (4H), 1.80 (1H), 1.21 (2H), 1.05 (6H), 0.97 (6H) ppm.
b) Preparation of RG-L₁-B₁-OSucc. (RG = H)

In a similar procedure the di-t-butylsilane analog was prepared.

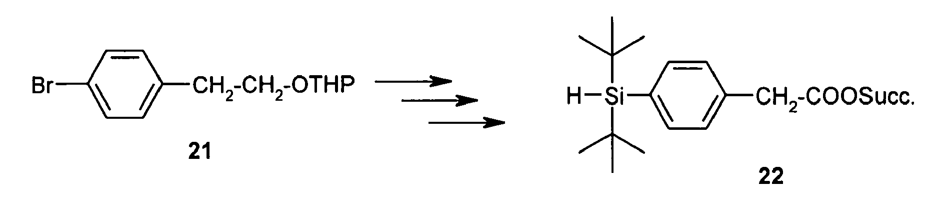

### Synthesis of Ic-1

a) Synthesis of H-Y--E: Solid-phase peptide synthesis (SPPS) involves the stepwise addition of amino acid residues to a growing peptide chain that is linked to an insoluble support or matrix, such as polystyrene. The C-terminal residue of the peptide is first anchored to a commercially available support (e.g., Rink amide resin) with its amino group protected with an N-protecting agent, fluorenylmethoxycarbonyl (FMOC) group. The amino protecting group is removed with suitable deprotecting agent such as piperidine for FMOC and the next amino acid residue (in N-protected form) is added with a coupling agents such as dicyclohexylcarbodiimide (DCC), di-isopropyl-cyclohexylcarbodiimide (DCCI), hydroxybenzotriazole (HOBt). Upon formation of a peptide bond, the reagents are washed from the support. After addition of the final residue of (Y), the peptide is attached to the solid support is ready for the coupling of RG--L₁--B₁-OSucc.
   To a suspension of the resin -Y--E--RESIN in DMF (0.1 to 0.25 mmol, Example 1), RG-L₁-B₁-OSucc (**20** or **22, 2** - 4 equivalents) was added. After 3-8 hours, the resin washed with DMF and dichloromethane. The peptide la) was isolated from the resin using TFA:diisopropylsilane:phenol:water cocktail with concomitant removal of the protecting groups of amino acids. The product was purified by HPLC using appropriate TFA:H₂O:0.1TFA gradient using C₁₈-reverse phase column. The products were identified mass spectra (Table 4)
   Ic-1: HO-Si(iPr)₂-C₆H₄-CH₂-CO-Ava--Gln-Trp-Ala-Val-Gly-His(3Me)-4-Am,5-MeHpA-Leu-NH2. Mol. Wt.: Calculated: 1311.72, Found: 1312.80 (M⁺+1)
   1c-2: H-Si(tBu)₂-C₆H₄-CH₂-CO-Ava--Gln-Trp-Ala-Val-NMeGly-His(3Me)-4-Am,5-MeHpA-Cpa-NH2. Mol. Wt.: Calculated: 1355.79, Found: 1355.90 (M⁺+1).

### Example 8

### Synthesis of ¹⁹F--L₁--B₁--Y--E (IIB-c-1), (IIB-c-2)

5 mg of silanol or silane (Ic-1 or Ic-2) was solved in 600 µl THF and treated with 1.42 mg (24.47 µM) KF, 9.21 mg (24.47 µM) K222, 1.69 mg (12.23 µM) K₂CO₃ and 4.2 µl acetic acid. The reaction mixture was stirred at 50° for 30 min and followed by HPLC purification to give 30 - 41% of (IIB-c-1 and IIB-c-2) respectively (Table 5)
IIB-c-1: ¹⁹F-Si(iPr)₂-C₆H₄-CH₂-CO-Ava--Gln-Trp-Ala-Val-Gly-His(3Me)-4-Am,5-MeHpA-Leu-NH2. Mol. Wt.: Calculated: 1313.72, Found: 1314.80 (M⁺+1)
IIB-c-2: ¹⁹F-Si(tBu)₂-C₆H₄-CH₂-CO-Ava--Gln-Trp-Ala-Val-NMeGly-His(3Me)-4-Am,5-MeHpA-Cpa-NH2. Mol. Wt.: Calculated: 1371.79, Found: 1372.90 (M⁺+1)

### Example 9

F-18 Radiolabeling of lc-2 was carried out according to the general procedure described in Example 5 to give IIA-c-2 in 20% radiochemical yield. The product was identified by comparison with IIB-c-1 (Table 6)
IIA-c-2: ¹⁸F-Si(tBu)₂-C₆H₄-CH₂-CO-Ava--Gln-Trp-Ala-Val-NMeGIy-His(3Me)-4-Am,5-MeHpA-Cpa-NH2.
1c-1 can be radiolabeled with F-18 in a similar reaction to yield IIA-c-1.

**TABLE 4**

| | | | | | | |
|---|---|---|---|---|---|---|
| RG--L₁--B₁--Y--E (1c) (RG = HO, H; B₁ = CO) | | | | | | |
| 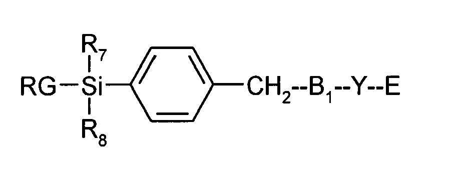 | | | | | | |
| 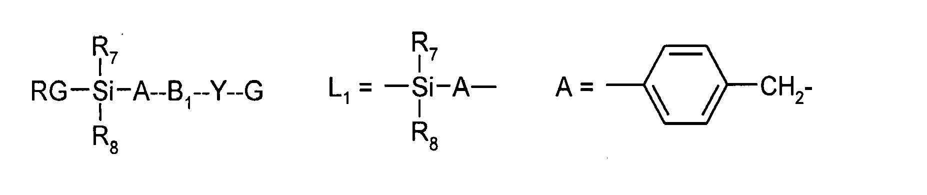 | | | | | | |

| RG | R₇ | R₈ | B₁ | Y | | E |
|---|---|---|---|---|---|---|
| | | | | | | |
| 1c-1 | HO | i-Pr | i-Pr | CO | -Ava- | -Gln-Trp-Ala-Val-Gly-His(3Me)-4-Am,5-MeHpA-Leu-NH2 |
| | | | | | | |
| 1 c-2 | H | t-Bu | t-Bu | CO | -Ava- | -Gln-Trp-Ala-Val-NMeGIy-His(3Me)-4-Am,5-MeHpA-Cpa-NH2 |
| | | | | | | |
| 1 c-3 | H | t-Bu | t-Bu | CO | Arg-Ava | -Gln-Trp-Ala-Val-NMeGIy-His-Sta-Leu-NH2 |
| 1 c-4 | H | t-Bu | t-Bu | CO | Arg-Ava | -Gln-Trp-Ala-Val-Gly-His(3Me)-Sta-Leu-NH2 |

**TABLE 5**

| | | | | | |
|---|---|---|---|---|---|
| ¹⁹F-L₂-B₂--Y--E(IIB-c) (B₁ = CO) | | | | | |
| 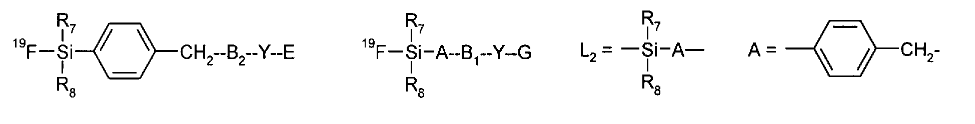 | | | | | |

| | R₇ | R₈ | B₁ | Y | E |
|---|---|---|---|---|---|
| IIB-c-1 | i-Pr | i-Pr | CO | -Ava- | -Gln-Trp-Ala-Val-Gly-His(3Me)- 4-Am-5-MeHpA - 4-amino-5-methylheptanoic acid - Leu-NH2 |
| IIB-c-2 | t-Bu | t-Bu | CO | -Ava- | -Gin-Trp-Ala-Val-NMeGly-His(3Me)-4-Am-5-MeHpA - 4-amino-5-methylheptanoic acid -Cpa-NH2 |

**TABLE 6**

| | | | | | |
|---|---|---|---|---|---|
| ¹⁸F-L₂-B₂--Y--E(IIB-c) (B₂ = CO) | | | | | |
| 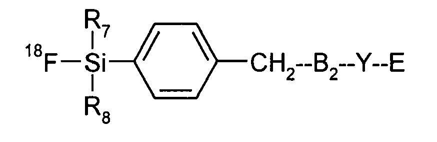 | | | | | |
| 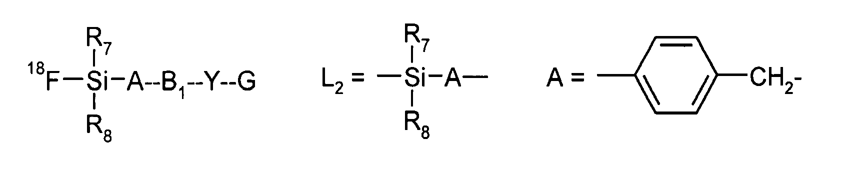 | | | | | |

| | R₇ | R₈ | B₁ | Y | E |
|---|---|---|---|---|---|
| IIA-c-2 | t-Bu | t-Bu | CO | -Ava- | -Gln-Trp-Ala-Val-NMeGIy-His(3Me)-4-Am-5-MeHpA - 4-amino-5-methylheptanoic acid -Cpa-NH2 |

### Example 10

### Synthesis of RG--L₁--B₁--Y--E (Id)

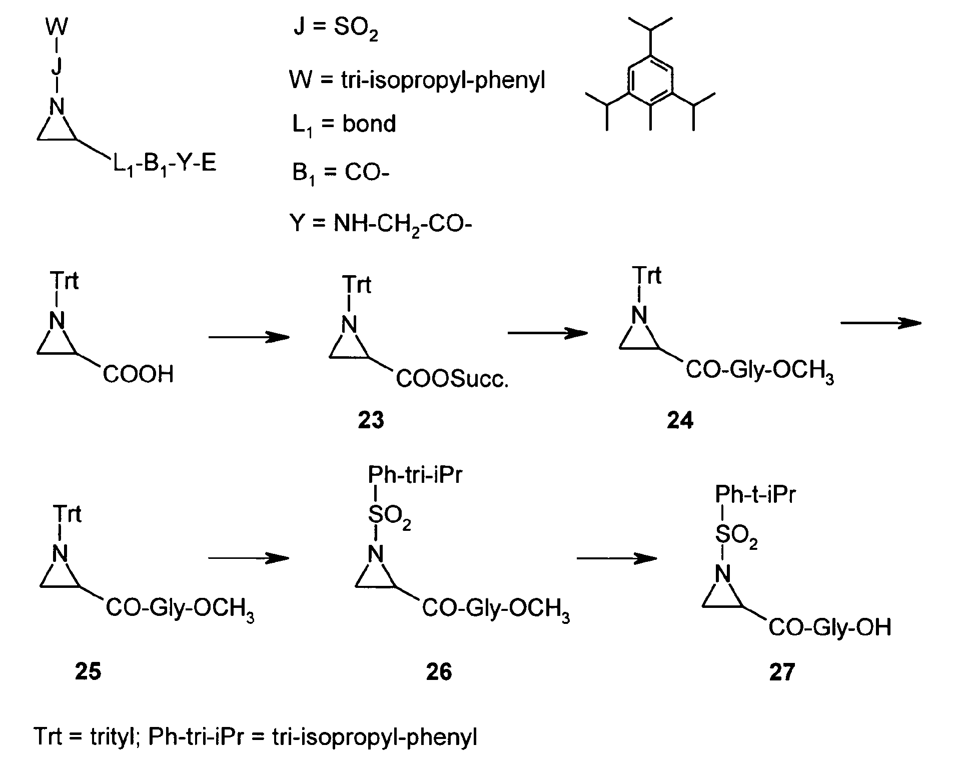

a) N-Trityl-azirine-carboxylicacid-succinimidate ester **23**: 910 mg (2.76 mmol) N-trityl-aziridine-carboxylic acid were dissolved in dichloromethane, 1.34 g (3.04 mmol) BOP and 318 mg (2.76 mmol) N-hydroxysuccinimide were added and the solution was cooled down to 0°C. Then 0,76 ml (4.42 mmol) ethyl diisopropylamine was added slowly and the reaction was stirred overnight at rt. The reaction mixture was diluted with dichloromethane, washed with 10% citric acid and brine, dried over sodium sulfate and concentrated. The residue was purified by chromatography on silica gel to give 760 mg (64%) of **23**.
b) N-[N-(Trityl)-aziridine-2-carbonyl]-glycine methyl ester **24**: 218 mg (1.74 mmol) glycine methylester hydrochloride was solved in DMF and treated with 0,36 ml (2.6 mmol) triethyl amine. After 30 min at rt 740 mg (1.74 mmol) **23** was added.
   The reaction mixture was stirred for 2h at 50°C and then concentrated. The residue was purified by chromatography on silica gel to give 550 (79%) of **24**.
c) N-[N-(tri.isopropylphenylsulfony)-aziridine-2-carbonyl]-glycine methyl ester: 2.3 g (5.74 mmol) **23** was solved in 95 ml chloroform, cooled down to 0°C and titrated with trifluoro acetic acid until complete conversion. The mixture was neutralized with saturated sodium bicarbonate solution and concentrated. The residue (**25**) was suspended in 95 ml ethyl acetate and 95 ml saturated sodium bicarbonate solution followed by (11.49 mmol) tri-isopropylphenylsulfonyl chloride.
   The reaction mixture was stirred overnight at rt. The phases were separated, the aqueous phase was extracted with ethyl acetate and the combined organic phases were dried over sodium sulphate and concentrated. The residue was purified by chromatography on silica gel to give (21-47%) of **26**.
   ¹H-NMR (MeOD): δ = 7.83 (d, 2H), 7.45 (d, 2H), 3.89 (s, 2H), 3.67 (s, 3H), 3.30 (d, 1 H), 2.76 (d, 1 H), 2.50 (d, 1 H), 2.44 (s, 3H)
d) N-[N-(tri.isopropylphenylsulfony)-aziridine-2-carbonyl]-glycine: A solution of **26** (1 mmol) was solved in 15 ml tetrahydrofuran, cooled down to 0°C and treated with 0.71 ml (1.2 equivalents) of 2N sodium hydroxide solution. The reaction mixture was stirred at rt for 2h and concentrated. The residue was taken up in water, carefully neutralized with citric acid and extracted with ethyl acetate. The combined organic phases were washed with brine, dried over sodium sulphate, filtrated and concentrated. The product **27** (90-97%) was used without further purification.
   ¹H-NMR (MeOD): δ = 7.27 (s, 2H), 4.27 (sept, 2H), 3.90 (d, 2H), 3.99 (dd, 1 H), 2.93 (sept, 1H), 2.78 (d, 1 H), 2.55 (d, 1 H), 1.30-1.23 (m, 18H)

Synthesis of RG--L₁--B₁--Y--E (Id): Coupling of 27 to resin bond H-Y-E-RESIN, was accomplished according to the general method outlined in Example 1. 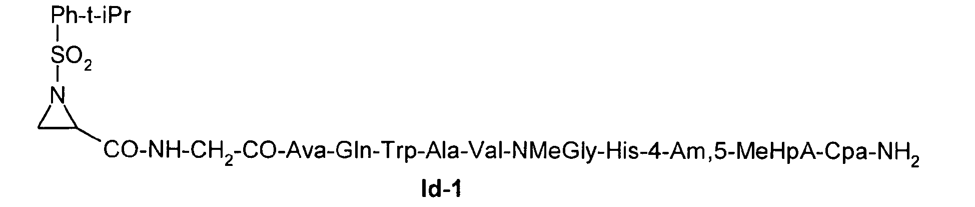 MS: Calculated: 1453.80, Found: 772.8 ((M⁺+1)/2)

### Aminoacid abbreviations

All natural amino acids are represented by 3-letter codes. Unless otherwise stated all the aminoacids have L-configurations.
Sta - Statine
His(3Me) - 3 - methylhisitidine 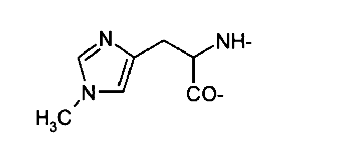 Ava - 5-aminovaleric acid
AOC - 8-aminooctanoic acid
tBuGly - t-butylglycine
tBuAla - t-butylalanine
βhLeu - β-homoleucine
βhlle - β-homoisoleucine
Lys(Me)₂ - ε-N,N-dimethyllysine
DOA - 3,6-dioxa-8-aminooctanoic acid
4-Am-5-MeHpA - 4-amino-5-methylheptanoic acid
4-Am-5-MeHxA - 4-amino-5-methylhexanoic acid
1,4-cis-ACHC - 1,4-cis-aminocyclohexamecarboxylic acid
AHMHxA - (3R,4S)-4-amino-3-hydroxy-5-methylhexanoic acid

*In vitro* binding affinity and specificity of Bombesin analogs for the human bombesin 2 receptor (GRPR) were assessed via a competitive receptor-binding assay using ¹²⁵I-[Tyr⁴]-Bombesin (Perkin Elmer; specific activity 81.4 TBq/mmol) as GRPR-specific radioligand. The assay was performed based on the scintillation proximity assay (SPA) technology (Carpenter JW et al., Meth Mol Biol 2002; 190:31-49) using GRPR-containing cell membranes (Perkin Elmer) and wheat germ agglutinin (WGA)-coated PVT beads (Amersham Bioscience).

Briefly, GRPR-containing membranes and WGA-PVT beads were mixed in assay buffer (50 mM Tris/HCl pH 7.2, 5 mM MgCl₂, 1 mM EGTA, Complete protease inhibitor (Roche Diagnostics GmbH) and 0.3% PEI) to give final concentrations of approximately 100 µg/ml protein and 40 mg/ml PVT-SPA beads. The ligand ¹²⁵I-[Tyr⁴]-Bombesin was diluted to 0.5 nM in assay buffer. The test compounds were dissolved in DMSO to give 1 mM stock solutions Lateron, they were diluted in assay buffer to 8 pM - 1.5 µM.

The assay was then performed as follows: First, 10 µl of compound solution to be tested for binding were placed in white 384 well plates (Optiplate-384, Perkin-Elmer). At next, 20 µl GRPR/WGA-PVT bead mixture and 20 µl of the ligand solution were added. After 90 minutes incubation at room temperature, another 50 µl of assay buffer were added, the plate sealed and centrifuged for 10 min at 520xg at room temperature. Signals were measured in a TopCount (Perkin Elmer) for 1 min integration time per well. The IC₅₀ was calculated by nonlinear regression using the GraFit data analysis software (Erithacus Software Ltd.). Furthermore, the K_{I} was calculated based on the IC₅₀ for test compound as well as the K_{D} and the concentration of the ligand ¹²⁵I-[Tyr⁴]-Bombesin. Experiments were done with quadruple samples.

## Claims

1. Compound of general formula (I)
RG--L₁--B₁--Y--E (I)
wherein
RG is a group or groups of atoms or a reactive moiety attached to L₁ that can be displaced or form an adduct with Fluorine isotope to provide chemically and biologically stable bond,
L₁ is a moiety group or bond to which the reactive group (RG) is attached,
B₁ is a functional group or a chain containing functional group connecting linker to spacer,
Y is a bond or a spacer,
E is a peptide or peptidomimetic
and pharmaceutically acceptable salts of inorganic or organic acids thereof.

2. A compound according to claim 1 wherein
RG is selected from the group a), b), c) or d)
wherein
a) is 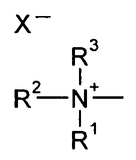 wherein R¹, R² and R³ are independently from each other C₁-C₆ alkyl or aralkyl, with the proviso that pharmaceutically acceptable salts thereof is a X⁻ salt,
wherein X⁻ is CF₃S(O)₂O⁻, C₄F₉S(O)₂O⁻, iodide anion, bromide anion, chloride anion, perchlorate anion (ClO₄⁻), phosphate anion, trifluoroaceate anion or other salts of inorganic or organic acids,
b) is 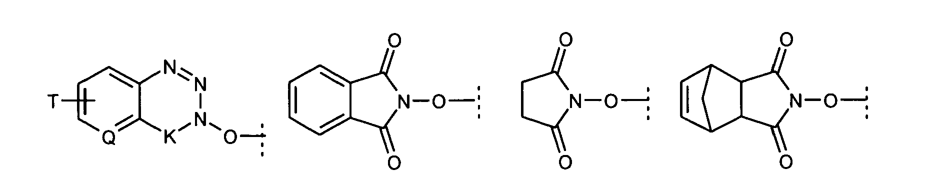 wherein,
T is H, or Cl,
Q is CH, or N,
K is absent, or C=O
c) represents a leaving group suitable for fluorination, selected from hydrogen, or OR⁴, wherein R⁴ represents hydrogen, C₁-C₁₀ alkyl, C₂-C₁₀ alkenyl, C₂-C₁₀ alkynyl, aryl,
d) is N-substituted aziridine 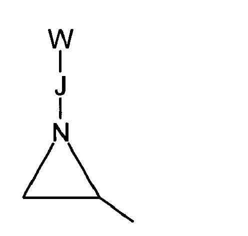
wherein J is SO₂, CO,
with the proviso that
when J is SO₂, then W is phenyl, substituted phenyl, NH₂, NHR³, NR³₂, linear or branched C₁-C₆ alkyl, polynuclear aryl or heteroaryl,
wherein substitution of the phenyl ring is independently or in combinations selected from linear or branched C₁-C₆ alkyl,
R³ is as defined above,
when J is CO, then W is phenyl, substituted phenyl, benzyloxy, fluorenylmethyl, methoxy, ethoxy or allyloxy, wherein substitution of the phenyl ring is independently or in combinations selected from linear or branched C₁-C₆ alkyl.

3. A compound according to claim 2 when RG is a) then R¹, R² and R³ are preferably and independently from each other aralkyl or linear or branched C₁-C₆ alkyl, whereas at least two moieties of R¹, R², R³ are alkyl.

4. A compound according to claim 3, wherein R¹ is aralkyl and R² and R³ are methyl.

5. A compound according to claim 4, wherein R¹, R²and R³are methyl.

6. A compound according to claim 2, wherein X is CF₃S(O)₂O⁻ or C₄F₉S(O)₂O⁻.

7. A compound according to claim 6, wherein X⁻ is CF₃S(O)₂O⁻.

8. A compound according to claim 1 or 2 when RG is b) then RG is selected from the group

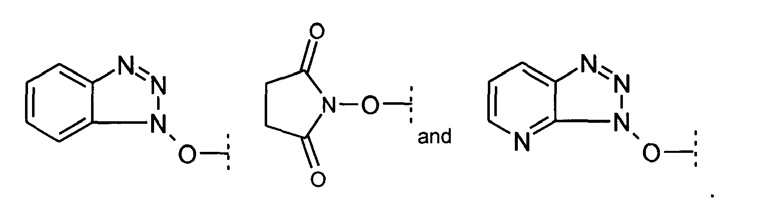

9. A compound according to claim 1 or 2 when RG is c) then RG represents a leaving group suitable for fluorination, selected from hydrogen or OR⁴, wherein R⁴ represents hydrogen, methyl, or ethyl.

10. A compound according to claim 1 or 2 when RG is d) then RG is selected from N-benzenesulfonylaziridinyl, N-p-toluenesulfonylaziridinyl, N-2,4,6-triisopropylsulfonylaziridinyl, N-3,4-dimethoxy-phenylsulfonylaziridinyl.

11. A compound according to claim 2 wherein when RG is a) or b), L1 is 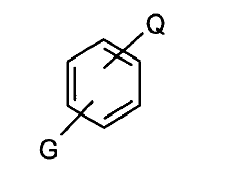 wherein
**G** is selected from -F, -Cl, -Br, -I, -NO, -NO₂, -NR⁴COCF₃, -NR⁴SO₂CF₃, - N(CF₃)₂, -NHCSNHR⁵, -N(SO₂R⁶)₂, -N(O)=NCONH₂, -NR⁵CN, -NHCSR⁶, - N≡C, -N=C(CF₃)₂, -N=NCF₃, -N=NCN, -NR⁵COR⁵, -NR⁵COOR⁶, -OSO₂CF₃, - OSO₂C₆H₅, -OCOR⁶, -ONO₂, -OSO₂R⁶, -O-C=CH₂, -OCF₂CF₃, -OCOCF₃, - OCN, -OCF₃, -C=N, -C(NO₂)₃, -COOR⁵, -CONR⁵R⁶, -CSNH₂, -CH=NOR⁵, - CH₂SO₂R⁵, -COCF₃, -CF₃, -CF₂CI-CBr₃, -CCIF₂, -CCl₃, -CF₂CF₃, -C=CR⁴, - CH=NSO₂CF₃, -CH₂CF₃, -COR⁶, -CH=NOR⁶, -CH₂CONH₂, -CSNHR⁶, - CH=NNHCSNH₂, -CH=NNHCONHNH₂, -C≡CF₃, -CF=CFCF₃, -CF₂-CF₂-CF₃, - CR⁵(CN) ₂, -COCF₂CF₂CF₃, -C(CF₃)₃, -C(CN)₃, -CR⁵=C(CN)₂, -1-pyrryl, - C(CN)=C(CN)₂, -C-pyridyl, -COC₆H₅, -COOC₆H₅, -SOCF₃, -SO₂CF₃, -SCF₃, - SO₂CN, -SCOCF₃, -SOR⁶, -S(OR⁶), -SC≡CR⁵, -SO₂R⁶, -SSO₂R⁶, -SR⁶, - SSR⁶, -SO₂CF₂CF₃, -SCF₂CF₃, -S(CF₃)=NSO₂CF₃, -SO₂C₆H₅, -SO₂N(R⁶)₂, - SO₂C(CF₃)₃, -SC(CF₃)₃, -SO(CF₃)=NSO₂CF₃, -S(O)=NCF₃, -S(O)=NR⁶, -S-C=CH₂, -SCOR⁶, -SOC₆H₅, -P(O)C₃F₇, -PO(R⁶)₂, -PO(N(R⁶)₂)₂, -P(N(R⁶)₂)₂, - P(O)R⁶₂, -PO(OR⁶)₂, and electron-withdrawing groups, wherein the respective substituent can be in ortho, meta or para position Q is hydrogen, -CN, -halo, -SO₂-R⁵ or nitro, wherein respective substituent can be in *ortho, meta* or para position,
R⁴ is hydrogen or C₁-C₆ linear or branched alkyl,
R⁵ is hydrogen or C₁-C₆ linear or branched alkyl,
R⁶ is C₁-C₆ linear or branched alkyl;
when RG is c), L1 is 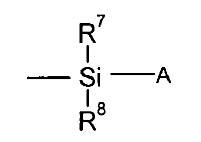 R⁷ and R⁸, independently, represent hydrogen, C₁-C₁₀ alkyl, branched C₁-C₈ alkyl, aryl, heteroaryl, or aralkyl and A represents a bond, C₁-C₁₀ alkyl or aryl, substituted aryl or heteroaryl; or
when RG is d), L1 is bond, linear or branched C₁-C₆ alkyl.

12. A compound according to claim 11 wherein
G is selected from -F, -Cl, -Br, -NO₂, -, -OSO₂R⁵, -OCF₃, -C≡N, -COOR⁴, - CONR⁴R⁵, -COCF₃, -CF₂CF₃, , -COR⁵, -CF₃, -C=CF₃, -CF₂-CF₂-CF₃, - COC₆H₅, -SO₂CF₃, -SCOCF₃, -SO₂R⁵, -SO₂CF₂CF₃, -SO₂C₆H₅, -SO₂N(R⁵) ₂, and -PO(OR⁵)₂, wherein the respective substituent can be in ortho, meta or para position.

13. A compound according to claim 11, wherein Q is selected from hydrogen, -CN, -fluoro, -chloro, -bromo or nitro, wherein respective substituent can be in ortho, meta or para position.

14. A compound according to claim 11 wherein R⁴ is hydrogen or linear or branched C₁-C₄ alkyl, R⁵ is hydrogen or linear or branched C₁-C₄ alkyl and R⁶ is linear or branched C₁-C₄ alkyl.

15. A compound according to claims 1 to 2 wherein
B1 is selected from
a bond,
-CO-,
-SO₂- with proviso that B1 is not SO₂ when RG is d),
-(CH₂)_{d}-CO-,
-SO-,
-C≡C-CO-, 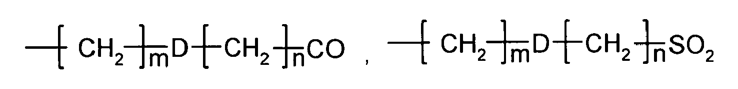 C(=O)-O,
-NR¹⁰,
-SO₂,
-NR¹⁰,
-NR¹¹,
-O,
-(S)ₚ,
-C(=O)NR¹²,
-NR¹²,
-C(=S)NR¹²,
-C(=S)O,
C₁-C₆ cycloalkyl,
alkenyl,
heterocycloalkyl,
substituted aryl or heteroaryl,
aralkyl, heteroaralkyl,
alkoxy, aryloxy, aralkoxy, directly bound to an alkyl chain
aryl,
-SO₂NR¹³-,
-NR¹³SO₂-,
-NR¹³C(=O)O-,
-NR¹³C(=O)NR¹²-,
-NH-NH-, and
-NH-O-,
wherein;
d is an integer between 1 and 6,
m and n, independently, can be any integer between 0 to 5;
D represents a bond, a sulphur atom, an oxygen atom, or NR⁹,
R⁹ represents hydrogen, C₁-C₁₀ alkyl, aryl, heteroaryl, or aralkyl,
p can be any integer between 1 and 3;
R¹⁰, R¹¹ and R¹², independently, represent hydrogen, C₁-C₁₀ alkyl, aryl, heteroaryl, or aralkyl, and
R¹³ represents hydrogen, substituted or non-substituted, linear or branched C₁-C₆ alkyl, aryl, cycloalkyl, heterocycloalkyl, aryl, heteroaryl, aralkyl, or heteroaralkyl.

16. A compound according to claim 15, wherein B1 is preferably selected from
-CO-,
-SO₂ with proviso that B1 is not SO₂ when RG is d), and
-C≡C-CO-,
wherein the respective substituent can be in meta or para position.

17. A compound according to all preceding claims wherein Y (spacer) is natural or unnatural amino acid sequence or non-amino acid group.

18. A compound according to claim 17 wherein Y is amino acid sequence with two (2) to twenty (20) amino acid residues.

19. A compound according to claim 18 wherein Y
is Arg-Ser, Arg-Ava, Lys(Me)2-β-ala, Lys(Me)2-ser, Arg-β-ala, Ser-Ser, Ser-Thr, Arg-Thr, S-alkylcysteine, Cysteic acid, thioalkylcysteine (S-S-Alkyl) or 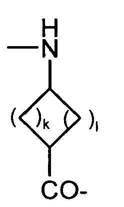 wherein k and I is 0-4.

20. A compound according to claim 17 wherein Y is non-amino acid moiety selected from
NH-(CH₂)ₚ-CO with p being an integer between 2 and 10 or
NH-(CH₂-CH₂-O)q-CH₂-CH₂-CO with q being an integer between 0 and 5,

21. A compound according to all preceding claims wherein E is a peptide comprising from 4 to 100 amino acids.

22. A compound according to claims 1 to 21 wherein E is selected from the group of bombesin, neuropeptide Y₁, Somatostatin receptor specific peptides, Cholecystokinin receptor peptides, Neurotensin analogs, LHRH agonists or antagonists, Gastrin releasing peptide, Integrins (α₃β₁, αᵥβ₃, αᵥβ₅, αIIb₃),Vasoactive intestinal peptide (VIP), Pituitary adenylate cyclase activating peptide (PACAP), Epidermal growth factor, Insulin growth factor, Angiotensin, chemokines, Thyrotropin releasing hormone, substrates and inhibitors for cell surface matrix metalloproteinase, Interleukins (IL-1, IL-4 and IL-6), Prolactin, and analogs thereof.

23. A compound according to claim 21 wherein E is bombesin, somatostatin or neuropeptide Y₁ and analogs thereof.

24. A compound according to claim 23 wherein E are bombesin analogs of sequence III or IV wherein
AA₁-AA₂-AA₃-AA₄-AA₅-AA₆-AA₇-AA₈-NT₁T₂ (type A) III
T₁ = T₂ =H, T₁ = H,T₂ = OH, T₁ = CH₃, T₂ = OH
AA₁ = Gln, Asn, Phe(4-CO-NH₂)
AA₂ = Trp, D-Trp
AA₃ = Ala, Ser, Val
AA₄ = Val, Ser. Thr
AA₅ = Gly, (N-Me)Gly
AA₆ = His, His(3-Me), (N-Me)His, (N-Me)His(3-Me)
AA₇ = Sta, Statine analogs and isomers, 4-Am,5-MeHpA, 4-Am,5-MeHxA and γ-substituted aminoacids.
AA₈ = Leu, Cpa, Cba, CpnA, Cha, t-buGly, tBuAla, Met, Nle, iso-Bu-Gly,
AA₁-AA₂-AA₃-AA₄-AA₅-AA₆-AA₇-AAs-NT₁T₂ (type B) IV
T₁ = T₂ =H, T₁ = H,T₂ = OH, T₁ = CH₃, T₂ = OH
AA₁ = Gln, Asn, Phe(4-CO-NH₂)
AA₂ = Trp, D-Trp
AA₃ = Ala, Ser, Val
AA₄ = Val, Ser. Thr
AA₅ = βAla, β²- and β³-amino acids as shown below 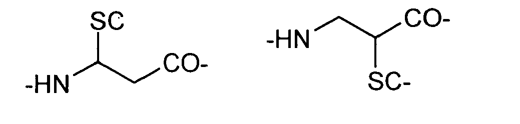 wherein SC represents side chain found in proteinogenic amino acids and homologs of proteinogenic amino acids,
AA₆ = His, His(3-Me), (N-Me)His, (N-Me)His(3-Me)
AA₇ = Phe, Tha, Nal,
AA₈ = Leu, Cpa, Cba, CpnA, Cha, t-buGly, tBuAla, Met, Nle, iso-Bu-Gly.

25. Compound selected from
la-1 4-(Trimethylammonium)-3-cyano-benzoyl-Arg-Ava-Gln-Trp-Ala-Val-NMeGIy-His-Sta-Leu-NH2,
la-2 4-(Trimethylammonium)-3-cyano-benzoyl-Arg-Ava-Gln-Trp-Ala-Val-Gly-His(Me)-Sta-Leu-NH2,
la-3 4-(Trimethylammonium)-3-cyano-benzoyl-Arg-Ava-Gln-Trp-Ala-Val-NMeGIy-His(3Me)-Sta-Leu-NH2,
la-4 4-(Trimethylammonium)-3-cyano-benzoyl-1,4-cis-Achc-Gln-Trp-Ala-Val-Gly-His(3Me)-Sta-Leu-NH2,
la-5 4-(Trimethylammonium)-3-cyano-benzoyl-Gln-Trp-Ala-Val-Gly-His(3Me)-Sta-Leu-NH2,
la-6 4-(Trimethylammonium)-3-cyano-benzoyl-AOC-Gln-Trp-Ala-Val-Gly-His(3Me)-Sta-Leu-NH2 ,
la-7 4-(Trimethylammonium)-3-cyano-benzoyl-Ava-Gln-Trp-Ala-Val-NMeGly-His(3Me)-Sta-Cpa-NH2,
la-8 4-(Trimethylammonium)-3-cyano-benzoyl-Ava-Gln-Trp-Ala-Val-Gly-His(3Me)-4-Am,5-MeHpA-Leu-NH2,
la-9 4-(Trimethylammonium)-3-cyano-benzoyl-Ava-Gln-Trp-Ala-Val-Gly-His(3Me)-Sta-Leu-NH2 ,
la-10 4-(Trimethylammonium)-3-cyano-benzoyl-Lys(Me)2-βAla-Gln-Trp-Ala-Val-Gly-His(3Me)-Sta-Leu-NH2,
la-11 4-(Trimethylammonium)-3-cyano-benzoyi-Lys(Me)2-βAla-Gln-Trp-Ala-Val-Gly-His(3Me)-Sta-Leu-NH2,
la-12 4-(Trimethylammonium)-3-cyano-benzoyl-Arg-Ser-Gln-Trp-Ala-Val-Gly-His(3Me)-4-Am,5-MeHpA-Leu-NH2,
la-13 4-(Trimethylammonium)-3-cyano-benzoyl-Ser-Ser-Gln-Trp-Ala-Val-Gly-His(3Me)-4-Am,5-MeHpA-Leu-NH2,
la-14 4-(Trimethylammonium)-3-cyano-benzoyl-Lys(Me)2-Ser-Gln-Trp-Ala-Val-Gly-His(3Me)-4-AM-5-MeHpA-Leu-NH2,
la-15 4-(Trimethylammonium)-3-cyano-benzoyl-Arg-Ser-Gln-Trp-Ala-Val-Gly-His(3Me)-Sta-Leu-NH2,
la-16 4-(Trimethylammonium)-3-cyano-benzoyl-Lys(Me)2-βAla-Gln-Trp-Ala-Val-Gly-His(3Me)-4-Am,5-MeHpA-Leu-NH2,
la-17 4-(Trimethylammonium)-3-cyano-benzoyl-Ava-Gln-Trp-Ala-Val-Gly-His-4-Am,5-MeHpA- -Leu-NH2,
la-18 4-(Trimethylammonium)-3-trifluoromethyl-benzoyl-Arg-Ava-Gln-Trp-Ala-Val-Gly-His(3Me)-Sta-Leu-NH2,
la-19 4-(Trimethylammonium)-3-trifluoromethyl-benzoyl-Arg-Ava-Gln-Trp-Ala-Val-NMeGly-His(3Me)-Sta-Leu-NH2,
la-20 4-(Trimethylammonium)-3-trifluoromethyl-benzoyl-1,4-cis-Achc-Gln-Trp-Ala-Val-Gly-His(3Me)-Sta-Leu-NH2,
la-21 4-(Trimethylammonium)-3-trifluoromethyl-benzoyl-Gln-Trp-Ala-Val-Gly-His(3Me)-Sta-Leu-NH2,
la-22 4-(Trimethylammonium)-3-trifluoromethyl-benzoyl-Arg-flAia-Gln-Trp-Ala-Val-Gly-His(3Me)-4-Am,5-MeHpA-Leu-NH2,
la-23 4-(Trimethylammonium)-3-cyano-benzoyl-Ava-Gln-Trp-Ala-Val-NMeGly-His(3Me)-4-Am,5-MeHpA-Cpa-NH2,
la-24 4-(Trimethylammonium)-3-cyano-benzoyl -Ser-Ser-Gln-Trp-Ala-Val-Gly-His(3Me)-Sta-Leu-NH2,
la-25 4-(Trimethylammonium)-3-cyano-benzoyl-DOA-Gln-Trp-Ala-Val-Gly-His(3Me) Sta-Leu-NH2, 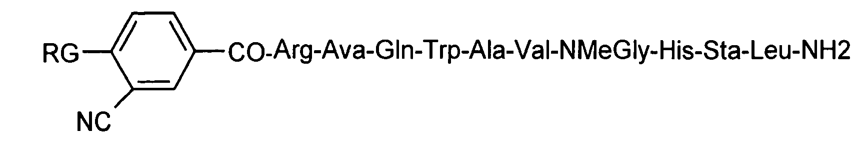 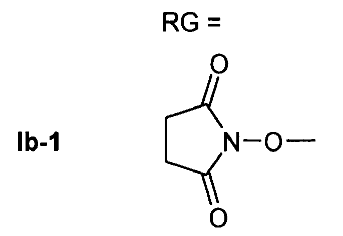 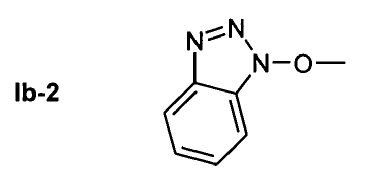 1 a-66: 4-(Trimethylammonium)-3-cyano-benzoyl-Ava-ε-c[Lys-(NMe)Phe-1Nal-D-Trp-Lys-Thrl
1 a-67: 4-(Trimethylammonium)-3-cyano-benzoyl-Ava-β-c[Dpr-Met-(NMe)Phe-Tyr-D-Trp-Lys]
1a-68: 4-(Trimethylammonium)-3-cyano-benzoyl-Ava-DCys-Leu-Ile-Thr-Arg-Cys-Ara-Tyr-NH₂]
1a-69: 4-(Trimethylammonium)-3-cyano-benzoyl-Ava-DCys-Leu-Ile-Val-Arg-Cys-Arg-Tyr-NH₂].

26. A compound of formula (II)
F--L₂--B₂--Y--E (II)
wherein
F is fluorine isotope
L₂ is a moiety group or bond to which F is attached
B₂ is a functional group or chain containing functional group connecting L₂ with the spacer Y
Y is a bond or spacer
E is a peptide or peptidomimetic
and pharmaceutically acceptable salts of inorganic or organic acids thereof.

27. A compound of formula (II) according to claim 26 wherein fluoro isotope F is ¹⁸F or ¹⁹F.

28. A compound according to claim 26 wherein L₂ is a moiety group or bond to which F is attached as described for L₁ when RG is a), b) c) or
L₂ is α-(substituted)amino-ethyl to which F is attached at β-position when RG is d), J and W are defined above

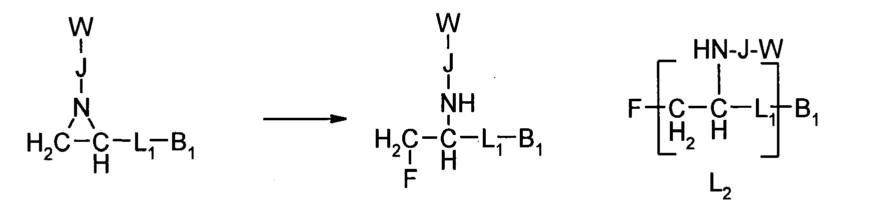

29. A compound according to claim 28 wherein L₂ 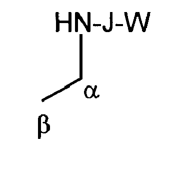 J and W are defined above.

30. A compound according to claim 26 wherein Y and E are defined as in the preceding claims.

31. A compound according to claim 26 wherein B₂ is identical to B₁.

32. Compounds selected from
IIA-a-1 4-[18]Fluoro-3-cyano-benzoyl-Arg-Ava-Gln-Trp-Ala-Val-NMeGly-His-Sta-Leu-NH2
IIA-a-2 4-[18]Fluoro-3-cyano-benzoyl-Arg-Ava-Gln-Trp-Ala-Val-His(Me)-Sta-Leu-NH2
IIA-a-3 4-[18]Fluoro-3-cyano-benzoyl-Arg-Ava-Gln-Trp-Ala-Val-NMeGly-His(3Me)-Sta-Leu-NH2
IIA-a-4 4-[18]Fluoro-3-cyano-benzoyl-1,4-cis-Achc-Gln-Trp-Ala-Val-Gly-His(3Me)-Sta-Leu-NH2
IIA-a-5 4-[18]Fluoro-3-cyano-benzoyl-Gln-Trp-Ala-Val-Gly-His(3Me)-Sta-Leu-NH2 (Y = bond)
IIA-a-6 4-[18]Fluoro-3-cyano-benzoyl-AOC-Gln-Trp-Ala-Val-Gly-His(3Me)-Sta-Leu-NH2
IIA-a-7 4-[18]Fluoro-3-cyano-benzoyl-Ava-Gln-Trp-Ala-Val-NMeGIy-His(3Me)-Sta-Cpa-NH2
IIA-a-8 4-[18]Fluoro-3-cyano-benzoyl-Ava-Gln-Trp-Ala-Val-Gly-His(3Me)-FA4-Am,5-MeHpA-Leu-NH2
IIA-a-9 4-[18]Fluoro-3-cyano-benzoyl-Ava-Gin-Trp-Ala-Val-Gly-His(3Me)-Sta-Leu-NH2
IIA-a-10 4-[18]Fluoro-3-cyano-benzoyl-Lys(Me)2-βAla-Gln-Trp-Ala-Val-Gly-His(3Me)-Sta-Leu-NH2
IIA-a-11 4-[18]Fluoro-3-cyano-benzoyl-Lys(Me)2-βAla-Gln-Trp-Ala-Val-Gly-His(3Me)-Sta-Leu-NH2
IIA-a-12 4-[18]Fluoro-3-cyano-benzoyl-Arg-Ser-Gln-Trp-Ala-Val-Gly-His(3Me)-4-Am,5-MeHpA-Leu-NH2
IIA-a-13 4-[18]Fluoro-3-cyano-benzoyl-Ser-Ser-Gln-Trp-Ala-Val-Gly-His(3Me)-4-Am,5-MeHpA-Leu-NH2
IIA-a-14 4-[18]Fluoro-3-cyano-benzoyl-Lys(Me)2-Ser-Gln-Trp-Ala-Val-Gly-His(3Me)-4-Am,5-MeHpA-Leu-NH2
IIA-a--15 4-[18]Fluoro-3-cyano-benzoyl-Arg-Ser-Gln-Trp-Ala-Val-Gly-His(3Me)-Sta-Leu-NH2
IIA-a-16 4-[18]Fluoro-3-cyano-benzoyl-Lys(Me)2-βAla-Gln-Trp-Ala-Val-Gly-His(3Me)-4-Am,5-MeHpA-Leu-NH2
IIA-a-17 4-[18]Fluoro-3-cyano-benzoyl-Ava-Gln-Trp-Ala-Val-Gly-His-4-Am,5-MeHpA-Leu-NH2,
IIA-a-18 4-[18]Fluoro-3-trifluoromethyl-benzoyl-Arg-Ava-Gln-Trp-Ala-Val-Gly-His(3Me)-Sta-Leu-NH2,
IIA-a-19 4-[18]Fluoro-3-trifluoromethyl-benzoyl-Arg-Ava-Gln-Trp-Ala-Val-NMeGIy-His(3Me)-Sta-Leu-NH2,
IIA-a-20 4-[18]Fluoro-3-trifluoromethyl-benzoyl-1,4-cis-Achc-Gln-Trp-Ala-Val-Gly-His(3Me)-Sta-Leu-NH2,
IIA-a-21 4-[18]Fluoro-3-trifluoromethyl-benzoyl-Gln-Trp-Ala-Val-Gly-His(3Me)-Sta-Leu-NH2,
IIA-a-22 4-[18]Fluoro-3-trifluoromethyl-benzoyl-Arg-βAla-Gln-Trp-Ala-Val-Gly-His(3Me)-4-Am,5-MeHpA-Leu-NH2,
IIA-c-2: ¹⁸F-Si(tBu)₂-C₆H₄-CH₂-CO-Ava--Gln-Trp-Ala-Val-NMeGly-His(3Me)-4-Am,5-MeHpA-Cpa-NH2,
IIB-c-1: ¹⁹F-Si(iPr)₂-C₆H₄-CH₂-CO-Ava--Gln-Trp-Ala-Val-Gly-His(3Me)-4-Am,5-MeHpA-Leu-NH2,
IIB-c-2: ¹⁹F-Si(tBu)₂-C₆H₄-CH₂-CO-Ava--Gln-Trp-Ala-Val-NMeGly-His(3Me)-4-Am,5-MeHpA-Cpa-NH2,
IIB-a-1 4-[19]-Fluoro-3-cyano-benzoyl-Arg-Ava-Gln-Trp-Ala-Val-NMeGIy-His-Sta-Leu-NH2,
IIB-a--2 4-[19]-Fluoro-3-cyano-benzoyl-Arg-Ava-Gln-Trp-Ala-Val-His(Me)-Sta-Leu-NH2,
IIB-a-3 4-[19]-Fluoro-3-cyano-benzoyl-Arg-Ava-Gln-Trp-Ala-Val-NMeGIy-His(3Me)-Sta-Leu-NH2,
IIB-a-4 4-[19]-Fluoro-3-cyano-benzoyl-1,4-cis-Achc-Gln-Trp-Ala-Val-Gly-His(3Me)-Sta-Leu-NH2,
IIB-a-5 4-[19]-Fluoro-3-cyano-benzoyl-Gln-Trp-Ala-Val-Gly-His(3Me)-Sta-Leu-NH2,
IIB-a-6 4-[19]-Fluoro-3-cyano-benzoyl-AOC-Gln-Trp-Ala-Val-Gly-His(3Me)-Sta-Leu-NH2,
IIB-a-7 4-[19]-Fluoro-3-cyano-benzoyl-Ava-Gln-Trp-Ala-Val-NMeGly-His(3Me)-Sta-Cpa-NH2,
IIB-a-8 4-[19]-Fluoro-3-cyano-benzoyl-Ava-Gln-Trp-Ala-Val-Gly-His(3Me)-4-Am,5-MeHpA-Leu-NH2,
IIB-a-9 4-[19]-Fluoro-3-cyano-benzoyl-Ava-Gln-Trp-Ala-Val-Gly-His(3Me)-Sta-Leu-NH2,
IIB-a-10 4-[19]-Fluoro-3-cyano-benzoyl-Lys(Me)2-βAla-Gln-Trp-Ala-Val-Gly-His(3Me)-Sta-Leu-NH2,
IIB-a-11 4-[19]-Fluoro-3-cyano-benzoyl-Lys(Me)2-βAla-Gln-Trp-Ala-Val-Gly-His(3Me)-Sta-Leu-NH2,
IIB-a-12 4-[19]-Fluoro-3-cyano-benzoyl-Arg-Ser-Gln-Trp-Ala-Val-Gly-His(3Me)-4-Am,5-MeHpA-Leu-NH2,
IIB-a-13 4-[19]-Fluoro-3-cyano-benzoyl-Ser-Ser-Gln-Trp-Ala-Val-Gly-His(3Me)-4-Am,5-MeHpA-Leu-NH2,
IIB-a-14 4-[19]-Fluoro-3-cyano-benzoyl-Lys(Me)2-Ser-Gln-Trp-Ala-Val-Gly-His(3Me)-4-Am,5-MeHpA-Leu-NH2,
IIB-a-15 4-[19]-Fluoro-3-cyano-benzoyl-Arg-Ser-Gln-Trp-Ala-Val-Gly-His(3Me)-Sta-Leu-NH2,
IIB-a-16 4-[19]-Fluoro-3-cyano-benzoyl-Lys(Me)2-βAla-Gln-Trp-Ala-Val-Gly-His(3Me)-4-Am,5-MeHpA-Leu-NH2,
IIB-a-17 4-[19]-Fluoro-3-cyano-benzoyl-Ava-Gln-Trp-Ala-Val-Gly-His-4-Am,5-MeHpA-Leu-NH2,
IIB-a-18 4-[19]-Fluoro-3-trifluoromethyl-benzoyl-Arg-Ava-Gln-Trp-Ala-Val-Gly-His(3Me)-Sta-Leu-NH2,
IIB-a-19 4-[19]-Fluoro-3-trifluoromethyl-benzoyl-Arg-Ava-Gln-Trp-Ala-Val-NMeGly-His(3Me)-Sta-Leu-NH2,
IIB-a-20 4-[19]-Fluoro-3-trifluoromethyl-benzoyl-1,4-cis-Achc-Gln-Trp-Ala-Val-Gly-His(3Me)-Sta-Leu-NH2,
IIB-a-21 4-[19]-Fluoro-3-trifluoromethyl-benzoyl-Gln-Trp-Ala-Val-Gly-His(3Me)-Sta-Leu-NH2,
IIB-a-22 4-[19]-Fluoro-3-trifluoromethyl-benzoyl-Arg-βAla-Gln-Trp-Ala-Val-Gly-His(3Me)-4-Am,5-MeHpA-Leu-NH2,
IIB-a-23 4-[19]-Fluoro-3-cyano-benzoyl-Ava-Gln-Trp-Ala-Val-NMeGly-His(3Me)-4-Am,5-MeHpA-Cpa-NH2,
IIB-a-24 4-[19]-Fluoro-3-cyano-benzoyl -Ser-Ser-Gln-Trp-Ala-Val-Gly-His(3Me)-Sta-Leu-NH2 ,
IIB-a-25 4-[19]-Fluoro-3-cyano-benzoyl-DOA-Gln-Trp-Ala-Val-Gly-His(3Me)-Sta-Leu-NH2,
IIB-a-26 3,4-[19]-Difluorobenzoyl-Ava-Gln-Trp-Ala-Val-NMeGIy-His-Sta-Leu-NH2,
IIB-a-27 3,4-[19]-Difluorobenzoyl-Ava-Gln-Trp-Ala-Val-NMeGly-His-FA02010-Cpa-NH2,
IIB-a-28 3,4-[19]-Difluorobenzoyl-Ava-Gln-Trp-Ala-Val-NMeGly-His-4-Am,5-MeHpA-tbuGly-NH2,
IIB-a-29 3,4-[19]-Difluorobenzoyl-Ava-Gln-Trp-Ala-Val-NMeGIy-His(3Me)-Sta-Leu-NH2,
IIB-a-30 3,4-[19]-Difluorobenzoyl-Ava-Gln-Trp-Ala-Val-NMeGIy-His(3Me)-Sta-tBuGly-NH2,
IIB-a-31 3,4-[19]-Difluorobenzoyl-Ava-Gln-Trp-Ala-Val-Gly-His(3Me)-Sta-Leu-NH2,
IIB-a-32 3,4-[19]-Difluorobenzoyl-Ava-Gln-Trp-Ala-Val-NMeGIy-His(3Me)-4-Am,5-MeHpA-Leu-NH2,
IIB-a-33 3,4-[19]-Difluorobenzoyl-Ava-Gln-DTrp-Ala-Val-Gly-His-4-Am,5-MeHpA-tbuGly-NH2,
IIB-a-34 3,4-[19]-Difluorobenzoyl-Ava-Gln-DTrp-Ala-Val-Gly-His-4-Am-5-MeHxA-Cpa-NH2,
IIB-a-35 3,4-[19]-Difluorobenzoyl-Ava-Gln-Trp-Ala-Val-NMeGly-His(3Me)-Sta-Cpa-NH2,
IIB-a-36 3,4-[19]-Difluorobenzoyl-Ava-Gln-DTrp-Ala-Val-Gly-His-Sta-tbuAla-NH2,
IIB-a-37 3,4-[19]-Difluorobenzoyl-Arg-Ava-Gin-Trp-Ala-Val-NMeGly-His-Sta-Leu-NH2,
IIB-a-38 3,4-[19]-Difluorobenzoyl-Gln-Trp-Ala-Val-Gly-His(3Me)-Sta-Leu-NH2,
IIB-a-39 3,4-[19]-Difluorobenzoyl-Arg-Ava-Gln-Trp-Ala-Val-NMeGly-His(3Me)-Sta-Leu-NH2,
IIB-a-40 3,4-[19]-Difluorobenzoyl-Arg-Ava-Gln-Trp-Ala-Val-Gly-His(3Me)-Sta-Leu-NH2,
IIB-a-41 3,4-[19]-Difluorobenzoyl-Arg-βAla-Arg-Gln-Trp-Ala-Val-Gly-His(3Me)-Sta-Leu-NH2,
IIB-a-42 3,4-[19]-Difluorobenzoyl-Ava-Gln-Trp-Ala-Val-Gly-His(3Me)-Sta-Cpa-NH2,
IIB-a-43 3,4-[19]-Difluorobenzoyl-Ava-Gln-Trp-Ala-Val-Gly-His(3Me)-Sta-tBuGly-NH2,
IIB-a-44 3,4-[19]-Difluorobenzoyl-Arg-Arg-Gln-Trp-Ala-Val-NMeGly-His(3Me)-Sta-Leu-NH2,
IIB-a-45 3,4-[19]-Difluorobenzoyl-Arg-βAla-Gln-Trp-Ala-Val-NMeGly-His(3Me)-Sta-Leu-NH2
IIB-a-46 3,4-[19]-Difluorobenzoyl-Gln-Trp-Ala-Val-Gly-His(3Me)-4-Am,S-MeHpA-Leu-NH2,
IIB-a-47 3,4-[19]-Difluorobenzoyl-Ava-Gln-Trp-Ala-Val-NMeGly-His(3Me)-4-Am,5-MeHpA-Cpa-NH2,
IIB-a-48 3,4-[19]-Difluorobenzoyl-Ava-Gln-Trp-Ala-Val-Gly-His(3Me)-4-Am,5-MeHpA-Leu-NH2,
IIB-a-49 3,4-[19]-Difluorobenzoyl-Ava-Gln-Trp-Ala-Val-Gly-NMeHis-4-Am,5-MeHpA-Cpa-NH2,
IIB-a-49 3,4-[19]-Difluorobenzoyl-Ava-Gln-Trp-Ala-Val-Gly-NMeHis(3Me)-4-Am,5-MeHpA-Leu-NH2,
IIB-a-50 3,4-[19]-Difluorobenzoyl-Ava-Gln-Trp-Ala-Val-Gly-NMeHis-4-Am,5-MeHpA-Leu-NH2,
IIB-a-51 3,4-[19]-Difluorobenzoyl-Ava-Gln-Trp-Ala-Val-NMeGly-Hls-AHMHxA -Leu-NH2,
IIB-a-52 3,4-[19]-Difluorobenzoyl-Ava-Gln-Trp-Ala-Val-βAla-NMeHis-Tha-Cpa-NH2,
IIB-a-53 3,4-[19]-Difluorobenzoyl-Ava-Gln-Trp-Ala-Val-βAla-NMeHis-Phe-Cpa-NH2,
IIB-a-54 3,4-[19]-Difluorobenzoyl-Ava-Gln-Trp-Ala-Val-βAla-NMeHis-Phe-Leu-NH2,
IIB-a-55 3,4-[19]-Difluorobenzoyl-Ava-Gln-Trp-Ala-Val-βAla-DHis-Phe-Leu-NH2,
IIB-a-56 3,4-[19]-Difluorobenzoyl-Ava-Gln-Trp-Ala-Val-βAla-His-ßhLeu-Leu-NH2,
IIB-a-57 3,4-[19]-Difluorobenzoyl-Ava-Gln-Trp-Ala-Val-βAla-His-ßhlle-Leu-NH2,
IIB-a-58 3,4-[19]-Difluorobenzoyl-Ava-Gln-Trp-Ala-Val-βAla-His-ßhLeu-tbuGly-NH2,
IIB-a-59 3,4-[19]-Difluorobenzoyl-Ava-Gln-Trp-Ala-Val-βAla-His(3Me)-Phe-Tha-NH2,
IIB-a-60 3,4-[19]-Difluorobenzoyl-Ava-Gln-Trp-Ala-Val-βAla-His(3Me)-Phe-Nle-NH2,
IIB-a-51 3,4-[19]-Difluorobenzoyl-Ava-Gln-Trp-Ala-Val-βAla-NMeHis-Phe-tbuGly-NH2,
IIB-a-52 3,4-[19]-Difluorobenzoyl-Ava-Gln-Trp-Ala-Val-βAla-NMeHis-Tha-tbuGly-NH2,
IIB-a-53 3,4-[19]-Difluorobenzoyl-Ava-Gln-Trp-Ala-Val-βAla-His(3Me)-Tha-tbuGly-NH2,
IIB-a-54 3,4-[19]-Difluorobenzoyl-Ava-Gln-Trp-Ala-Val-βAla-His(3Me)-Phe-Cpa-NH2,
IIB-a-55 3,4-[19]-Difluorobenzoyl-Ava-Gln-Trp-Ala-NMeVal-βAla-His-Phe-Leu-NH2,
IIB-a-56 3,4-[19]-Difluorobenzoyl-Ava-Gln-Trp-Ala-Val-βAla-His-NMePhe-Leu-NH2,
IIB-a-57 3,4-[19]-Difluorobenzoyl-Ava-Gln-DTrp-Ala-Val-βAla-His-Phe-Leu-NH2,
IIB-a-58 3,4-[19]-Difluorobenzoyl-Ava-Gln-Trp-DAla-Val-βAla-His-Phe-Leu-NH2,
IIB-a-59 3,4-[19]-Difluorobenzoyl-Ava-Gln-Trp-Ala-DVal-βAla-His-Phe-Leu-NH2,
IIB-a-60 3,4-[19]-Difluorobenzoyl-Ava-Gln-Trp-Ala-Val-βAla-His-DPhe-Leu-NH2,
IIB-a-61 3,4-[19]-Difluorobenzoyl-Ava-Gln-Trp-Ala-Val-βAla-His-βhlle-tbuGly-NH2,
IIB-a-62 4-[19]-Fluoro-3-cyano-phenylsulfonyl -Ava-Gln-Trp-Ala-Val-NMeGly-His-4-Am,5-MeHpA-Cpa-NH2,
IIB-a-63 4-[19]-Fluoro-3-cyano-phenylsulfonyl -Ava-Gln-Trp-Ala-Val-NMeGly-His-Sta-Cpa-NH2,
IIB-a-64 4-[19]-Fluoro-3-cyano-phenylsulfonyl -Ava-Gln-Trp-Ala-Val-NMeGly-His-Sta-tbuAla-NH2
IIB-a-65 4-[19]-Fluoro-3-cyano-phenylsulfonyl -Ava-Gln-Trp-Ala-Val-NMeGly-His-4-Am,5-MeHpA-tbuAla-NH2
IIA-a-66: 4-[18]Fluoro-3-cyano-benzoyl- Ava-ε-c[Lys-(NMe)Phe-1Nal-D-Trp-Lys-Thr],
IIA-a-67: 4-[18]Fluoro-3-cyano-benzoyl- Ava-β-c[Dpr-Met-(NMe)Phe-Tyr-D-Trp-Lys],
IIB-a-66: 4-[19]Fluoro-3-cyano-benzoyl- Ava-ε-c[Lys-(NMe)Phe-1 Nal-D-Trp-Lys-Thr],
IIB-a-67: 4-[19]Fluoro-3-cyano-benzoyl- Ava-β-c[Dpr-Met-(NMe)Phe-Tyr-D-Trp-Lys],
IIA-a-68: 4-[18]Fluoro-3-cyano-benzoyl- Ava-DCys-Leu-Ile-Thr-Arg-Cys-Arg-Tyr-NH₂,
IIA-a-69: 4-[18]Fluoro-3-cyano-benzoyl- Ava- DCys-Leu-Ile-Val-Arg-Cys-Arg-Tyr-NH₂,
IIA-a-68: 4-[19]Fluoro-3-cyano-benzoyl- Ava-DCys-Leu-Ile-Thr-Arg-Cys-Arg-Tyr-NH₂,
IIA-a-69: 4-[19]Fluoro-3-cyano-benzoyl- Ava- DCys-Leu-Ile-Val-Arg-Cys-Arg-Tyr-NH₂.

33. A compound of the formula (II) according to claim 1 or 2 for use as a positron emitting tomography (PET) diagnostic agent, when the isotope is ¹⁸F.

34. A compound of the formula (II) according to claim 26 for use in biological assays and chromatographic identification, when the isotope is ¹⁹F..

35. Use of compound according to the formula (I) for the manufacture of formula (II) as a diagnostic agent.

36. Method for the manufacture of PET diagnostic agent (II) wherein the isotope is ¹⁸F or ¹⁹F.

37. Method of radiofluorination of a compound of formula I according to claims 1 to 25 for the manufacture of a compound of formula II according to claims 26 to 32.

38. Method of radiofluorination according to claim 37 wherein the reaction temperature is about room temperature to about 80°C.

39. Method of radiofluorination according to claim 37, where the fluorination agent K¹⁸F, H¹⁸F, KH¹⁸F₂ tetraalkylammonium salt of ¹⁸F or 4,7,13,16,21,24-Hexaoxa-1,10-diazabicyclo[8.8.8]-hexacosane K18F (crownether salt Kryptofix K18F).

40. Method of radiofluorination according to claim 39 wherein the fluorination agent is tetraalkylammonium salt of ¹⁸F or 4,7,13,16,21,24-Hexaoxa-1,10-diazabicyclo[8.8.8]-hexacosane K18F (crownether salt Kryptofix K18F).

41. A method according to claim 37, for the preparation of compounds of the formula (II) wherein the fluorine isotope is ¹⁹F.

42. A kit comprising a compound of the formula (I) as disclosed in claims 1 to 2 along with acceptable diluent, excipient or adjuvant supplied as a mixture with the compound of the formula (I) or independently for the manufacture of (II).

43. A peptide of sequence
Seq ID 1 Gln-Trp-Ala-Val-NMeGly-His-Sta-Leu-NH2
Seq ID 2 Gln-Trp-Ala-Val-Gly-His(Me)-Sta-Leu-NH2
Seq ID 3 Gln-Trp-Ala-Val-NMeGly-His(3Me)-Sta-Leu-NH2
Seq ID 4 Gln-Trp-Ala-Val-Gly-His(3Me)-Sta-Leu-NH2
Seq ID 5 Gln-Trp-Ala-Val-Gly-His(3Me)-Sta-Leu-NH2
Seq ID 6 Gln-Trp-Ala-Val-Gly-His(3Me)-Sta-Leu-NH2
Seq ID 7 Gln-Trp-Ala-Val-NMeGly-His(3Me)-Sta-Cpa-NH2
Seq ID 8 Gln-Trp-Ala-Val-Gly-His(3Me)-4-Am,5-MeHpA-Leu-NH2
Seq ID 9 Gln-Trp-Ala-Val-Gly-His(3Me)-Sta-Leu-NH2
Seq ID 10 Gln-Trp-Ala-Val-Gly-His(3Me)-Sta-Leu-NH2
Seq ID 11 Gln-Trp-Ala-Val-Gly-His(3Me)-Sta-Leu-NH2
Seq ID 12 Gln-Trp-Ala-Val-Gly-His(3Me)-4-Am,5-MeHpA-Leu-NH2
Seq ID 13 Gln-Trp-Ala-Val-Gly-His(3Me)-4-Am,5-MeHpA-Leu-NH2
Seq ID 14 Gln-Trp-Ala-Val-Gly-His(3Me)-4-AM-5-MeHpA-Leu-NH2
Seq ID 15 Gln-Trp-Ala-Val-Gly-His(3Me)-Sta-Leu-NH2
Seq ID 16 Gin-Trp-Ala-Val-Gly-His(3Me)-4-Am,5-MeHpA-Leu-NH2
Seq ID 17 Gln-Trp-Ala-Val-Gly-His-4-Am,5-MeHpA- -Leu-NH2
Seq ID 18 Gln-Trp-Ala-Val-Gly-His(3Me)-Sta-Leu-NH2
Seq ID 19 Gln-Trp-Ala-Val-NMeGly-His(3Me)-Sta-Leu-NH2
Seq ID 20 Gln-Trp-Ala-Val-Gly-His(3Me)-Sta-Leu-NH2
Seq ID 21 Gln-Trp-Ala-Val-Gly-His(3Me)-Sta-Leu-NH2
Seq ID 22 Gln-Trp-Ala-Val-Gly-His(3Me)-4-Am,5-MeHpA-Leu-NH2
Seq ID 23 Gin-Trp-Ala-Val-NMeGly-His(3Me)-4-Am,5-MeHpA-Cpa-NH2
Seq ID 24 Gln-Trp-Ala-Val-Gly-His(3Me)-Sta-Leu-NH2
Seq ID 25 Gln-Trp-Ala-Val-Gly-His(3Me)-Sta-Leu-NH2
Seq ID 26 Gln-Trp-Ala-Val-NMeGIy-His-Sta-Leu-NH2
Seq ID 27 Gln-Trp-Ala-Val-NMeGly-His-FA02010-Cpa-NH2
Seq ID 28 Gln-Trp-Ala-Val-NMeGly-His-4-Am,5-MeHpA-tbuGly-NH2
Seq ID 29 Gln-Trp-Ala-Val-NMeGIy-His(3Me)-Sta-Leu-NH2
Seq ID 30 Gln-Trp-Ala-Val-NMeGly-His(3Me)-Sta-tBuGly-NH2
Seq ID 31 Gln-Trp-Ala-Val-Gly-His(3Me)-Sta-Leu-NH2
Seq ID 32 Gln-Trp-Ala-Val-NMeGly-His(3Me)-4-Am,5-MeHpA-Leu-NH2
Seq ID 33 Gln-DTrp-Ala-Val-Gly-His-4-Am,5-MeHpA-tbuGly-NH2
Seq ID 34 Gln-DTrp-Ala-Val-Gly-His-4-Am-5-MeHxA-Cpa-NH2
Seq ID 35 Gln-Trp-Ala-Val-NMeGly-His(3Me)-Sta-Cpa-NH2
Seq ID 36 Gln-DTrp-Ala-Val-Gly-His-Sta-tbuAla-NH2
Seq ID 37 Gln-Trp-Ala-Val-NMeGly-His-Sta-Leu-NH2
Seq ID 38 Gln-Trp-Ala-Val-Gly-His(3Me)-Sta-Leu-NH2
Seq ID 39 Gln-Trp-Ala-Val-NMeGly-His(3Me)-Sta-Leu-NH2
Seq ID 40 Gln-Trp-Ala-Val-Gly-His(3Me)-Sta-Leu-NH2
Seq ID 41 Gln-Trp-Ala-Val-Gly-His(3Me)-Sta-Leu-NH2
Seq ID 42 Gln-Trp-Ala-Val-Gly-His(3Me)-Sta-Cpa-NH2
Seq ID 43 Gln-Trp-Ala-Val-Gly-His(3Me)-Sta-tBuGly-NH2
Seq ID 44 Gln-Trp-Ala-Val-NMeGIy-His(3Me)-Sta-Leu-NH2
Seq ID 45 Gln-Trp-Ala-Val-NMeGIy-His(3Me)-Sta-Leu-NH2
Seq ID 46 Gln-Trp-Ala-Val-Gly-His(3Me)-4-Am,5-MeHpA-Leu-NH2
Seq ID 47 Gln-Trp-Ala-Val-NMeGly-His(3Me)-4-Am,5-MeHpA-Cpa-NH2
Seq ID 48 Gln-Trp-Ala-Val-Gly-His(3Me)-4-Am,5-MeHpA-Leu-NH2
Seq ID 49 Gln-Trp-Ala-Val-Gly-NMeHis-4-Am,5-MeHpA-Cpa-NH2
Seq ID 49 Gln-Trp-Ala-Val-Gly-NMeHis(3Me)-4-Am,5-MeHpA-Leu-NH2
Seq ID 50 Gln-Trp-Ala-Val-Gly-NMeHis-4-Am,5-MeHpA-Leu-NH2
Seq ID 51 Gln-Trp-Ala-Val-NMeGly-Hls-AHMHxA -Leu-NH2
Seq ID 52 Gln-Trp-Ala-Val-βAla-NMeHis-Tha-Cpa-NH2
Seq ID 53 Gln-Trp-Ala-Val-βAla-NMeHis-Phe-Cpa-NH2
Seq ID 54 Gln-Trp-Ala-Val-βAla-NMeHis-Phe-Leu-NH2
Seq ID 55 Gln-Trp-Ala-Val-βAla-DHis-Phe-Leu-NH2
Seq ID 56 Gln-Trp-Ala-Val-βAla-His-ßhLeu-Leu-NH2
Seq ID 57 Gln-Trp-Ala-Val-βAla-His-ßhlle-Leu-NH2
Seq ID 58 Gln-Trp-Ala-Val-βAla-His-ßhLeu-tbuGly-NH2
Seq ID 59 Gln-Trp-Ala-Val-βAla-His(3Me)-Phe-Tha-NH2
Seq ID 60 Gln-Trp-Ala-Val-βAla-His(3Me)-Phe-Nle-NH2
Seq ID 61 Gln-Trp-Ala-Val-βAla-NMeHis-Phe-tbuGly-NH2
Seq ID 62 Gln-Trp-Ala-Val-βAla-NMeHis-Tha-tbuGly-NH2
Seq ID 63 Gln-Trp-Ala-Val-βAla-His(3Me)-Tha-tbuGly-NH2
Seq ID 64 Gln-Trp-Ala-Val-βAla-His(3Me)-Phe-Cpa-NH2
Seq ID 65 Gln-Trp-Ala-NMeVal-βAla-His-Phe-Leu-NH2
Seq ID 66 Gln-Trp-Ala-Val-βAla-His-NMePhe-Leu-NH2
Seq ID 67 Gln-DTrp-Ala-Val-βAla-His-Phe-Leu-NH2
Seq ID 68 Gln-Trp-DAla-Val-βAla-His-Phe-Leu-NH2
Seq ID 69 Gln-Trp-Ala-DVal-βAla-His-Phe-Leu-NH2
Seq ID 70 Gln-Trp-Ala-Val-βAla-His-DPhe-Leu-NH2
Seq ID 71 Gln-Trp-Ala-Val-βAla-His-ßhlle-tbuGly-NH2
Seq ID 72 Gln-Trp-Ala-Val-NMeGly-His-4-Am,5-MeHpA-Cpa-NH2
Seq ID 73 Gin-Trp-Ala-Val-NMeGly-His-Sta-Cpa-NH2
Seq ID 74 Gln-Trp-Ala-Val-NMeGly-His-Sta-tbuAla-NH2
Seq ID 75 Gin-Trp-Ala-Val-NMeGly-His-4-Am,5-MeHpA-tbuAla-NH2
Seq ID 76 Gln-Trp-Ala-Val-NMeGly-His-Sta-Leu-NH2
Seq ID 77 Gln-Trp-Ala-Val-His(Me)-Sta-Leu-NH2
Seq ID 78 Gln-Trp-Ala-Val-NMeGly-His(3Me)-Sta-Leu-NH2
Seq ID 79 Gln-Trp-Ala-Val-Gly-His(3Me)-Sta-Leu-NH2
Seq ID 79 Gln-Trp-Ala-Val-Gly-His(3Me)-Sta-Leu-NH2
Seq ID 80 Gln-Trp-Ala-Val-Gly-His(3Me)-Sta-Leu-NH2
Seq ID 81 Gln-Trp-Ala-Val-NMeGly-His(3Me)-Sta-Cpa-NH2
Seq ID 82 Gln-Trp-Ala-Val-Gly-His(3Me)-FA4-Am,5-MeHpA-Leu-NH2
Seq ID 83 Gln-Trp-Ala-Val-Gly-His(3Me)-Sta-Leu-NH2
Seq ID 84 Gln-Trp-Ala-Val-Gly-His(3Me)-Sta-Leu-NH2
Seq ID 85 Gln-Trp-Ala-Val-Gly-His(3Me)-Sta-Leu-NH2
Seq ID 86 Gln-Trp-Ala-Val-Gly-His(3Me)-4-Am,5-MeHpA-Leu-NH2
Seq ID 87 Gln-Trp-Ala-Val-Gly-His(3Me)-4-Am,5-MeHpA-Leu-NH2
Seq ID 88 Gln-Trp-Ala-Val-Gly-His(3Me)-4-Am,5-MeHpA-Leu-NH2
Seq ID 89 Gln-Trp-Ala-Val-Gly-His(3Me)-Sta-Leu-NH2
Seq ID 90 Gln-Trp-Ala-Val-Gly-His(3Me)-4-Am,5-MeHpA-Leu-NH2
Seq ID 91 Gln-Trp-Ala-Val-Gly-His-4-Am,5-MeHpA-Leu-NH2
Seq ID 92 Gln-Trp-Ala-Val-Gly-His(3Me)-Sta-Leu-NH2
Seq ID 93 Gln-Trp-Ala-Val-NMeGly-His(3Me)-Sta-Leu-NH2
Seq ID 94 Gln-Trp-Ala-Val-Gly-His(3Me)-Sta-Leu-NH2
Seq ID 95 Gln-Trp-Ala-Val-Gly-His(3Me)-Sta-Leu-NH2
Seq ID 96 Gln-Trp-Ala-Val-Gly-His(3Me)-4-Am,5-MeHpA-Leu-NH2
Seq ID 97 Gln-Trp-Ala-Val-Gly-His(3Me)-4-Am,5-MeHpA-Leu-NH2
Seq ID 98 Gin-Trp-Ala-Val-NMeGly-His(3Me)-4-Am,5-MeHpA-Cpa-NH2
Seq ID 99 Gln-Trp-Ala-Val-NMeGly-His-Sta-Leu-NH2
Seq ID 100 Gln-Trp-Ala-Val-Gly-His(3Me)-Sta-Leu-NH2
Seq ID 101 Gln-Trp-Ala-Val-Gly-His(3Me)- 4-Am-5-MeHpA - 4-amino-5-methylheptanoic acid -Leu-NH2
Seq ID 102 Gln-Trp-Ala-Val-NMeGly-His(3Me)- 4-Am-5-MeHpA - 4-amino-5-methylheptanoic acid -Cpa-NH2
Seq ID 103 Gln-Trp-Ala-Val-NMeGIy-His(3Me)- 4-Am-5-MeHpA - 4-amino-5-methylheptanoic acid -Cpa-NH2.

44. A compound according to claim 1 or 2 when RG is d) then RG is N-benzenesulfonylaziridinyl,p-toluenesulfonylaziridinyl or N-2,4,6-triisopropylsulfonylaziridinyl.
